# EUROPEAN PATENT APPLICATION

(11) **EP 1 679 309 A1**
(43) Date of publication of application: **12.07.2006**
(21) Application number: 04793164.7
(22) Date of filing: 22.10.2004
(51) Int. Cl.: C07D 239/42, C07D 239/69, C07D 409/12, A61K 31/505, A61K 31/506, A61K 31/517, A61K 45/00, A61P 1/00, A61P 1/04, A61P 1/10, A61P 1/12, A61P 7/10, A61P 9/02, A61P 9/06, A61P 9/12, A61P 11/00, A61P 11/06, A61P 25/00, A61P 25/08, A61P 25/16, A61P 25/18

(54) **ANTISTRESS DRUG AND MEDICAL USE THEREOF**

(30) Priority: 24.10.2003 JP 2003365237
(71) Applicant: ONO PHARMACEUTICAL CO., LTD., Osaka-shi, Osaka 541-8526 (JP)
(72) Inventor: OHMOTO, Kazuyuki, c/o Ono Pharmaceutical Co., Ltd, Mishima-gun, Osaka 618-8585 (JP); KATO, Masashi, c/o Ono Pharmaceutical Co., Ltd., Mishima-gun, Osaka 618-8585 (JP); KATSUMATA, Seishi, Ono Pharmaceutical Co., Ltd., Mishima-gun, Osaka 618-8585 (JP); MANAKO, Junichiro, c/o Ono Pharmaceutical Co, Ltd, Mishima-gun, Osaka 618-8585 (JP)
(74) Representative: Henkel, Feiler & Hänzel
(86) International application number: PCT/JP2004/016056
(87) International publication number: WO 2005/040135

(57) **Abstract**

The compound represented by formula (I) 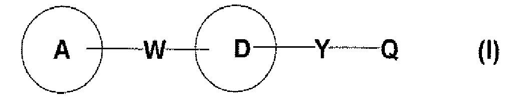 (wherein ringA is cyclic group which may have a substituent(s), Q is alkyl which may have a substituent(s) or cyclic ring which may have a substituent(s), ringD is cyclic ring which may have a substituent(s), W is a single bond or a spacer of which main chain has an atom number of 1-4, Y is a spacer of which main chain has an atom number of 1-4.), a salt thereof, an N-oxide thereof or a solvate thereof, or a prodrug thereof Since the compounds represented by formula (I), a salt thereof, an N-oxide thereof or a solvate thereof, or a prodrug thereof have the affinity to MBR, they are useful for the prevention and/or treatment for disease caused by stress.

## Description

### Technical field

The present invention relates to the nitrogen-containing heterocyclic compound which is usuful for the prevention and/or treatment for disease induced stress, preparation thereof and use thereof.

### Background Art

In 1977, mitochondrial benzodiazepine receptor (hereinafter, it is abbreviated as MBR.) was identified as a receptor that is different from a benzodiazepine binding site in GABA_{A} receptor to which benzodiazepines bind *("Science,* 198, 849-851 (1977)", *"Proc. Natl. Acad. Sci.,* 89, 3805-3809 (1977)"). Though a physiological function is not necessarily clarified, it has been reported to get involved in steroid synthesis, the differentiation and proliferation of cells, and the immune function modulation, *etc.* In peripheral tissue, there are MBRs in immune system cells such as red blood cell, platelet, monocyte, and macrophages besides adrenal cortex, heart, smooth muscle, kidney, lung, testis, and in central nervous system in plexus chorioideus, corpus pineale, olfactory bulb, cerebral cortex, and hippocampus, *etc.* Cells expressing MBRs in central nervous system have mainly been known to glial cells. They have been used as a marker of gliosis so that the MBR expression level increases along with the neurodegenerative disease such as Alzheimer's disease, cerebral ischemia, multiple scleosis, and Huntington's disease, *etc*.

There are MBRs in mitochondrial outer membrane, which transport cholesterol from intracellular to the internal membrane of mitochondria that is the active site of P-450scc. Steroid synthesized in the brain is called as neurosteroid. Cholesterol, which is the steroid precursor, is converted into pregnenolone metabolized with side-chain cleavage enzyme P-450scc. This process is the first process of steroid production system. However, it has been indicated that this transport process was the rate-determining process in steroid production system rather than metabolism with P-450scc. It has been thought that the neurosteroid content in the brain could be adjusted if the function of MBRs could be regulated. Actually, it has been reported that a diazepam binding inhibitor (hereinafter, it may be abbreviated as DBL), which was identified as an endogenous ligand of a benzodiazepine binding site in GABA_{A} receptor and MBRs, promoted the pregnenolone synthesis at mitochondrial fraction derived from rat brain and glioma cells.

It has been reported that DBI content in hippocampus increased by loading sound stressor to rat and DBI concentration in cerebrospinal fluid of patients with depressed mode rose. Therefore, it is expected that the amount of neurosteroid production can increase under stress condition. As experiment results supporting this, it has been reported that the various neurosteroid content in the brain increased by loading stressors to rats, such as forced swimming, foot shock, carbon dioxide exposure and constraint and so on.

Neurosteroids regulate the function of various receptors and ion channels positively or negatively according to the types thereof For example, though pregnenolone sulfate and dehydroepiandrosterone sulfate control the function of GABA_{A} receptor, progesterone, allopregnenolone and tetrahydroxycorticosterone activate it. In addition, though pregnenolone sulfate also controls the function of AMPA/kainate-type glutamate receptor, glycine receptor, and voltage-dependent calcium channel, activates NMDA-type glutamate receptor. Additionally, progesterone controls the function of acetylcholine receptor as well as glycine receptor. Further, though dehydroepiandrosterone sulfate activates the function of σ receptor, progesterone control adversely. Thus, it has been thought that as a result of balance between an excitatory signaling system and an inhibitory signaling system was collapsed by neurosteroid content in the brain varying under stress condition, the various stress-related diseases could be caused by changes of activities in nerve system, immune system and endocrine system which were regulated by these nerve systems. Further, considering it has been reported that pregnenolone sulfate reinforced NMDA-induced cell death in cultured hippocampal nerve cells and caused delayed cell death with DNA fragmentation in neural retina cells, it is suggested that there is possibility that pregnenolone sulfate at least partly takes part in the degeneration of hippocampus CA3 field under stress condition.

As mentioned above, the disrupted balance between an excitatory signaling system and an inhibitory signaling system caused by stressor load can be improved to the desirable balanced condition by the increase or the inhibition of neurosteroid production, which is useful for prevention or treatment for stress-related diseases. Therefore, it is expected that the compounds having affinity for MBRs are extremely useful for prevention and/or treatment for these diseases, if they are supplied.

As therapeutic agent for a stress-related disease, it has been known that the compound represented by formula (X) 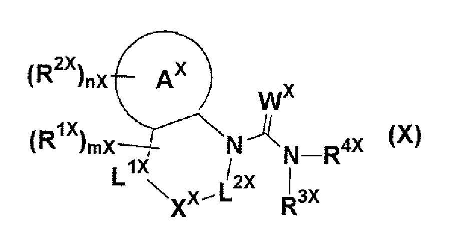 (wherein ring A^{X} is C5-8 mono-cyclic carbocyclic ring or 5-8 membered mono-heterocyclic ring having 1-2 nitrogen atom(s), 1-2 oxygen atom(s) and/or a sulfur atom; X^{X} is -CH₂-, -O-, -S-, *etc*.; L^{1X} and L^{2X} are each independently single bond, C1-4 alkylene or C2-4 alkenylene.; R^{1X} and R^{2X} are each independently C1-8 alkyl *etc*.; mX and nX is 0 or an integer of 1 to 4; R^{3X} is hydrogen atom, ringB^{X} *etc*.; ringB^{X} is C3-10 mono- or bi-carbocyclic ring or 5-10 membered mono- or bi-heterocyclic ring having 1-2 nitrogen atom(s), 1-2 oxygen atom(s) and/or a sulfur atom.; R^{4X} is hydrogen atom, C1-8 alkyl *etc*.), or a pharmaceutically acceptable salt thereof acted to MBRs (*see* WO03068753).

In contrast, it has been disclosed that the compound represented by formula (Y) 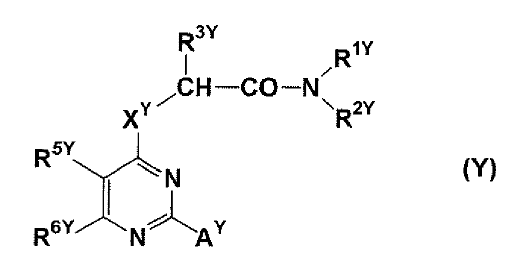 (wherein X^{Y} is -O- or NR^{4Y}-, R^{1Y} is a hydrogen atom, lower alkyl *etc.,* R^{2Y} is lower alkyl, unsubstitued or substituted phenyl *etc.,* R^{3Y} and R^{4Y} is similarly or differently hydrogen atom or lower alkyl, R^{5Y} is a hydrogen atom, halogen atom *etc.,* R^{6Y} is unsubstitued or substituted heteroaryl, A^{Y} is unsubstituted or substituted phenyl, or unsubstituted or substituted heteroaryl.) or physiologically acceptable acid addition salt and pharmaceutical composition consisting thereof selectively acted to MBRs *(see* JP2001-199982).

In addition, (1) N-{4-[2,5-bis(2,2,2-trifluoroethoxy)phenyl]pyrimidin-2-yl}-N'-phenylurea (Registry No. 671185-74-3), (2) N-{4-[2,5-bis(2,2,2-trifluoroethoxy)phenyl]pyrimidin-2-yl}-N'-(4-chlorophenyl)urea (Registry No. 671185-76-5), (3) N-{4-[2,5-bis(2,2,2-trifluoroethoxy)phenyl]pyrimidin-2-yl}-N'-(3-chlorophenyl)urea (Registry No. 671185-77-6) and (4) N-{4-[2,5-bis(2,2,2-trifluoroethoxy)phenyl]pyrimidin-2-yl}-2-thiophenecarboxamide (Registry No. 671185-81-2) have been known as regents.

### Disclosure of the invention

The problem of the present invention is that the compound having the affinity to MBRs as a preventive and/or therapeutic agent for a disease caused by stress is developed.

As a result of the present inventors made further investigation to find out the compound having the affinity for MBRs, they found out that the compounds represented by formula (I) accomplished the purpose and completed the present invention.

That is, the present invention relates to
1. A compound represented by formula (I) 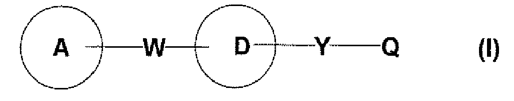 (wherein ringA is cyclic ring which may have a substituent(s), Q is alkyl which may have a substituent(s) or cyclic ring which may have a substituent(s), ringD is cyclic ring which may have a substituent(s), W is a single bond or a spacer of which main chain has an atom number of 1-4, Y is a spacer of which main chain has an atom number of 1-4.), a salt thereof, an N-oxide thereof or a solvate thereof, or a prodrug thereof,
2. The compound according to the above described 1, wherein ringA is (1) C3-10 mono- or bi-carbocyclic ring, or (2) 3-10 membered mono- or bi-heterocyclic ring containing 1 to 5 hetero atom(s) selected from oxygen atom(s), nitrogen atom(s) and sulfur atom(s), which may have a substituent(s),
3. The compound according to the above described 2, wherein ringA is benzene which may have a substituent(s),
4. The compound according to the above described 1, wherein W is a single bond,
5. The compound according to the above described 1, wherein Y is a spacer of which main chain has an atom number of 1-4 containing of hydrogen-bond acceptor site,
6. The compound according to the above described 1, wherein Y is 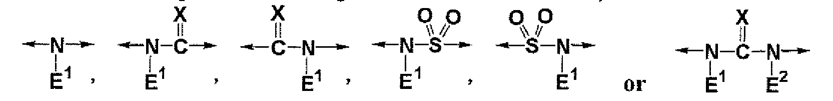 (wherein E¹ and E² are each independently a hydrogen atom(s) or a substituent(s), X is an oxygen atom or a sulfur atom, left-pointing arrow binds to ringD, right-pointing arrow binds to Q),
7. The compound according to the above described 1, wherein ringD is 3-10 membered mono-, bi-heterocyclic ring containing of 1 to 5 hetero atom(s) selected from oxygen atom(s), nitrogen atom(s) and sulfur atom(s), which may have a substituent(s),
8. The compound according to the above described 7, ringD is pyridine or pyrimidine which may have a substituent(s),
9. The compound according to the above described 8, which is the compound represented by formula (Ia) 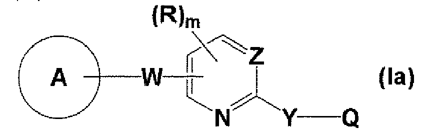 (wherein Z is a carbon atom or nitrogen atom, R is a hydrogen atom(s) or a substituent(s), m is an integer of 1-3, the other symbols have the same meanings as the above described 1.),
10. The compound according to the above described 9, which is the compound represented by formula (Ib) 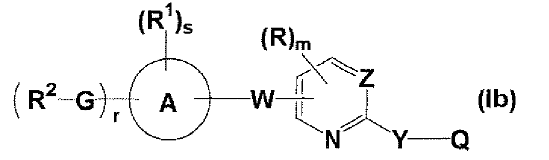 (wherein R¹ is a hydrogen atom(s) or a substituent(s), R² is C1-6 alkyl which is substituted with 1-5 halogen atom(s), G is -O- or -S-, r is an integer of 1-2, s is an integer of 1-4, however, sum of r and s is below 5, the other symbols have the same meanings as the described above 1 and 9.),
11. The compound according to the above described 10, wherein R² is C1-2 alkyl which is substituted with 1-5 fluorine atom(s),
12. The compound according to the above described 10, which is the compound represented by formula (Ib-1) 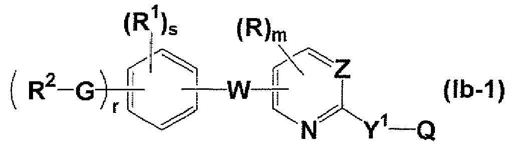 (wherein Y¹ is 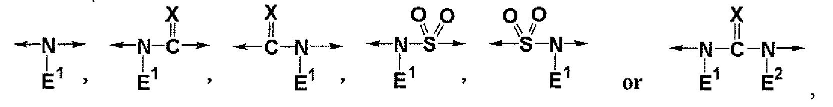 other symbols have the same meanings as the above described 1, 6 and 10, and
   wherein (1) N-{4-[2,5-bis(2,2,2-trifluoroethoxy)phenyl]pyrimidin-2-yl}-N'-phenylurea, (2) N-{4-[2,5-bis(2,2,2-trifluoroethoxy)phenyl]pyrimidin-2-yl}-N'-(4-chlorophenyl)urea, (3) N-{4-[2,5-bis(2,2,2-trifluoroethoxy)phenyl]pyrimidin-2-yl}-N'-(3-chlorophenyl)urea and (4) N-{4-[2,5-bis(2,2,2-trifluoroethoxy)phenyl]pyrimidin-2-yl}-2-thiophenecarboxamide are excepted.),
13. The compound according to the above described 10, the compound is
   (1) N-{4-[2,5-bis(difluoromethoxy)phenyl]pyrimidin-2-yl}thiophene-2-carboxamide,
   (2) N-{4-[2,5-bis(difuloromethoxy)phenyl]pyrimidin-2-yl}-N'-isopropylurea,
   (3) N-{4-[2,5-bis(difluoromethoxy)phenyl]pyrimidin-2-yl}methanesulfonamide,
   (4) N-{4-[2,5-bis(difluoromethoxy)phenyl]pyrimidin-2-yl}benzenesulfonamide,
   (5) N-{4-[2,5-bis(difluoromethoxy)phenyl]pyrimidin-2-yl}-1-phenylmethanesulfonamide,
   (6) N-{4-[2,5-bis(difluoromethoxy)phenyl]pyrimidin-2-yl}-4-methylbenzenesulfonamide,
   (7) N-{4-[2,5-bis(difluoromethoxy)phenyl]pyrimidin-2-yl}-3-methylbenzenesulfonamide,
   (8) N-{4-[2,5-bis(difluoromethoxy)phenyl]pyrimidin-2-yl}-3,5-dimethylbenzenesulfonamide,
   (9) N-{4-[2,5-bis(difluoromethoxy)phenyl]pyrimidin-2-yl}-3,5-dichlorobenzenesulfonamide,
   (10) N-{4-[2,5-bis(2,2,2-trifluoroethoxy)phenyl]pyrimidin-2-yl}benzenesulfonamide,
   (11) N-{4-[2,5-bis(2,2,2-trifluoroethoxy)phenyl]pyrimidin-2-yl}-4-methylbenzenesulfonamide,
   (12) N-{4-[2,5-bis(2,2,2-trifluoroethoxy)phenyl]pyrimidin-2-yl}-N-methylbenzenesulfonamide,
   (13) N-{4[2,5-bis(2,2,2-trifluoroethoxy)phenyl]pyrimidin-2-yl}-N,4-dimethylbenzenesulfonamide,
   (14) N-{4-[2,5-bis(difluoromethoxy)phenyl]pyrimidin-2-yl}-N-methylbenzenesulfonamide,
   (15) N-benzyl-N-{4-[2,5-bis(difluoromethoxy)phenyl]pyrimidin-2-yl}methanesulfonamide,
   (16) N-{4-[2,5-bis(difluoromethoxy)phenyl]pyrimidin-2-yl}-N-ethylbenzenesulfonamide,
   (17) N-{4-[2,5-bis(difluoroemethoxy)phenyl]pyrimidin-2-yl}-N-propylbenzenesulfonamide,
   (18) N-{4-[2,5-bis(difluoromethoxy)phenyl]pyrimidin-2-yl}-N-isobutylbenzenesulfonamide,
   (19) N-{4-[2,5-bis(difluoromethoxy)phenyl]pyrimidin-2-yl}-N-(2-methoxyethyl)benzenesulfonamide,
   (20) 4-[2,5-bis(2,2,2-trifluoroethoxy)phenyl]-N-ethyl-2-pyrimidinamine,
   (21) N-benzyl-4-[2,5-bis(2,2,2-trifluoroethoxy)phenyl]-N-methyl-2-pyrmidinamine, or
   (22) N-benzyl-4-[2,5-bis(2,2,2-trifluoroethoxy)phenyl]-2-pyrimidinamine,
14. A pharmaceutical composition comprising of the compound represented by formula (I) according to the above described 1, a salt thereof, an N-oxide thereof, a solvate thereof or a prodrug thereof,
15. The pharmaceutical composition according to the above described 14, wherein the pharmaceutical composition is preventive and/or therapeutic agent for mitochondrial benzodiazepine receptor mediated disease,
16. The pharmaceutical composition according to the above described 15, wherein the mitochondrial benzodiazepine receptor mediated disease is a disease caused by stress,
17. The pharmaceutical composition according to the above described 16, wherein the disease caused by stress is a central nervous system disease caused by stress, a respiratory system disease caused by stress and/or a digestive system disease caused by stress,
18. The pharmaceutical composition according to the above described 17, wherein the central nervous system disease caused by stress is anxiety-related disease, sleep disorder, depression and/or epilepsy, a respiratory system disease caused by stress is asthma, a digestive system disease caused by stress is irritable bowel syndrome,
19. A pharmaceutical composition combining of the compound represented by formula (I) according to the above described 1, a salt thereof, an N-oxide thereof, a solvate thereof or a prodrug thereof and one kind or more kind selected from antianxiety drugs, antidepressant drugs, antiparkinson drugs, therapeutic drugs for schizophrenia, antiepileptic drugs, therapeutic drugs for asthma, therapeutic drugs for peptic ulcer, adjustive drugs for gastrointestinal function, antidiarrheals, evacuants, antihypertensive drugs, antiarrhythmic drugs, inotropic drugs and therapeutic drugs for urination disorder,
20. A method for prevention and/or treatment for a mitochondrial benzodiazepine receptor mediated disease in mammals is characterized by administration effective dose of the compound represented by formula (I) according to the above described 1, a salt thereof, an N-oxide, a solvate or a prodrug thereof to the mammals, and
21. Use of the compound represented by formula (I) according to the above described 1, a salt thereof, an N-oxide thereof, a solvate thereof or a prodrug thereof for preparing a preventive and/or therapeutic agent for a mitochondrial benzodiazepine receptor mediated disease.

In the specification, disease caused by stress includes, for example, central nervous system disease caused by stress, respiratory system disease caused by stress, digestive system disease caused by stress, cardiovascular system disease caused by stress, uropathy/reproductive system disease caused by stress, gynecologic disease caused by stress, endocrine/metabolic disease caused by stress, ophthalmologic disease caused by stress, otolaryngological disease caused by stress, dental surgery /dentistry disease caused by stress, surgical/orthopedic disease caused by stress, skin disease caused by stress, other disease caused by stress. Central nervous system disease caused by stress, respiratory system disease caused by stress and/or digestive system disease caused by stress is/are preferred.

In the specification, central nervous system disease caused by stress includes, for example, anxiety related disease, neurosis, panic disorder, sleep disorder, depression, reactive depression, epilepsy, Parkinson's disease, Perkinsonian syndrome, schizophrenia, autonomic imbalance, Huntington's disease, Alzheimer's disease, affective disorder, cognitive disorder, migraine, tension headache, cluster headache, posttraumatic stress disorder, dissociative disorder, insomnia, nervous vomiting, nervous cough, psychogenic convulsive seizure, psychogenic syncopal attack, maladjustment to job, burn-out syndrome, chronic fatigue syndrome, writer's cramp, spastic torticollis and so on. Anxiety related disease, sleep disorder, depression and/or epilepsy is/are preferred.

In the specification, respiratory system disease caused by stress includes, for example, asthma, bronchial asthma, hyperventilation syndrome, laryngospasm, chronic obstructive pulmonary disease and so on. Asthma is preferred.

In the specification, digestive system disease caused by stress includes, for example, irritable bowel syndrome, peptic ulcer, functional dyspepsia, gastric ulcer, duodenal ulcer, ulcerative colitis, biliary tract dyskinesia, esophagospasm, gastric atony, aerophagy, chronic hepatitis, chronic panceatitis and so on. Irritable bowel syndrome is preferred.

In the specification, cardiovascular system disease caused by stress includes, for example, essential hypertension, arrhythmia, (neurological) angina pectoris, essential hypotension, orthostatic dysregulation, myocardial infarction, arteriosclerosis, vertigo and so on. Essential hypertension, arrhythmia and/or angina pectoris is/are preferred.

In the specification, uropathy/reproductive system disease caused by stress includes, for example, dysuria, nervous pollakiuria (irritable bladder), nocturia, enuresis, psychogenic ischuria, impotentia, prostatism, urethral syndrome and so on. Dysuria is preferred.

In the specification, gynecologic disease caused by stress includes, for example, menopausal disorder, menorrhalgia, emmeniopathy, premenstrual syndrome, infertility, frigidity, hyperemesis, abortion, premature birth and so on.

In the specification, endocrine/metabolic disease caused by stress includes, for example, anorexia nervosa, eating disorder, cibophobia, bulima, Bartter's syndrome, hyperthyroidism, diabetes, psychogenic polydipsia, adiposity, reflex hypoglycemia and so on.

In the specification, ophthalmologic disease caused by stress includes, for example, asthenopia, central retinitis, muscae volitantes, blepharospasm, primary glaucoma, vertigo and so on.

In the specification, otolaryngological diseases caused by stress includes, for example, susurrus aurium, vertigo, psychogenic deafness, chronic sinusitis, allergic rhinitis, smell disorder, stuttering, aphonia and so on.

In the specification, dental surgery/dentistry caused by stress includes, for example, temporomandibular arthrosis, glossopharyngeal neuralgia, spontaneous glossodynia, stomatitis, toothache, ozostomia, abnormal salivation, bruxism and so on.

In the specification, surgical/orthopedic disease caused by stress includes, for example, postoperative abdominal neurosis, dumping syndrome, polysurgery, plastic postoperative neurosis, rheumatoid arthritis, lumbago, cervico-omo-brachial syndrome, stiff neck, fibrositis, polyarthralgia, systemic myalgia, gout and so on.

In the specification, skin disease caused by stress includes, for example, chronic urticaria, atopic dermatitis, sudoresis, eczema, skin pruritus, alopecia areata and so on.

In the specification, other disease caused by stress includes, for example, cancer, systemic lupus erythematosus and so on.

In the specification, anxiety related disease includes, for example, neurosis, psychosomatic disorder, generalized anxiety disorder (GAD), social-anxiety disorder (SAD), panic disorder, hyperactivity disorder, attention-deficit, personality disorder, bipolar disorder, autism and so on.

In the specification, "cyclic group" in "cyclic group which may have a substituent(s)" represented by ringA, ringD and Q means, for example, carbocyclic ring and heterocyclic ring and so on. Carbocyclic ring means, for example, C3-20 mono-, bi-, tri- or tetra-aromatic carbocyclic ring partially or fully saturated, spiro-linked bi-, tri-, or tetra-carbocyclic ring, and bridged bi-, tri-, or tetra-carbocyclic ring and so on. C3-20 mono-, bi-, tri- or tetra-aromatic carbocyclic ring partially or fully saturated means, for example, benzene, azulene, naphthalene, phenanthrene, anthracene, triphenylene, chrysene, naphthacene, pleiadene, cyclopropane, cyclobutane, cyclopentane, cyclohexane, cycloheptane, cyclooctane, cyclononane, cyclodecane, cycloundecane, cyclododecane, cyclotridecane, cyclotetradecane, cyclopentadecane, cyclopentene, cyclohexene, cycloheptene, cyclooctene, cyclopentadiene, cyclohexadiene, cycloheptadiene, cyclooctadiene, pentalene, perhydropentalene, perhydroazulene, indene, perhydroindene, indane, dihydronaphthalene, tetrahydronaphthalene, perhydronaphthalene, heptalene, perhydroheptalene, biphenylene, as-indacene, s-indacene, acenaphthylene, acenaphtene, fluorene, phenalene, fluoranthene, acephenanthrylene, aceanthrylene, pyrene and so on. Spiro-linked bi-, tri-, or tetra-carbocyclic ring , and bridged bi-, tri-, or tetra-carbocyclic ring mean, for example, spiro[4.4]nonane, spiro[4.5]decane, spiro[5.5]undecane, bicyclo[2.2.1]heptane, bicyclo[2.2.1]hepta-2-ene, bicyclo[3.1.1]heptane, bicyclo[3.1.1]hepta-2-ene, bicyclo[2.2.2]octane, bicyclo[2.2.2]octa-2-ene, adamantane, noradamantane and so on. Heterocyclic ring means, for example, 3-20 membered mono-, bi-, tri-, or tetra-aromatic heterocyclic ring optionally partially or fully saturated containing 1 to 5 hetero atom(s) selected from oxygen atom(s), nitrogen atom(s) and/or sulfur atom(s) and so on. 3-20 membered mono-, bi-, tri-, or tetra-aromatic heterocyclic ring optionally partially or fully saturated containing 1 to 5 hetero atom(s) selected from oxygen atom(s), nitrogen atom(s) and/or sulfur atom(s) means, for example, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, pyrrolopyridine, benzoxazole, benzothiazole, benzimidazole, chromene, benzoxepine, benzoxazepine, benzoxadiazepine, benzothiepine, benzothiazepine, benzothiadiazepine, benzazepine, benzodiazepine, benzofurazan, benzothiadiazole, benzotriazole, carbazole, β-carboline, acridine, phenazine, dibenzofuran, xanthene, dibenzothiophene, phenothiazine, phenoxazine, phenoxathiin, thianthrene, phenanthridine, phenanthroline, perimidine, pyridonaphthyridine, pyrazoloisoquinoline, pyrazolonaphthyridine, pyrimidoindole, indolizinoindole, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazale, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, tetrahydropyrrolopyridine, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, tetrapyridonaphthyridine, tetrahydro-β-carboline, dihydroazepinoindole, hexahydroazepinoindole, tetrahydropyrazoloisoquinoline, tetrahydropyrazolonaphthyridine, dihydroazepinoindazole, hexahydroazepinoindazole, dihydropyrazolopyridoazepine, hexahydropyrazolopyridoazepine, tetrahydropyrimidoindole, dihydrothiazinoindole, tetrahydrothiazinoindole, dihydrooxazinoindole, tetrahydrooxazinoindole, hexahydroindolizinoindole, dihydroindolobenzdiazepine, octahydroindoloquinolizine, hexahydroimidazopyridoindole, hexahydropyrrolothiazepinoindole, dioxolane, dioxane, dithiolane, dithiane, dioxaindan, benzodioxane, chroman, benzodithiolane, benzodithiane, azaspiro[4.4]nonane, oxazaspiro[4.4]nonane, oxazaspiro[2.5]octane, dioxaspiro[4.4]nonane, azaspiro[4.5]decane, thiaspiro[4.5]decane, dithiaspiro[4.5]decane, dioxaspiro[4.5]decane, oxazaspiro[4.5]decane, azaspiro[5.5]undecane, oxaspiro[5.5]undecane, dioxaspiro[5.5]undecane, 2,3,4,9-tetrahydrospiro[β-carboline-1,1'-cyclopentane], azabicyclo[2.2.1]heptane, oxabicyclo[2.2.1]heptane, azabicyclo[3.1.1]heptane, azabicyclo[3.2.1]octane, oxabicyclo[3.2.1]octane, azabicyclo[2.2.2]octane, diazabicyclo[2.2.2]octane and so on.

In the specification, "substituent" in "cyclic group which may have a substituent(s)" represented by ringA, ringD and Q means, for example, (1) alkyl which may have a substituent(s), (2) alkenyl which may have a substituent(s), (3) alkynyl which may have a substituent(s), (4) carbocyclic ring which may have a substituent(s), (5) heterocyclic ring which may have a substituent(s), (6) hydroxyl which may have a substituent(s), (7) mercapto which may have a substituent(s), (8) amino which may have a substituent(s), (9) carbamoyl which may have a substituent(s), (10) sulfamoyl which may have a substituent(s), (11) carboxyl, (12) (alkyl which may have a substituent(s))oxycarbonyl, (13) sulfo (-SO₃H), (14) sulfino, (15) phosphono, (16) nitro, (17) cyano, (18) amidino, (19) imino, (20) dihydroborono (-B(OH)₂), (21) halogen atom (fluorine, chlorine, bromine, iodine), (22) alkylsulfinyl (C1-4 alkylsulfinyl *etc*., such as methylsulfinyl, ethylsulfinyl and so on.), (23) heterocyclic ring sulfinyl (C6-10 heterocyclic ring sulfinyl *etc*., such as phenylsulfinyl and so on.), (24) alkylsulfonyl (C1-4 alkylsulfonyl *etc*., such as methylsulfonyl, ethylsulfonyl and so on.), (25) heterocyclic ring sulfonyl (C6-10 heterocyclic ring sulfonyl *etc.,* such as phenylsulfonyl and so on.), (26) acyl, (27) oxo, (28) thioxo, (29) (C1-6 alkoxyimino)methyl (*e*.*g*. (methoxyimino)methyl *etc*.) and so on. These optional substituents may be substituted 1-5 at the replaceable position. In addition, a substituent in "cyclic group which may have a substituent(s)" represented by ringA and a substituent in "cyclic group which may have a substituent(s)" represented by ringD may be together to form ethylene (-CH₂-CH₂-).

Alkyl in "(1) alkyl which may have a substituent(s)" as substituent means straight-chain or branched-chain C1-20 alkyl and so on, such as methyl, ethyl, n-propyl, isopropyl, n-butyl, isobutyl, *sec*-butyl, *tert*-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, icosyl and so on. Here, substituent of alkyl means, for example, hydroxyl, mercapto, amino, carboxyl, nitro, cyano, mono- or di-C1-6 alkylamino, (*e*.*g*. methylamino, ethylamino, propylamino, dimethylamino, diethylamino *etc*.), N-heterocyclic ring amino (*e*.*g*. N-phenylamino *etc*.), N-heterocyclic ring-N-alkylamino (*e*.*g*. N-phenyl-N-methylamino, N-phenyl-N-ethylamino, N-phenyl-N-propylamino, N-phenyl-N-butylamino, N-phenyl-N-pentylamino, N-phenyl-N-hexylamino *etc*.), acylamino, N-acyl-N-alkylamino, C1-6 alkoxy (e.g. methoxy, ethoxy, propoxy, isopropoxy, hexyloxy *etc.),* C3-7 cycloalkyl-C1-6 alkoxy (*e*.*g*. cyclohexylmethyloxy, cyclopentylethyloxy *etc.),* C3-7 cycloalkyloxy (*e*.*g*. cyclohexyloxy *etc.*), C7-15 aralkyloxy (*e*.*g*. benzyloxy, phenetyloxy, phenylpropyloxy, naphthylmethyloxy, naphthylethyloxy *etc.*), phenoxy, C1-6 alkoxycarbonyl (*e.g.* methoxycarbonyl, ethoxycarbonyl, *tert*-butoxycarbanyl *etc*.), C1-6 alkylcarbonyloxy (*e.g.* acetoxy, ethylcarbonyloxy *etc*.), C1-6 alkylthio (*e*.*g*. methylthio, ethylthio, propylthio, isopropylthio, butylthio, pentylthio, hexylthio *etc*.), halogen atom (fluorine, chlorine, bromine, iodine), alkylsufonyl (C1-4 alkylsulfonyl *etc.,* such as methylsulfonyl, ethylsulfonyl and so on.), heterocyclic ring sulfonyl (C6-10 heterocyclic ring sulfonyl *etc.,* such as phenylsulfonyl and so on.), carbamoyl which may have a substituent(s) (*e*.*g*. unsubstituted carbamoyl, N-mono-C1-6 alkylcarbamoyl (*e*.*g*. N-methylcarbamoyl, N-ethylcarbamoyl, N-propylearbamoyl, N-isopropylcarbamoyl, N-butylcarbamoyl *etc*.), N,N-diC1-6 alkylcarbamoyl (*e*.*g*. N,N-dimethylcarbamoyl, N,N-diethylcarbamoyl, N,N-dipropylcarbamoyl, N,N-dibutylcarbamoyl *etc*.), piperidin-1-ylcarbamoyl *etc.),* acyl, carboycyclic ring which may have a substituent(s) and heterocyclic ring which may have a substituent(s) and so on. These optional substituents may be substituted 1-5 at the replaceable position. Here, alkyl in N-acyl-N-alkylamino means straight-chain or branched-chain C1-6 alkyl and so on, such as methyl, ethyl, n-propyl, isopropyl, n-butyl, isobutyl, *sec*-butyl, *tert*-butyl, pentyl, hexyl *etc.* In addition, acyl in acyl, acylamino and N-acyl-N-alkylamino has the same meanings as below described "(26) acyl". In addition, carbocyclic ring which may have a substituent(s) and heterocyclic ring which may have a substituent(s) have the same meanings as below described "(4) carbocyclic ring which may have a substituent(s)" and "(5) heterocyclic ring which may have a substituent(s)".

Alkenyl in "(2) alkenyl which may have a substituent(s)" as substituent means straight-chain or branched-chain C2-8 alkenyl and so on, such as ethenyl, propenyl, butenyl, pentenyl, hexenyl, heptenyl, octenyl *etc.* Here, substituent of alkenyl has the same meanings as above described substituent in "(1) alkyl which may have a substituent(s)".

Alkynyl in "(3) alkynyl which may have a substituent(s)" as substituent means straight-chain or branched-chain C2-8 alkynyl and so on, such as ethynyl, propynyl, butynyl, pentynyl, hexynyl, heptynyl, octynyl *etc.* Here, substituent of alkynyl has the same meanings as above described substituent in "(1) alkyl which may have a substituent(s)".

Carbocyclic ring in "(4) carbocyclic ring which may have a substituent(s)" as substituent has the same meanings as above described carbocyclic ring represented by ringA. Here, substituent of carbocyclic ring means, for example, straight-chain or branched-chain C1-8 alkyl which may be substituted with hydroxyl (*e*.*g*. methyl, ethyl, n-propyl, isopropyl, n-butyl, isobutyl, *sec*-butyl, *tert*-butyl, pentyl, hexyl, heptyl, octyl *etc.*), straight-chain or branched-chain C2-6 alkenyl (*e.g.* ethenyl, propenyl, butenyl, pentenyl, hexenyl *etc*.), straight-chain or branched-chain C2-6 alkynyl (*e.g.* ethynyl, propynyl, butynyl, pentynyl, hexynyl *etc.*), hydroxyl, straight-chain or branched-chain C1-6 alkoxy (*e*.*g*. methoxy, ethoxy, propoxy, isopropoxy, butoxy, isobutyloxy, *tert-*butoxy, pentyloxy, hexyloxy *etc*.), mercapto, straight-chain or branched-chain C1-6 alkylthio (*e*.*g*. methylthio, ethylthio, propylthio, isopropylthio, butylthio, isobutylthio, *tert*-butylthio, pentylthio, hexylthio *etc*.), amino, mono- or di-C1-6 alkylamino (*e*.*g*. methylamino, ethylamino, propylamino, isopropylamino, butylamino, isobutylamino, *tert*-butylamino, pentylamino, hexylamino, dimethylamino, diethylamino, dipropylamino, N-methyl-N-ethylamino *etc.),* halogen atom (fluorine, chlorine, bromine, iodine), cyano, nitro, carboxyl, straight-chain or branched-chain C1-6 alkoxycarbonyl (*e*.*g*. methoxycarbonyl, ethoxycarbonyl, *tert*-butoxycarbonyl *etc.*), straight-chain or branched-chain C1-6 alkylcarbonyloxy (*e*.*g*. acetoxy, ethylcarbonyloxy *etc*.), trihalomethyl (*e*.*g*. trifluoromethyl *etc*.), trihalomethoxy (*e*.*g*. trifluoromethoxy *etc*.), trihalomethylthio (*e*.*g*. trifluoromethylthio *etc*.), dihalomethylthio (*e*.*g*. difluoromethylthio *etc*.). oxo, carbocyclic ring (it has the same meanings as above described carbocyclic ring represented by ringA), heterocyclic ring (it has the same meanings as above described heterocyclic ring represented by ringA) and so on. These optional substituents may be substituted 1-4 at the replaceable position.

Heterocyclic ring in "(5) heterocyclic ring which may have a substituent(s)" as substituent has the same meanings as above described heterocyclic ring represented by ringA. Here, substituent of heterocyclic ring has the same meanings as above described substituent in "(4) carbocyclic ring which may have a substituent(s)".

Substituent in "(6) hydroxyl which may have a substituent(s)", "(7) mercapto which may have a substituent(s)" and "(8) amino which may have a substituent(s)" as substituent means, for example, alkyl which may have a substituent(s) (it has the same meanings as above described "(1) alkyl which may have a substituent(s)".), alkenyl which may have a substituent(s) (it has the same meanings as above described "(2) alkenyl which may have a substituent(s)".), alkynyl which may have a substituent(s) (it has the same meanings as above described "(3) alkynyl which may have a substituent(s)".), carbocyclic ring which may have a substituent(s) (it has the same meanings as above described "(4) carbocyclic ring which may have a substituent(s)".), heterocyclic ring which may have a substituent(s) (it has the same meanings as above described "(5) heterocyclic ring which may have a substituent(s)".), alkylsulfonyl (C1-4 alkylsulfonyl *etc.*, such as methylsulfonyl, ethylsulfonyl and so on.), heterocyclic ring sulfonyl (C6-10 heterocyclic ring sulfonyl *etc.*, such as phenylsulfonyl and so on.), acyl (it has the same meanings as below described "(26) acyl".) and so on. In addition, in case of "(8) amino which may have a substituent(s)", these optional substituents may be substituted 1-2 at the replaceable position.

Substituent in "(9) carbamoyl which may have a substituent(s)" and "(10) sulfamoyl which may have a substituent(s)" as substituent means, for example, alkyl which may have a substituent(s) (it has the same meanings as above described "(1) alkyl which may have a substituent(s)".), alkenyl which may have a substituent(s) (it has the same meanings as above described "(2) alkenyl which may have a substituent(s)".), alkynyl which may have a substituent(s) (it has the same meanings as above described "(3) alkynyl which may have a substituent(s)".), carbocyclic ring which may have a substituent(s) (it has the same meanings as above described "(4) carbocyclic ring which may have a substituent(s)".), heterocyclic ring which may have a substituent(s) (it has the same meanings as above described "(5) heterocyclic ring which may have a substituent(s)".) and so on. These optional substituents may be substituted 1-2 at the replaceable position.

Alkyl which may have a substituent(s) in "(12) (alkyl which may have a substituent(s)) oxycarbonyl" as substituent has the same meanings as above described "(1) alkyl which may have a substituent(s)".

"(26) acyl" as substituent means, for example, formyl, alkylcarbonyl which may have a substituent(s) (wherein alkyl which may have a substituent(s) has the same meanings as above described "(1) alkyl which may have a substituent(s)".), alkenylcarbonyl which may have a substituent(s) (wherein alkenyl which may have a substituent(s) has the same meanings as above described "(2) alkenyl which may have a substituent(s)".), alkynylcarbonyl which may have a substituent(s) (wherein alkynyl which may have a substituent(s) has the same meanings as above described "(3) alkynyl which may have a substituent(s)".), carbocyclic ring carbonyl which may have a substituent(s) (wherein carbocyclic ring which may have a substituent(s) has the same meanings as above described "(4) carbocyclic ring which may have a substituent(s)".), heterocyclic ring carbonyl which may have a substituent(s) (wherein heterocyclic ring which may have a substituent(s) has the same meanings as above described "(5) heterocyclic ring which may have a substituent(s)".) and so on.

In the specification, "alkyl which may have a substituent(s)" represented by Q has the same meanings as above described "(1) alkyl which may have a substituent(s)" of "substituent" in "cyclic group which may have a substituent(s)" represented by ringA. "Alkyl which may have a substituent(s)" may be together with "substituent" represented by below described E¹ and Y to form aziridine, azetidine, pyrrolidine, piperidine or perhydroazepine, which may have a substituent(s).

In the specification, "C3-10 mono- or bi-carbocyclic ring which may have a substituent(s)" represented by ringA means C3-10 mono-, bi-aromatic carbocyclic ring partially or fully saturated. C3-10 mono-, bi-aromatic carbocyclic ring partially or fully saturated means, for example, benzene, cyclopropane, cyclobutane, cyclopentane, cyclohexane, cycloheptane, cyclooctane, cyclononane, cyclodecane, cyclopentene, cyclohexene, cycloheptene, cyclooctene, cyclononene, cyclodecene, cyclopentadiene, cyclohexadiene, cycloheptadiene, cyclooctadiene, cyclononadiene, cyclodecadiene, cycloheptatriene, cyclooctatriene, cyclononatriene, cyclodecatriene, pentalene, indane, indene, naphthalene, azulene, perhydropentalene, perhydroindene, dihydronaphthalene, tetrahydronaphthalene, perhydronaphthalene, perhydroazulene and so on.

In the specification, "3-10 membered mono- or bi-heterocyclic ring containing 1 to 5 hetero atom(s) selected from oxygen atom(s), nitrogen atom(s) and sulfur atom(s), which may have a substituent(s)" represented by ringA means, for example, 3-10 membered mono-, or bi-aromatic heterocyclic ring optionally partially or fully saturated containing 1 to 5 hetero atom(s) selected from oxygen atom(s), nitrogen atom(s) and/or sulfur atom(s) and so on. 3-10 membered mono-, bi-aromatic heterocyclic ring optionally partially or fully saturated containing 1 to 5 hetero atom(s) selected from oxygen atom(s), nitrogen atom(s) and/or sulfur atom(s) means, for example, pyrrole, imidazole, triazole, tetrazole, pyrazole, pyridine, pyrazine, pyrimidine, pyridazine, azepine, diazepine, furan, pyran, oxepine, oxazepine, thiophene, thiain (thiopyran), thiepine, oxazole, isoxazole, thiazole, isothiazole, oxadiazole, oxazine, oxadiazine, oxazepine, oxadiazepine, thiadiazole, thiazine, thiadiazine, thiazepine, thiadiazepine, isoindole, isobenzofuran, isobenzothiophene, indazole, isoquinoline, phthalazine, naphthyridine, quinoxaline, quinazoline, cinnoline, benzoxazole, benzothiazole, benzimidazole, carbazole, acridine, indoloxazepine, indoloxadiazepine, indolothiazepine, indolothiadiazepine, indoloazepine, indolodiazepine, benzofurazan, benzotriazole, imidazothiazole, pyrroline, pyrrolidine, imidazoline, imidazolidine, triazoline, triazolidine, tetrazoline, tetrazolidine, dihydropyridine, dihydropyrazine, dihydropyrimidine, dihydropyridazine, piperidine, tetrahydropyridine, piperazine, tetrahydropyrimidine, tetrahydropyridazine, dihydrofuran, tetrahydrofuran, dihydropyran, tetrahydropyran, dihydrothiophene, tetrahydrothiophene, dihydrothiain (dihydrothiopyran), tetrahydrothiain (tetrahydrothiopyran), dihydrooxazole, tetrahydrooxazole, dihydroisoxazole, tetrahydroisoxazole, dihydrothiazole, tetrahydrothiazole, dihydroisothiazole, tetrahydroisothiazole, morpholine, thiomorpholine, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, dihydrobenzoxazole, perhydrobenzoxazole, dihydrobenzothiazole, perhydrobenzothiazole, dihydrobenzimidazole, perhydrobenzimidazole, dihydrobenzotriazole, camphor, tetrahydrocarbazole, perhydrocarbazole, dihydroacridine, tetrahyroacridine, perhydroacridine, dihydroimidazothiazole, perhydroimidazothiazole, 1,3-dioxaindane, 1,4-benzodioxane, quinuclidine, aziridine, dioxane, oxirane, thioxirane, azetidine, oxetane, thioxetane and so on.

In the specification, "3-10 membered mono-, bi-heterocyclic ring containing of 1 to 5 hetero atom(s) selected from oxygen atom(s), nitrogen atom(s) and sulfur atom(s), which may have a substituent(s)" represented by ringD has the same meanings as above described "3-10 membered mono-, bi-heterocyclic ring containing of 1 to 5 hetero atom(s) selected from oxygen atom(s), nitrogen atom(s) and sulfur atom(s), which may have a substituent(s)" represented by ringA.

In the specification, "substituent" of "pyridine or pyrimidine which may have a substituent(s)" represented by ringD has the same meanings as above described "substituent" in "cyclic group which may have a substituent(s)" represented by ringA. Said "substituent" may be together with above described "substituent" in "cyclic group which may have a substituent(s)" represented by ringA to form ethylene (-CH₂CH₂-).

In the specification, "single bond" represented by W means ringA and ringD directly binding without intervention of other atoms.

In the specification, "a spacer of which main chain has an atom number of 1-4" represented by W and Y means the distance that 1-4 atom(s) of main chain is(are) connected. Here, "atom number of main chain" is counted to be minimal. "A spacer of which main chain has an atom number of 1-4" means, for example, divalent group combining voluntarily 1-4 selected from methylene (-CH₂-) which may have 1 or 2 substituent(s), ethenylene (-CH=CH-) which may have 1 or 2 substituent(s), ethynylene (-CH≡CH-), nitrogen atom (-NH-) which may have a substituent, -CO-, -O-, -S-, -SO-and -SO₂- and so on. Here, substituent of methylene, ethenylene and nitrogen atom has the same meanings as describe above "substituent" in cyclic group which may have a substituent(s) represented by ringA. Concretely, it includes, for example, -CR¹⁰¹R¹⁰²-, -NE¹-, -CO-, -O-, -S-, -NE¹CO-, -CONE¹-, -NE¹CONE²-, -NE¹SO₂-, -SO₂NE¹-, -NR¹⁰³COCR¹⁰¹R¹⁰²-, -CONR¹⁰³CR¹⁰¹R¹⁰²- (wherein R¹⁰¹, R¹⁰², R¹⁰³, E¹ and E² are each independently hydrogen atom, or have the same meanings as above described "substituent" in "cyclic group which may have a substituent(s)" represented by ringA.) and so on.

In the specification, "hydrogen-bond acceptor site" in "a spacer of which main chain has an atom number of 1-4 containing of hydrogen-bond acceptor site" represented by Y means the group containing of atoms having unshared electron pair. It includes, for example, -CO-, -CS-, -C(=NR¹⁰³)-, -SO₂-, -SO-, NE¹- (wherein E¹ has the same meanings as above described.) and so on.

In the specification, substituents represented by R, R¹ and R³ each independently have the same meanings as above described "substituent" in "cyclic group which may have a substituent(s)" represented by ringA.

In the specification, substituents represented by E¹ and E² each independently have the same meanings as above described "substituent" in "cyclic group which may have a substituent(s)" represented by ringA. However, "substituent" represented by E¹ may be together with "alkyl which may have a substituent(s)" represented by Q and Y to form aziridine, azetidine, pyrrolidine, piperidine or perhydroazepine which may have a substituent(s) (said substituent has the same meanings as described above "substituent" in "cyclic group which may have a substituent(s)" represented by ringA.).

In the specification, halogen atom in C1-6 alkyl which is substituted with 1-5 halogen atom(s) represented by R² means fluorine atom, chlorine atom, bromine atom, iodine atom. C1-6 alkyl means straight-chain or branched-chain C1-6 alkyl *etc.,* such as methyl, ethyl, n-propyl, isopropyl, n-butyl, isobutyl, *sec*-butyl, *tert*-butyl, pentyl, hexyl and so on. As C1-6 alkyl which is substituted with 1-5 halogen atom(s), C1-2 alkyl which is substituted with 1-5 fluorine atom(s) is preferred. C1-2 alkyl which is substituted with 1-3 fluorine atom(s) is more preferred. Trifluoromethyl, difluoromethyl, 2,2,2-trifluoroethyl is particularly preferred.

As "cyclic group" in "cyclic group which may have a substituent(s)" represented by ringA, C3-10 mono- or bi-carbocyclic ring, or 3-10 membered mono- or bi-heterocyclic ring containing 1 to 5 hetero atom(s) selected from oxygen atom(s), nitrogen atom(s) and sulfur atom(s), which may have a substituent(s) is preferred. Benzene which may have a substituent(s), naphthalene or pyridine which may have a substituent(s) is more preferred. Benzene which may have a substituent(s) is particularly preferred.

As "cyclic group" in "cyclic group which may have a substituent(s)" represented by Q, C3-10 mono- or bi-carbocyclic ring, or 3-10 membered mono- or bi-heterocyclic ring containing 1 to 5 hetero atom(s) selected from oxygen atom(s), nitrogen atom(s) and sulfur atom(s), which may have a substituent(s) is preferred. Benzene which may have a substituent(s), thiophene which may have a substituent(s) is more preferred.

As "alkyl which may have a substituent(s)" represented by Q, C1-8 alkyl which may be substituted by 1-5 R⁴(s) (wherein R⁴ means, for example, halogen atom, phenyl, phenoxy, benzyloxy, hydroxyl and so on.) is preferred. Methyl, trifluoromethyl, ethyl, isopropyl, benzyl, phenoxymethyl, or benzyloxymethyl is more preferred.

As "cyclic group" in "cyclic group which may have a substituent(s)" represented by ringD, 3-10 membered mono-heterocyclic ring containing 1 to 5 hetero atom(s) selected from oxygen atom(s), nitrogen atom(s) and sulfur atom(s) is preferred. Pyrimidine or pyridine is more preferred. Pyrimidine is particularly preferred.

As "substituent" in "cyclic group which may have a substituent(s)" represented by ringA, ringD or Q, or "substituent" represented by R, R¹, R³, 1-5 substituent(s) selected voluntarily from C1-8 alkyl, C2-8 alkenyl, C2-8 alkynyl, C1-8 alkoxy or C1-8 alkylthio, which may be substituted with 1-5 R⁵(s) (wherein C1-8 alkoxy means, for example, methoxy, ethoxy, n-propoxy, ispropoxy, n-butoxy, isobutoxy, *sec*-butoxy, *tert*-butoxy, pentyloxy, hexyloxy, heptyloxy, octyloxy *etc*., C1-8 alkylthio means, for example, methylthio, ethylthio, n-propylthio, isopropylthio, n-butylthio, isobutylthio, *sec*-butylthio, *tert*-butylthio, pentylthio, hexylthio, heptylthio, octylthio *etc.,* R⁵ means, for example, hydroxyl or halogen atom *etc*.), carbocyclic ring, heterocyclic ring, hydroxyl, mercapto, amino, NR⁶R⁷ (wherein R⁶ and R⁷ are each independently hydrogen atom, C1-8 alkyl or acetyl.), carboxyl, C1-6 alkoxycarbonyl, nitro, cyano, halogen atom, C1-6 acyl (*e*.*g*. formyl, ethanoyl, propanoyl, butanoyl, pentanoyl, hexanoyl *etc*.) and oxo is(are) preferred. 1-3 substituent(s) selected voluntarily from methyl, trifluoromethyl, 2,2,2-trifluoroethyl, cyano, methoxy, trifluoromethoxy, difluoromethoxy, 2,2,2-trifluoroethoxy, isopropoxy, nitro, halogen atom and acetylamino is(are) more preferred.

As W, single bond is preferred.

As Y, a spacer of which main chain has an atom number of 1-4 containing of hydrogen-bond acceptor site is preferred. 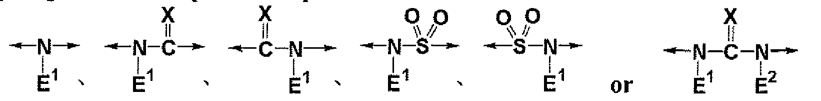 (wherein E¹ and E² are each independently a hydrogen atom(s) or a substituent(s), X is an oxygen atom or a sulfur atom, left-pointing arrow binds to ringD, right-pointing arrow binds to Q) is more preferred. 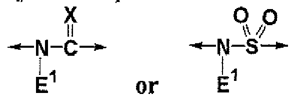 (wherein all symbols have the same meanings as described above.) is particularly preferred.

As Z, nitrogen atom is preferred.

As R, hydrogen atom is preferred.

As X, oxygen atom is preferred.

As E¹, hydrogen atom, methyl, ethyl, propyl, isobutyl, 2-methoxyethyl, benzyl, benzoyl or thiophen-2-ylcarbonyl is preferred.

As E², hydrogen atom is preferred.

Among the compounds represented by formula (I), preferable compounds include, for example, the compound represented by formula (Ia) 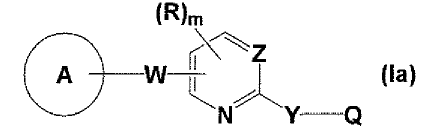 (wherein all symbols have the same meanings as described above.), the compound represented by formula (Ib) 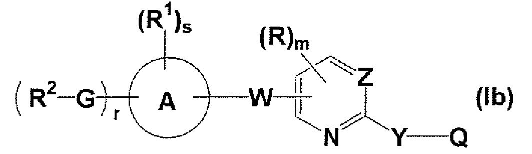 (wherein all symbols have the same meanings as described above.), a salt thereof, an N-oxide thereof or a solvate thereof, or a prodrug thereof

Among the compounds represented by formula (Ia), preferable compounds include, for example, the compound represented by formula (Ia-1) 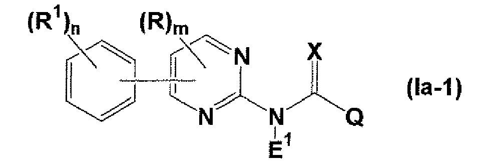 (wherein n is an integer of 1 to 5, the other symbols have the same meanings as described above.), the compound represented by formula (Ia-2) 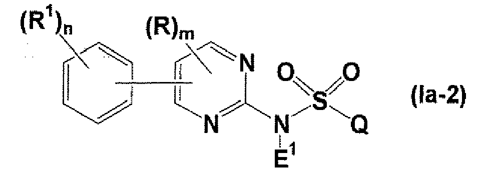 (wherein all symbols have the same meanings as described above.), a salt thereof, an N-oxide thereof or a solvate thereof, or a prodrug thereof.

Among the compounds represented by formula (Ia-1), preferable compounds include, for example, the compound represented by formula (Ia-1-1) 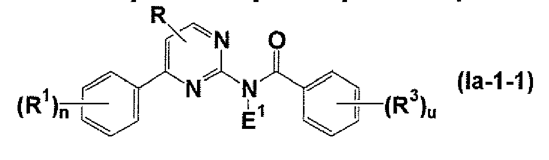 (wherein R³ is hydrogen atom or substituent, u is an integer of 1 to 5, the other symbols have the same meanings as described above.), the compound represented by formula (Ia-1-2) 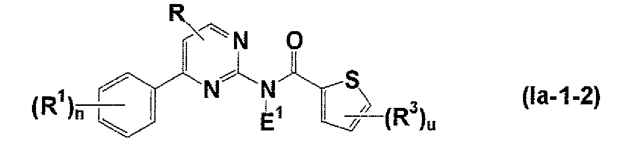 (wherein all symbols have the same meanings as described above.), a salt thereof, an N-oxide thereof or a solvate thereof, or a prodrug thereof.

Among the compounds represented by formula (Ia-2), preferable compounds include, for example, the compound represented by formula (Ia-2-1) 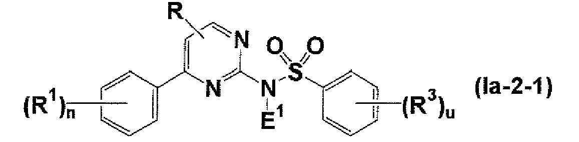 (wherein all symbols have the same meanings as described above.), the compound represented by formula (Ia-2-2) 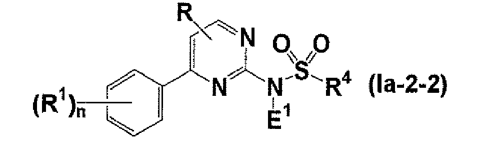 (wherein R⁴ has the same meanings as above described "alkyl which may have a substituent(s)" represented by Q, the other symbols have the same meanings as described above.), the compound represented by formula (Ia-2-3) 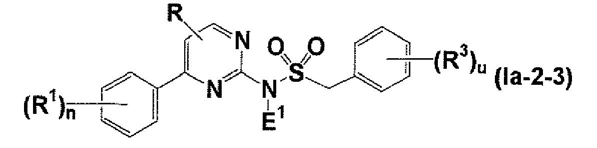 (wherein all symbols have the same meanings as described above.), a salt thereof, an N-oxide thereof or a solvate thereof, or a prodrug thereof.

Among the compounds represented by formula (Ib), preferable compounds include, for example, the compound represented by formula (Ib-1) 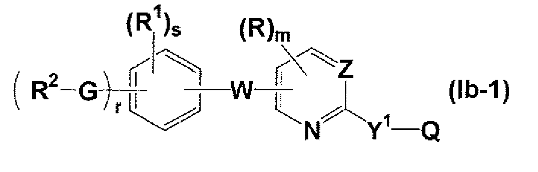 (wherein all symbols have the same meanings as described above.), the compound represented by formula (Ib-2) 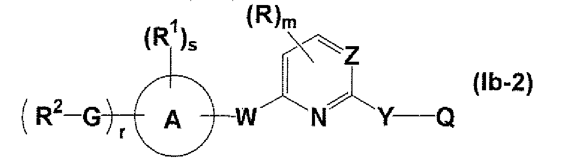 (wherein all symbols have the same meanings as described above.), a salt thereof an N-oxide thereof or a solvate thereof, or a prodrug thereof

Among the compounds represented by formula (IB-1), preferable compounds include, for example, the compound represented by formula (Ib-1-1) 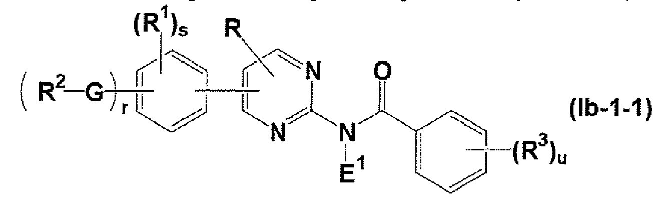 (wherein all symbols have the same meanings as described above.), the compound represented by formula (Ib-1-2) 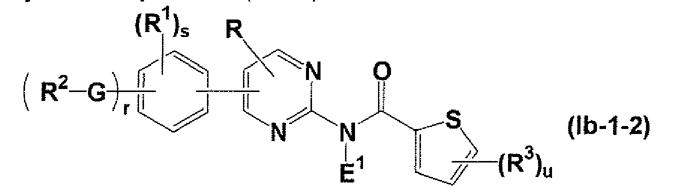 (wherein all symbols have the same meanings as described above.), the compound represented by formula (Ib-1-3) 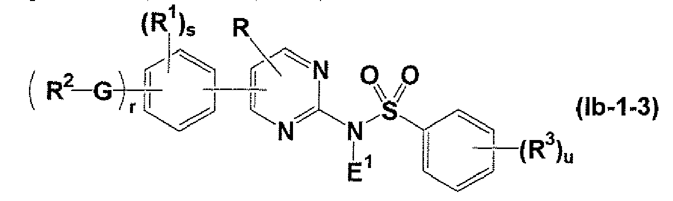 (wherein all symbols have the same meanings as described above.), the compound represented by formula (Ib-1-4) 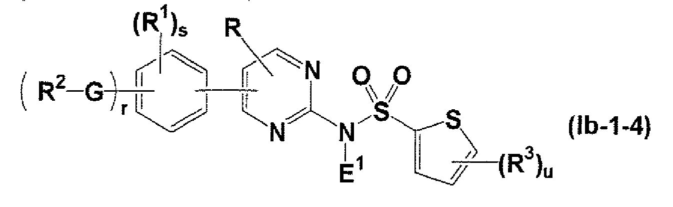 (wherein all symbols have the same meanings as described above.), a salt thereof, an N-oxide thereof or a solvate thereof, or a prodrug thereof.

Among the compounds represented by formula (Ib-2), preferable compounds include, for example, the compound represented by formula (Ib-2-1) 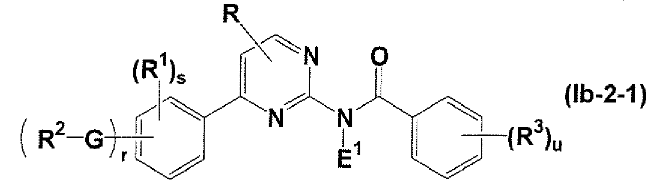 (wherein all symbols have the same meanings as described above.), the compound represented by formula (Ib-2-2) 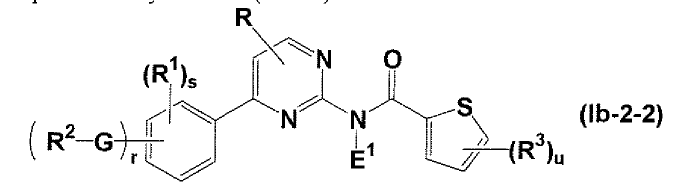 (wherein all symbols have the same meanings as described above.), the compound represented by formula (Ib-2-3) 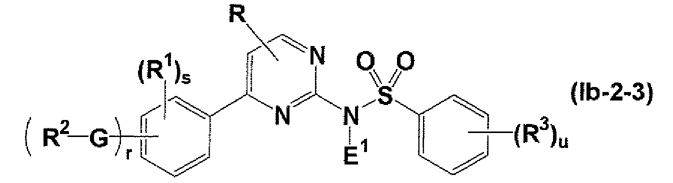 (wherein all symbols have the same meanings as described above.), the compound represented by formula (Ib-2-4) 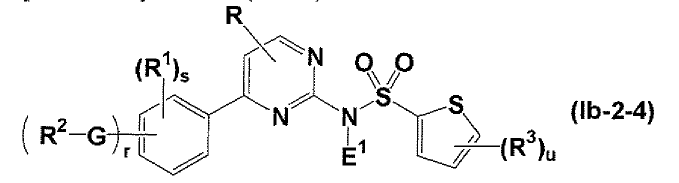 (wherein all symbols have the same meanings as described above.), a salt thereof, an N-oxide thereof or a solvate thereof, or a prodrug thereof.

Among the compounds represented by formula (I), the compound represented by formula (Ic) 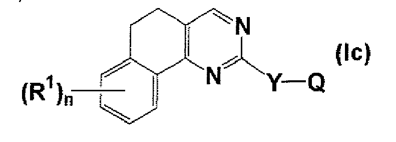 (wherein all symbols have the same meanings as described above.), the compound represented by formula (Id) 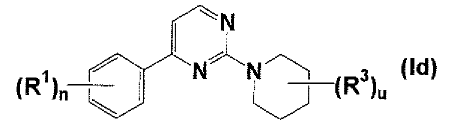 (wherein all symbols have the same meanings as described above.), a salt thereof, an N-oxide thereof or a solvate thereof, or a prodrug thereof is preferred.

More preferable compounds include all compounds of the present invention showed in Examples.

Preferable compounds for preparing the composition of a preventive and/or therapeutic agent for a mitochondrial benzodiazepine receptor mediated disease include all compounds of the present invention showed in Examples and the compounds showed below (1)-(16).
(1) N-{4-[2,5-bis(2,2,2-trifluoroethoxy)phenyl]pyrimidin-2-yl}thiophene-2-carboxamide,
(2) N-(4-pyridin-2-ylpyrirnidin-2-yl)benzamide,
(3) N-{5-(methylsulfonyl)-4-[4-(trifluoromethyl)phenyl]pyrimidin-2-yl}acetamide,
(4) N-[4-(2,3-dihydro-1,4-benzodioxin-6-yl)pyrimidin-2-yl]-4-methylbenzamide,
(5) N-[4-(5-chloro-3-methyl-1-benzothien-2-yl)pyrimidin-2-yl]benzamide,
(6) 2,2,2-trifluoro-N-[4-(2-methylimidazo[1,2-a]pyridin-3-yl)pyrimidin-2-yl]acetamide,
(7) N-[4-(2-methylimidazo[1,2-a]pyridin-3-yl)pyrimidin-2-yl]acetoamide,
(8) 4-chloro-N-(5,6-dihydrobenzo[h]quinazolin-2-yl)benzamide,
(9) N-(5,6-dihydrobenzo[h]quinazolin-2-yl)-4-methoxybenzamide,
(10) N-(5,6-dihydrobenzo[h]quinazolin-2-yl)-3-(trifluoromethyl)benzamide,
(11) N-(5,6-dihydrobenzo[h]quinazolin-2-yl)-2-[(4-methylphenyl)sulfanyl]acetamide,
(12) N-(5,6-dihydrobenzo[h]quinazolin-2-yl)-2-(propylsulfanyl)acetamide,
(13) N-(5,6-dihydrobenzo[h]quinazolin-2-yl)-2-(isopropylsulfanyl)acetamide,
(14) 4-amino-N-[4-(4-fluorophenyl)pyrimidin-2-yl]benzenesulfonamide,
(15) N-[4-(3-methylpyrazin-2-yl)pyrimidin-2-yl]benzenesulfonamide and
(16) 4-amino-N-(4-thien-2-ylpyrimidin-2-yl)benzenesulfonamide.

### [Isomer]

Unless otherwise specified, all isomers are included in the present invention. For example, alkyl, alkenyl, alkynyl, alkoxy, alkylthio, alkylene, alkenylene, alkynylene, alkylidene and alkenylidene group means straight-chain or branched-chain ones. In addition, isomers on double bond, ring, fused ring (E-, Z-, cis-, trans-isomer), isomers generated from asymmetric carbon atom(s) (R-, S-isomer, α-, β-configuration, enantiomer, diastereomer), optically active isomers (D-, L-, d-, 1-isomer), polar compounds generated by chromatographic separation (more polar compound, less polar compound), equilibrium compounds, rotational isomers, mixtures thereof at voluntary ratios and racemic mixtures are also included in the present invention.

### [Salt, N-oxide and solvate]

The salts of the compounds represented by formula (I) include all of pharmaceutically acceptable ones. As pharmaceutically salts, non-toxic, water-soluble salts are preferred. The suitable salts include for example, salts of alkali metals (*e.g.*, potassium, sodium, lithium, *etc.*), salts of alkaline earth metals (*e.g.*, calcium, magnesium, *etc*.), ammonium salts (*e*.*g*., tetramethylammonium salt, tetrabutylammonium salt, *etc*.), pharmaceutical acceptable salts of organic amine *(e.g.,* triethylamine, methylamine, dimethylamine, cyclopentylamine, benzylamine, phenethylamine, piperidine, monoethanolamine, diethanolamine, tris(hydroxymethyl)methylamine, lysine, arginine, N-methyl-D-glucamine, *etc*.), acid addition salts (salts of inorganic acids (*e*.*g*., hydrochloride, hydrobromide, hydroiodide, sulfate, phosphate, nitrate *etc.),* and salts of organic acids (*e*.*g*., acetate, trifluoroacetate, lactate, tartrate, oxalate, fumarate, maleate, benzoate, citrate, methanesulfonate, ethanesulfonate, benzenesulfonate, toluenesulfonate, isethionate, glucuronate, gluconate *etc*.).

In addition, N-oxide of the compound represented by formula (I) means nitrogen atom of the compound represented by formula (I) is oxidized. The N-oxide of the compound in the present invention may be the above-mentioned salts of alkali (earth) metals, ammonium salts, salts of organic amine, acid addition salts and so on.

The suitable solvates of the compound represented by formula (I) include for example, hydrates, solvates of the alcohols (*e.g.*, ethanol *etc.),* and so on. The solvates are preferably nontoxic and water-soluble. In addition, the solvate of the compound in the present invention included the solvate of salts of alkali (earth) metals, ammonium salts, salts of organic amine, acid addition salts, N-oxide and so on of the above-mentioned compound of the present invention.

The compound of the present invention may be converted into the above-mentioned salt, the above-mentioned N-oxide, the above-mentioned solvates by known methods.

### [Prodrug]

The prodrug of the compounds represented by formula (I) means a compound is the compound represented by formula (I) by reaction with enzymes, gastric acids and so on within an organism. The prodrug of the compounds represented by formula (I) include, when the compounds represented by formula (I) have amino, the prodrug is the compounds the amino of which is acylated, alkylated, phosphorylated (*e.g.*, the compounds are that the amino of the compounds represented by formula (I) is eicosanoated, alanylated, pentylaminocarbonylated, (5-methyl-2-oxo-1,3-dioxolan-4-yl)methoxycarbonylated, tetrahydrofuranated, pyrrolidylmethylated, pivaloyloxymethylated, acetoxymethylated, tert-butylated, *etc*.); when the compounds represented by formula (I) have hydroxyl, the prodrug is the compounds the hydroxyl of which are acylated, alkylated, phosphorylated, borated (*e.g.*, the compounds are that the hydroxyl of the compounds represented by formula (I) are acetylated, palmitoylated, propanoylated, pivaloylated, succinylated, fumarylated, alanylated, dimethylaminomethylcarbonylated *etc.);* when the compounds represented by formula (I) have carboxyl, the prodrug is the compound the carboxyl of which are esterified, amidated (*e*.*g*., the compounds are that the carboxyl of the compounds represented by formula (I) is ethylesterified, phenylesterified, carboxymethylesterified, dimethylaminomethylesterified, pivaloyloxymethylesterified, ethoxycarbonyloxyethylesterified, phthalidylesterified, (5-methyl-2-oxo-1,3-dioxolen-4-yl)methylesterified, cyclohexyloxycarbonylethylesterified, methylamidated *etc*.); and so on. These compounds can be prepared by known methods. In addition, the prodrug of the compound represented by formula (I) may be either hydrate or non-hydrate. In addition, the prodrug of the compound represented by formula (I) may be converted into the compound represented by formula (I) under the physiological condition which is described in "the development of medicine" vol.7 "molecular design" published in 1991 Hirokawa shoten p.p. 163-198. Further, the compound represented by formula (I) may be labeled with isotopes (*e*.*g*. ³H, ¹⁴C, ³⁵S, ¹²⁵I *etc.)* and so on.

### [Pharmacological activity]

As pharmacological test other than one described in Examples, for example, there are the methods as follows.
Determination of pregnenolone in rat adrenocortical mitochondria:
The steroid productivity of the compound in the present invention can be evaluated using rat adrenocortical mitochondria.
After intraperitoneal administration of 20mg/mL cycloheximide solution (1 mL) to male SD rats, 101 U/mL adrenocorticotropic hormone (ACTH) solution (0.3 mL) is intraperitoneally administered to them in five minutes. 20 minutes after ACTH administration, the rats are sacrificed by cervical dislocation and bilateral adrenal cortexes are removed at once. The removed adrenal cortexes are homogenized in buffer A (composition: 50 mmol/L Tris-HCl; 250 mmol/L sucrose) and then the suspension is centrifuged at 2000g for 3 minutes at 4°C. The obtained supernatant is centrifuged at 12500 g for 10 minutes at 4°C. The pellet is washed with buffer A twice and suspended in buffer B (composition: 250 mmol/L sucrose; 10 mmol/L potassium phosphate buffer; 15 mmol/L triethanolamine; 20 mmol/L potassium chloride; 5 mmol/L magnesium chloride; 10 µmol/L trilostane; 10 µmol/L SU10603) for experiments. Assay buffer which includes malic acid (150 mmol/L), β-NADP⁺ (5 mmol/L) and the compound in the present invention is incubated for 5 minutes at 37°C. Then, crude mitochondrial membrane fraction derived from rat adrenal cortex is added and further incubated for 10 minutes at 37°C to produce pregnenolone (final concentration of the compound: 1 µmol/L). After incubation, the reaction is terminated by addition of ethanol, extracted by addition of n-hexane and then evaporated to dryness. The residue is dissolved in buffer C (composition: 0.1% gelatin; phosphate buffered salts solution), centrifuged and then the collected supernatant is determined as samples for measurement. [³H] pregnenolone (10000 cpm; 100 µL), anti-pregnenolone antibody (ICN Biomedicals Inc; 100 µL) and sample (100 µL) are mixed and incubated overnight at 4°C. After the reaction, the mixture is added by dextran/charcoal (200 µL), mixed well, kept on ice for 10 minutes and then centrifuged. The radioactivity of the supernatant is measured by liquid scintillation counter. The pregnenolone in the sample is calculated from the standard curve,
Effect of the compound in the present invention on increase in pregnenolon content in the brain by loading stressor:
It can be confirmed that MBR antagonist can inhibit steroid production in the brain, as follows.
Male Wistar rats are loaded with psychological stressor *(Brain Res.,* 641, 21-28 (1994)). Water is stored up to about 10 cm depth in a container of which the platform is set up at the center. Rats in the non-treated group are loaded without administration and stressor. In contrast, rats in the stressor loaded group are orally administered with the vehicle or the compounds and 30 minutes later the rats are put on the platform to be loaded with stressor. One hour later from starting to load, the rats are irradiated by microwave (output: about 6.5 kW, exposure time: 0.96s) using microwave applicator (Muromachi Kikai Co., Ltd.) and then the bilateral hippocampuses are removed and weighed. The hippocampuses are added by internal standard substance (D₄-pregnenolone 20 ng), water (1 mL) and diethylether/n-hexane (9:1, 3 mL) and stirred. The mixture is crushed by ultrasonic waves, stirred again, centrifuged at 3000 rpm for 5 minutes and the organic layer is transferred to new tube with Pasteur pipet. The water phase is extracted with diethylether/n-hexane (9:1, 3 mL) again and the organic layer is mixed to the above-mentioned extract. After reduced pressure to dryness, the residue is dissolved with 150 µL water/acetonitrile (1:9) again and measured by liquid chromatography/mass spectrometry (LC-MS). The measurement condition is shown as follows.

| | |
|---|---|
| LC (Liquid chromatography): | Hewlett Packard series 1100, |
| Column: | Inertsil ODS-3, 3 µm, 2,1^{ψ} × 100 mm, |
| Temperature: | room temperature, |
| Mobile phase: | 5 mmol/L CH₃CO₂NH₄/MeCN (10:90), |
| Flow rate: | 0.2 mL/min, |
| Injection volume: | 40 µL, |
| MS (Micro spectrometry): | Quattoro II (Micromass), |
| Ionization mode: | Atmosphere Pressure Chemical Ionization (APCI), |
| positive; Corona: | 3.4 kV, |
| Sheath gas: | N₂ (50 L/hr), |
| Source temperature: | 180°C, |
| Probe temperature: | 550°C, |
| Detection: Pregnenolone: | m/z 317.2 (cone: 10V), |
| D₄-pregnenolone: | m/z 321.2 (cone: 10V). |

### [Processes for the preparation of the compound in the present invention]

The compound in the present invention represented by formula (I) can be prepared by combining the known processes, for example, the following processes or the processes shown in Examples, which is the properly improved processes described in "*Comprehensive Organic Transformations: A Guide to Functional Group Preparations,* 2nd Edition", "Richard C. Larock, John Wiley & Sons Inc, 1999". Still, ingredients may be used as salts in the following each processes for the preparation. As these salts, the salts described as the pharmaceutically acceptable ones in the above-mentioned formula (I) can be used.
[A] Among the compounds represented by formula (I), the compound, wherein Y is -N(E¹)-C(=X)-, that is, the compound represented by formula (I-A) 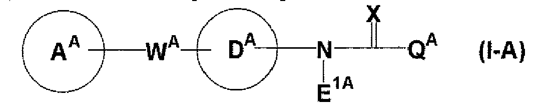 (wherein A^{A}, Q^{A}, D^{A}, E^{1A} and W^{A} have the same meanings as above described A, Q, D, E¹ and W, respectively. But carboxyl, hydroxyl, amino or mercapto included the group represented by A^{A}, Q^{A}, D^{A}, E^{1A} and W^{A} are, if necessary, protected. The other symbols have the same meanings as described above.) can be prepared by following [1], [2] or [3].
   [1] The compound represented by formula (I-A) can be prepared by treating with amidation or thioamidation of the compound represented by formula (II) 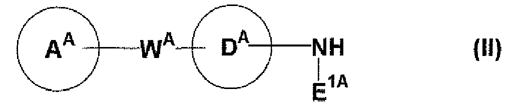 (wherein all symbols have the same meanings as described above.) with the compound represented by formula (III) 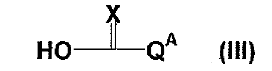 (wherein all symbols have the same meanings as described above.), if necessary, followed by subjecting to a deprotection reaction of protection group.

The reaction with the compound represented by formula (II) and the compound represented by formula (III) is carried out, for example, by the method (1) using acid halide, (2) using mixed acid anhydride, (3) using condensing agent *etc.*

These methods are explained concretely as follows.
(1) The method using acid halide is carried out, for example by reacting the compound represented by formula (III) in an organic solvent (e.g. chloroform, dichloroform, diethylether, tetrahydrofuran, dimethoxyethane *etc*.) or the absence of solvent, with acid halide agent (*e*.*g*. oxalylchloride, thionylchloride *etc*.) at the temperature from -20°C to reflux temperature, and reacting the obtained acid halide with the compound represented by formula (II) in a organic solvent (*e.g.* chloroform, dichloromethane, diethylether, tetrahydrofuran, acetonitrile, ethyl acetate *etc*.) under the presence of a base (*e.g.* pyridine, triethylamine, dimethylaniline, dimethylaminopyridine, diisopropylethylamine *etc.)* at the temperature of from -20°C to reflux temperature. In addition, it is carried out by reacting the obtained halide with the compound represented by formula (II) in an organic solvent (*e.g.* dioxane, tetrahydrofuran, dichloromethane *etc.),* under the presence or absence of phase-transfer catalyst (*e*.*g*. quaternary ammonium salt *etc.,* for example, tetrabutylammoniumchloride, triethylbenzylammoniumchloride, tri-n-octylmethylammoniumchloride, trimethyldecylammoniumchloride, tetramethylammoniumbromide and so on.), using alkaline solution (*e.g.* sodium bicarbonate or sodium hydroxide solution *etc.)* at the temperature from -20°C to reflux temperature.
(2) The method using mixed acid anhydride is carried out, for example the compound represented by formula (III) with an acid halide (*e*.*g*. pivaloyl chloride, tosyl chloride, mesyl chloride *etc.),* or an acid derivative (*e.g.* chloroethyl formate, chloroisobutyl formate *etc*.) in an organic solvent (*e.g.* chloroform, dichloromethane, diethylether, tetrahydrofuran *etc.*) or the absence of solvent, under the presence of a base (*e.g.* pyridine, triethylamine, dimethylaniline, dimethylaminopyridine, diisopropylethylamine *etc.)* at the temperature from -20°C to reflux temperature, and by reacting the obtained mixed acid anhydride with the compound represented by formula (II) in an organic solvent (*e*.*g*. chloroform, dichloromethane, diethylether, tetrahydrofuran *etc.*) at the temperature from -20°C to reflux temperature.
(3) The method using condensing agent is carried out, for example by reacting the compound represented by formula (III) with the compound represented by formula (II) in an organic solvent (*e*.*g*. chloroform, dichloromethane, dimethylformamide, diethylether, tetrahydrofuran *etc*.), or in the absence of solvent, under the presence or the absence of a base (*e*.*g*. pyridine, triethylamine, dimethylaniline, dimethylaminopyridine *etc*.) using the condensing agent (*e*.*g*. 1,3-dicyclohexylcarbodiimide (DCC), 1-ethyl-3-[3-(dimethylamino)propyl]carbodiimide (EDC), 1,1'-carbonyldiimidazole (CDI), 2-chloro-1-methylpyridinium iodine, 1-propanephosphonic acid cyclic anhydride (PPA) *etc*.), using or not using 1-hydroxybenztriazole (HOBt) at the temperature from -20°C to reflux temperature.

These reactions (1), (2) and (3) are all preferably carried out under the anhydrous condition in the presence of inert gases (argon, nitrogen *etc*.).

The deprotection reaction of a protective group for carboxyl, hydroxyl, amino, or mercapto is known, and it includes
(1) alkaline hydrolysis,
(2) deprotection reaction under acidic conditions,
(3) deprotection reaction by hydrogenolysis,
(4) deprotection reaction of a silyl group,
(5) deprotection reaction using metals,
(6) deprotection reaction using metal complexes, and so on.

These methods are described concretely as follows.
(1) The deprotection reaction by alkaline hydrolysis is, for example, carried out in an organic solvent (*e*.*g*., methanol, tetrahydrofuran, or dioxane *etc*.) using a hydroxide of an alkali metal (*e*.*g*., sodium hydroxide, potassium hydroxide, or lithium hydroxide *etc*.), a hydroxide alkaline earth metal (*e*.*g*., barium hydroxide, or calcium hydroxide *etc*.), or a carbonate (*e*.*g*., sodium carbonate or potassium carbonate, *etc*.), or an aqueous solution thereof, or a mixture thereof at a temperature of 0 to 40°C.
(2) The deprotection reaction under acidic conditions is carried out, for example, in an organic solvent (*e*.*g*., dichloromethane, chloroform, dioxane, ethyl acetate, or anisole *etc*.) in an organic acid *(e.g.,* acetic acid, trifuloroacetic acid, methansulfonic acid, or p-tosylate, *etc*.), or an inorganic acid *(e.g.,* hydrochloric acid, or sulfuric acid, *etc.)* or a mixture thereof *(e.g.,* hydrogen bromide/acetic acid, *etc*.) at a temperature of 0 to 100°C.
(3) The deprotection reaction by hydrogenolysis is carried out, for example, in a solvent (*e*.*g*., ethers (*e*.*g*., tetrahydrofuran, dioxane, dimethoxyethane (DME), or diethylether, *etc*.), alcohols (*e*.*g*., methanol, or ethanol, *etc*.), benzenes (*e*.*g*., benzene, or toluene *etc*.), ketones (*e*.*g*., acetone, or methylethylketone, *etc*.), nitriles (*e*.*g*., actetonitrile *etc*.), amides (*e*.*g*., DMF *etc*.), water, ethyl acetate, acetic acid, or a mixed solvent of at least two of these *etc*.) in the presence of a catalyst (*e*.*g*., palladium-carbon, palladium black, palladium hydroxide-carbon, platinum oxide, or Raney nickel, *etc.*) under the hydrogen atmosphere at normal pressure or under pressurization, or in the presence of ammonium formate at a temperature of 0 to 200°C.
(4) The deprotection reaction of a silyl group is carried out, for example, in a water-miscible organic solvent (*e*.*g*., tetrahydrofuran, or acetonitrile, *etc*.) using tetrabutylammonium fluoride at a temperature of 0 to 40°C.
(5) The deprotection reaction using metals is carried out, for example, in an acidic solvent (*e*.*g*., acetic acid, pH 4.2-7.2 buffer solution, or a mixture of a solution thereof and an organic solvent of tetrahydrofran *etc*.) in the presence of zinc powder, if necessary sonicating, at the temperature of 0 to 40°C.
(6) The deprotection reaction using metal complexes is carried out, for example, in an organic solvent (*e*.*g*., dichloro methane, DMF, THF, ethyl acetate, acetonitrile, dioxane, ethanol *etc*.), water, or a mixture thereof, in the presence of a trap reagent *(e.g.,* tributyltine hydride, triethylsilane, dimedone, morpholine, diethylamine, pyrrolidine, *etc.),* an organic acid *(e.g.,* acetic acid, formic acid, 2-ethylhexanoic acid, *etc.)* and/or salts of organic acid *(e.g.,* sodium 2-ethylhexanoate, potassium 2-ethylhexanoate *etc.),* in the presence or absence of a phosphine reagent *(e.g.,* triphenylphosphine *etc*.), using metal complexes (*e*.*g*., tetrakistriphenylphosphinepalladium(0), dichlorobis(triphenylphosphine)palladium(II), palladium acetate(II), tris(triphenylphosphine)rhodium(I) chloride *etc*.) at the temperature of 0 to 40°C.

In addition, the deprotection reaction except the above-mentioned processes can be carried out, for example, by the process described in T.W. Greene, *Protective Groups in Organic Synthesis*, Wiley, New York, 1999.

The protection group for carboxyl includes, for example, methyl, ethyl, allyl, t-butyl, trichloroethyl, benzyl (Bn), phenacyl, p-methoxybenzyl, trityl, 2-chlorotrityl or structure thereof bound to solid phase carrier and so on.

The protection group for hydroxyl includes, for example, methyl, trytyl, methoxymethyl (MOM), 1-ethoxyethyl (EE), methoxyethoxymethyl (MEM), 2-tetrahydropyranyl (THP), trimethylsilyl (TMS), triethylsilyl (TES), t-butyldimethylsilyl (TBDMS), t-butyldiphenylsilyl (TBDPS), acetyl (Ac), pivaloyl, benzoyl, benzyl (Bn), p-methoxybenzyl, allyloxycarbonyl (Alloc), 2,2,2-trichloroethoxycarbonyl (Troc), and so on.

The protection group of amino includes benzyloxycarbonyl, t-butoxycarbonyl, allyloxycarbonyl (Alloc), 1-methyl-1-(4-biphenyl) ethoxycarbonyl (Bpoc), trifluoroacetyl, 9-fluorenylmethoxycarbonyl, benzyl (Bn), p-methoxybenzyl, benzyloxymethyl (BOM), 2-(trimethylsilyl) ethoxymethyl (SEM) and so on.

The protection group of mercapto includes, for example, benzyl, methoxybenzyl, methoxymethyl (MOM), 2-tetrahydropyranyl (THP), diphenylmethyl, acetyl (Ac) and so on.

The protective group for carboxyl, hydroxyl, amino or mercapto is not particularly limited to the above mentioned groups, so long as it can be easily and selectively left. For example, those described in T.W. Greene, *Protective Groups in Organic Synthesis,* Wiley, New York, 1999 can be used.

As will be easily understood by those skilled in the art, the intended compounds in the present invention can be easily prepared by choosing these deprotection reactions.
[2] The compound represented by formula (I-A) can be prepared by reacting the compound represented by formula (II) with the compound represented by formula (IV) 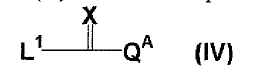 (wherein L¹ is deprotection group such as halogen atom, imidazolyl *etc*., the other symbols have the same meanings as described above.), if necessary, followed by subjecting to a deprotection reaction of protection group.

The reaction with the compound represented by formula (II) and the compound represented by formula (IV) is carried out, for example, by reacting the compound represented by formula (IV) with the compound represented by formula (II) in an organic solvent (*e*.*g*. chloroform, dichloromethane, diethylether, tetrahydrofuran, acetonitrile, ethyl acetate *etc*.) under the presence of a base (*e*.*g*. pyridine, triethylamine, dimethylaniline, dimethylaminopyridine, diisopropylethylamine *etc*.) at a temperature from -20°C to reflux temperature. In addition, the reaction can be carried out by reacting the compound represented by formula (IV) with the compound represented by formula (II) in an organic solvent (*e*.*g*. dioxane, tetrahydrofuran, dichloromethane *etc*.) using alkali aqueous solution (*e.g.* sodium bicarbonate water, sodium hydroxide aqueous solution *etc.)* in the presence or absence of phase-transfer catalyst (*e.g.* quaternary ammonium salt such as tetrabutylammonium chloride, triethylbenzylammonium chloride, tri-n-octylmethylammonium chloride, trimethyldecylammonium chloride, tetramethylammonium bromide *etc*.) at a temperature from 0 to 40°C.

The deprotection reaction of the protective group can be carried out by above described method.
[3] The compound represented by formula (I-A) can be prepared by reacting the compound represented by formula (V) 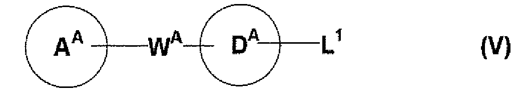 (wherein all symbols have the same meanings as described above.) with the compound represented by formula (VI) 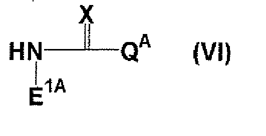 (wherein all symbols have the same meanings as described above.), if necessary, followed by subjecting to a deprotection reaction of protection group.

The reaction with the compound represented by formula (V) and the compound represented by formula (VI) can be carried out by pursuant method of the above described reaction with the compound represented by formula (II) and the compound represented by formula (IV).
[B] Among the compounds represented by formula (I), the compound, wherein Y is -N(E¹)-SO₂-, that is, the compound represented by formula (I-B) 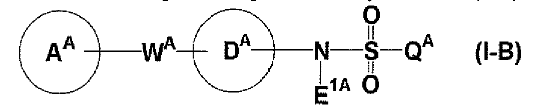 (wherein all symbols have the same meanings as described above.) can be prepared by following [1], [2] or [3].
   [1] The compound represented by formula (I-B) can be prepared by reacting the compound represented by formula (II) with the compound represented by formula (VII) 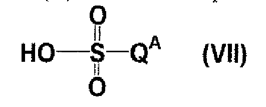 (wherein all symbols have the same meanings as described above.), if necessary, followed by subjecting to a deprotection reaction of protection group.

The reaction with the compound represented by formula (II) and the compound represented by formula (VII) is carried out, for example, by the method (1) using acid halide, (2) using mixed acid anhydride, (3) using condensing agent *etc*. These methods can be carried out by pursuant method of above described.

The deprotection reaction of the protective group can be carried out by above described method.
[2] The compound represented by formula (I-B) can be prepared by reacting the compound represented by formula (II) with the compound represented by formula (VIII) 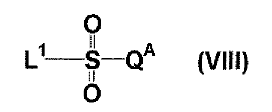 (wherein all symbols have the same meanings as described above.), if necessary, followed by subjecting to a deprotection reaction of protection group.

The reaction with the compound represented by formula (II) and the compound represented by formula (VIII) can be carried out by pursuant method of the above described reaction with the compound represented by formula (II) and the compound represented by formula (IV).

The deprotection reaction of the protective group can be carried out by above described method.
[3] The compound represented by formula (I-B) can be prepared by reacting the compound represented by formula (V) with the compound represented by formula (IX) 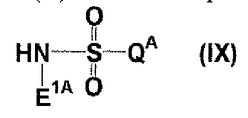 (wherein all symbols have the same meanings as described above.), if necessary, followed by subjecting to a deprotection reaction of protection group.

The reaction with the compound represented by formula (V) and the compound represented by formula (IX) can be carried out by pursuant method of the above described reaction with the compound represented by formula (II) and the compound represented by formula (IV).
[C] Among the compounds represented by formula (I), the compound, wherein Y is -N(E¹)-C(=X)-NH-, that is, the compound represented by formula (I-C) 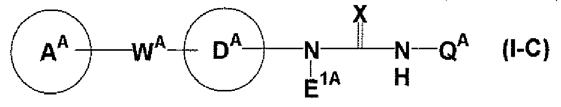 (wherein all symbols have the same meanings as described above.) can be prepared by the following method.

The compound represented by formula (I-C) can be prepared by reacting the compound represented by formula (II) with the compound represented by formula (X)

**X=C=N―Q**^{**A**} **(X)**

(wherein all symbols have the same meanings as described above.), if necessary, followed by subjecting to a deprotection reaction of protection group.

The reaction with the compound represented by formula (II) and the compound represented by formula (X) is carried out, for example, in an organic solvent (e.g. toluene, benzene, xylene, tetrahydrofuran, dichloromethane, diethylether, 1,2-dichloroethane, dimethylformamide *etc*.) at a temperature from 0°C to reflux temperature.

This reaction is preferably carried out under the anhydrous condition in the presence of inert gases.

The deprotection reaction of the protective group can be carried out by above described method.
[D] Among the compounds represented by formula (I), the compound, wherein Y is -N(E¹)-CH₂-, that is, the compound represented by formula (I-D) 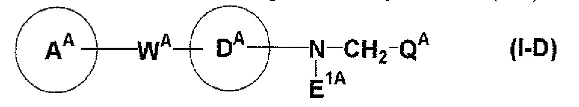 (wherein all symbols have the same meanings as described above.) can be prepared by the following method.

The compound represented by formula (I-D) can be prepared by reacting the compound represented by formula (II) with the compound represented by formula (XI)

**L**^{**2**}**―CH**_{**2**}**-Q**^{**A**} **(XI)**

(wherein L² is leaving group such as halogen atom, mesyloxy (OMs), tosyloxy (OTs), trifluoromethanesulfonyloxy (OTf), alkylthio, alkylsulfinyl, alkylsulfonyl, hydroxysulfonyl *etc.* The other symbols have the same meanings as described above.), if necessary, followed by subjecting to a deprotection reaction of protection group.

The reaction with the compound represented by formula (II) and the compound represented by formula (XI) is carried out, for example, in an organic solvent (*e*.*g*. tetrahydrofuran, dichloromethane, chloroform, benzene, toluene, xylene, hexane, heptane, cyclohexane, diethylether, dioxane, acetone, ethylmethylketone, acetonirile, dimethylsulfoxide, dimethylformamide, dimethylacetamide, ethyl acetate *etc*.), under the presence of a base (*e*.*g*. potassium carbonate, sodium carbonate, cesium carbonate, sodium halide *etc.)* under the presence or the absence of a catalyst (*e*.*g*. potassium iodide, sodium iodide, tetra-n-butylammonium iodide *etc*.) at a temperature from 0°C to reflux temperature.

The deprotection reaction of the protective group can be carried out by above described method.
[E] Among the compounds represented by formula (I), the compound, wherein Y is -C(=X)-N(E¹)-, that is, the compound represented by formula (I-E) 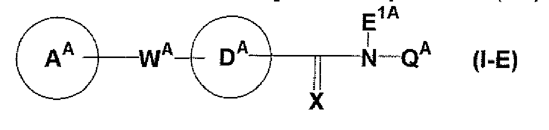 (wherein all symbols have the same meanings as described above.) can be prepared by the following [1] or [2].

[1] The compound represented by formula (I-E) can be prepared by reacting the compound represented by formula (XII) 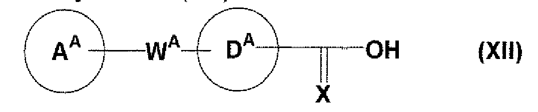 (wherein all symbols have the same meanings as described above.) with the compound represented by formula (XIII) 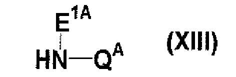 (wherein all symbols have the same meanings as described above.), if necessary, followed by subjecting to a deprotection reaction of protection group.

The reaction with the compound represented by formula (XII) and the compound represented by formula (XIII) can be carried out by pursuant method of the above described reaction with the compound represented by formula (II) and the compound represented by formula (III).

The deprotection reaction of the protective group can be carried out by above described method.
[2] The compound represented by formula (I-E) can be prepared by reacting the compound represented by formula (XIV) 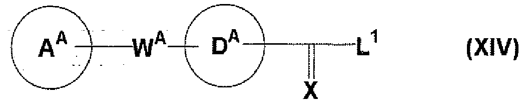 (wherein all symbols have the same meanings as described above.) with the compound represented by formula (XIII), if necessary, followed by subjecting to a deprotection reaction of protection group.

The reaction with the compound represented by formula (XIV) and the compound represented by formula (XIII) can be carried out by pursuant method of the above described reaction with the compound represented by formula (II) and the compound represented by formula (IV).

The deprotection reaction of the protective group can be carried out by above described method.
[F] Among the compounds represented by formula (I), the compound, wherein Y is -SO₂N(E¹)-, that is, the compound represented by formula (I-F) 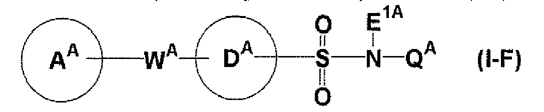 (wherein all symbols have the same meanings as described above.) can be prepared by the following [1] or [2].

[1] The compound represented by formula (I-F) can be prepared by reacting the compound represented by formula (XV) 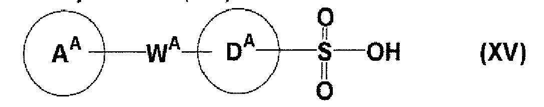 (wherein all symbols have the same meanings as described above.) with the compound represented by formula (XIII), if necessary, followed by subjecting to a deprotection reaction of protection group.

The reaction with the compound represented by formula (XV) and the compound represented by formula (XIII) can be carried out by pursuant method of the above described reaction with the compound represented by formula (II) and the compound represented by formula (VII).

The deprotection reaction of the protective group can be carried out by above described method.
[2] The compound represented by formula (I-F) can be prepared by reacting the compound represented by formula (XVI) 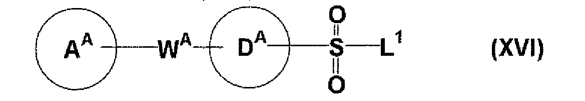 (wherein all symbols have the same meanings as described above.) with the compound represented by formula (XIII), if necessary, followed by subjecting to a deprotection reaction of protection group.

The reaction with the compound represented by formula (XVI) and the compound represented by formula (XIII) can be carried out by pursuant method of the above described reaction with the compound represented by formula (II) and the compound represented by formula (VIII).

The deprotection reaction of the protective group can be carried out by above described method.
[G] Among the compounds represented by formula (I), the compound, wherein W is a single bond, that is, the compound represented by formula (I-G) 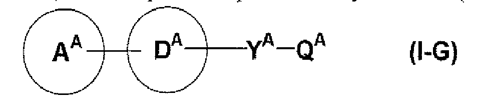 (wherein Y^{A} has the same meanings as above described Y. But carboxyl, hydroxyl, amino or mercapto included the group represented by Y^{A} are, if necessary, protected. The other symbols have the same meanings as described above.) can be prepared by the following [1] or [2].

[1] The compound represented by formula (I-G) can be prepared by reacting the compound represented by formula (XVII) 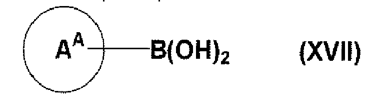 (wherein all symbols have the same meanings as described above.) with the compound represented by formula (XVIII) 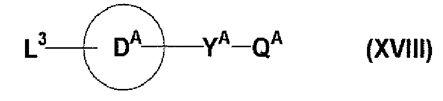 (wherein L³ is halogen atom, the other symbols have the same meanings as described above.), if necessary, followed by subjecting to a deprotection reaction of protection group.

The reaction with the compound represented by formula (XVII) and the compound represented by formula (XVIII) is known, and it is carried out, for example, by reacting in an organic solvent (*e*.*g*. benzene, toluene, N,N-dimethylformamide, 1,4-dioxane, teterahydrofuran, methanol, acetonitrile, dimethoxyethane, acetone *etc*.), under the presence of a base (*e*.*g*. sodium ethylate, sodium hydroxide, potassium hydroxide, triethylamine, sodium carbonate, sodium hydrogen carbonate, potassium carbonate, cesium carbonate, thallium carbonate, tripotassium carbonate, cesium fluoride, barium hydroxide, tetrabutylammonium fluoride *etc.)* or aqueous solution thereof, or mixture thereof and a catalyst (*e*.*g*. tetrakis(triphenylphosphine) palladium (Pd(PPh₃)₄), bis(triphenylphosphine) palladium dichloride (PdCl₂(PPh₃)₂), palladium acetate (Pd(OAc)₂), palladium black, 1,1'-bis(diphenylphosphinoferrocene)dichloropalladium (PdCl₂(dppf)₂), diallylpalladium dichloride (PdCl₂(allyl)₂), phenylbis(triphenylphosphine) palladium iodide (PhPdI(PPh)₃)₂) *etc*.) at a temperature from 0°C to 120°C.

The deprotection reaction of the protective group can be carried out by above described method.
[2] The compound represented by formula (I-G) can be prepared by reacting the compound represented by formula (XIX) 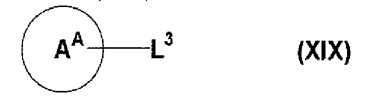 (wherein all symbols have the same meanings as described above.) with the compound represented by formula (XX) 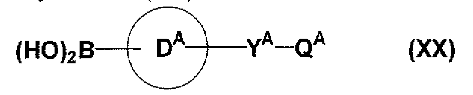 (wherein all symbols have the same meanings as described above.), if necessary, followed by subjecting to a deprotection reaction of protection group.

The reaction with the compound represented by formula (XIX) and the compound represented by formula (XX) can be carried out by pursuant method of the above described reaction with the compound represented by formula (XVII) and the compound represented by formula (XVIII).

The deprotection reaction of the protective group can be carried out by above described method.

The compounds represented by formula (II) to (XX) used as starting materials or reagents are well known per se or can be easily prepared by the known processes, for example, processes described in *"Comprehensive Organic Transformations:*", *"Chemishe Berichte,* 96, 1505 (1963)", *"Chemishe Berichte,* 98, 1081, (1965)", "*The Journal of Organic Chemistry,* 53, 4137 (1988)", "*Bioorganic and Medicinal Chemistry Letters,* 9, 2569-2572 (1999)", *"Synlett,* 6, 829-831 (2000)", *"Synthesis,* 55-62 (2001)", "Journal of the *Chemical Society Perkin Transactions,* 1, 1847 (2002)" and so on.

The compounds represented by formula (V) used as starting materials or reagents can be easily prepared by the process shown in the reaction process 1 or the pursuant process thereof

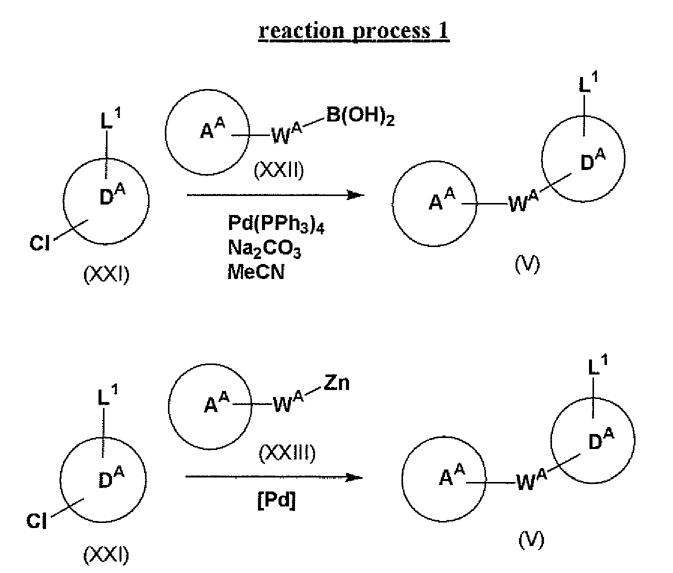

In addition, among the compound represented by formula (V) used as starting materials or reagents, the compound, wherein ringA^{A} is benzene, W^{A} is a single bond, D^{A} is pyrimidine, L¹ is a chlorine atom, that is, the compound represented by formula (Va) can be easily prepared by the process shown in the reaction process 2 or the pursuant process thereof.

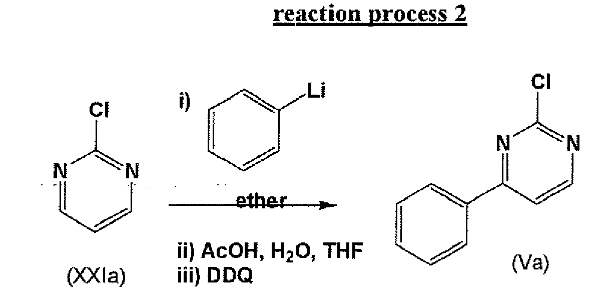

In the reaction process 1 and 2, the compound represented by formula (XXI) or (XXIa) as starting materials or reagents is well known per se or can be easily prepared by known process.

In each reaction processes, Ph is phenyl, Me is methyl, Ac is acetyl, THF is tetrahydrofuran, DDQ is 2,3-dichloro-5,6-dicyano-1,4-benzoquinone, the other symbols have the same meanings as described above.

In each reaction in the present specification, solid-phase supported reagent accordingly supported to macromolecule polymer (e.g. polystyrene, polyacrylamide, polypropylene, polyethyleneglycol *etc*.) may be used.

In each reaction in the present specification, reaction products may be purified in an ordinary manner, for example, through normal-pressure or reduced-pressure distillation, or through high-performance liquid chromatography with silica gel or magnesium silicate, thin-layer chromatography, or column chromatography, ionexchange resin, scavenger resin or through washing or recrystallizaion and so on. The purification may be effected in each reaction or after some reactions.

### [Toxicity]

Toxicity of the compound represented by formula (I) is very low, and it is safe enough to use as a pharmaceutical agent.

### [Effect of the Invention]

Since the compounds of the present invention represented by formula (I), a salt thereof, an N-oxide thereof or a solvate thereof, or a prodrug thereof have the affinity to MBR, they are useful for the prevention and/or treatment for disease induced or exacerbated and/or reignited by stressor or useful for the prevention and/or treatment for disease caused by stress.

The disease induced or exacerbated and/or reignited by stressor or the disease caused by stress include, for example, central nervous system diseases caused by stress (*e*.*g*. anxiety related disease (neurosis, psychosomatic disorder, generalized anxiety disorder (GAD), social-anxiety disorder (SAD), panic disorder, hyperactivity disorder, attention-deficit, personality disorder, bipolar disorder, autism *etc*.), sleep disorder, depression, reactive depression, epilepsy, Parkinson's disease, Perkinsonian syndrome, schizophrenia, autonomic dystonia, Huntington's disease, Alzheimer's disease, affective disorder, cognitive disorder, migraine, tension headache, cluster headache, posttraumatic stress disorder, dissociative disorder, insomnia, nervous vomiting, nervous cough, psychogenic convulsive seizure, psychogenic syncopal attack, maladjustment to job, burn-out syndrome, chronic fatigue syndrome, writer's cramp, spastic torticollis, *etc*.), respiratory system diseases caused by stress (*e*.*g*. asthma, bronchial asthma, hyperventilation syndrome, laryngeal spasm, chronic obstructive pulmonary diseases, *etc*.), digestive system diseases caused by stress (*e.g.* irritable bowel syndrome, peptic ulcer, functional dyspepsia, gastric ulcer, duodenal ulcer, ulcerative colitis, biliary tract dyskinesia, esophageal spasm, gastric atony, aerophagy, chronic hepatitis, chronic panceatitis, *etc.*), cardiovascular system diseases caused by stress (*e*.*g*. essential hypertension, arrhythmia, (neurological) angina pectoris, essential hypotension, orthostatic dysregulation, myocardial infarction, arteriosclerosis, vertigo *etc.*), uropathy·reproductive system diseases caused by stress (*e*.*g*. dysuria, nervous pollakisuria (hyperreflexic bladder), nocturia, enuresis, psychogenic ischuria, impotentia, prostatism, urethral syndrome *etc.*), gynecologic disorder caused by stress (*e*.*g*. menopausal disorder, menstrual pain, premenstrual syndrome, infertility, frigidity, serious vomiting of pregnancy, abortion, immature birth, *etc*.), endocrine and metabolic disease caused by stress (*e.g.* anorexia nervosa, eating disorder, anorexia, hyperphagia, Bartter's syndrome, hyperthyroidism, diabetes, psychogenic polydipsia, adiposity, reflex hypoglycemia *etc.*), ophthalmologic diseases caused by stress (*e.g.* asthenopia, central retinitis, floaters, blepharospasm, primary glaucoma, vertigo *etc*.), otolaryngological diseases caused by stress (*e*.*g*. tinnitus, vertigo, psychogenic deafness, chronic sinusitis, allergic rhinitis, smell disorder, stuttering, aphonia, *etc*.), dental surgery and dentistry caused by stress (*e*.*g*. temporomandibular arthrosis, glossopharyngeal neuralgia, sudden glossodynia, stomatitis, toothache, ozostomia, abnormal salivation, bruxism *etc*.), surgical and orthopedic diseases caused by stress (*e.g.* postoperative abdominal neurosis, dumping syndrome, polysurgery, plastic postoperative neurosis, rheumatoid arthritis, low back pain, cervico-omo-brachial syndrome, stiff neck, fibrositis, polyarthralgia, systemic myalgia, gout, *etc.),* skin diseases caused by stress (*e.g.* chronic urticaria, atopic dermatitis, hyperhidrosis, eczema, skin pruritus, alopecia areata, *etc*.) and other diseases caused by stress *(e.g.* cancer, systemic lupus erythematosus *etc*.).

The compounds in the present invention may be administered in combination with other pharmaceutical preparations for the purpose of 1) complement and/or enhancement of preventing and/or treating effect of the compounds in the present invention, 2) improvement of dynamics/absorption and lowering of dose of the compounds in the present invention and/or 3) alleviation of side effect of the compounds in the present invention.

The compounds in the present invention and other pharmaceutical preparations may be administered in the form of formulation having these components incorporated in one preparation or may be administered in separate preparations. In the case where these pharmaceutical preparations are administered in separate preparations, they may be administered simultaneously or at different times. In the latter case, the compounds in the present invention may be administered before the other pharmaceutical preparations. Alternatively, the other pharmaceutical preparations may be administered before the compounds in the present invention. The method for the administration of these pharmaceutical preparations may be same or different.

The other pharmaceutical preparations may be low-molecular compounds. In addition, they may be macromolecular protein, polypeptide, polynucleotide (DNA, RNA, and gene), antisense, decoy, antibody or vaccine and so on. The dose of the other pharmaceutical preparations can be accordingly selected as a standard of clinical dose. Additionally, the compounding ratio of the compounds in the present invention and the other pharmaceutical preparations can be accordingly selected by the age and body weight of administering object, the administration method, the administration time, the object disease, the symptom, the combination *etc.* For example, the other pharmaceutical preparations may be used from 0.01 to 100 parts by weight relative to 1 part by weight of the compounds in the present invention. The other pharmaceutical preparations may be administered at appropriate ratio combining one or more arbitrarily selected from the homogeneous groups or heterogeneous groups as follows. The other pharmaceutical preparations do not only include ones which have ever been found but ones which will be found from now based on the above-mentioned mechanism.

The other pharmaceutical preparations which may combine the compounds in the present invention include, for example, antianxiety drugs (*e.g.* benzodiazepine anxiolytics, thienodiazepine anxiolytics, non-benzodiazepine anxiolytics, serotonergic drugs, CRF antagonists, tachykinin NK₁ antagonists *etc.*), antidepressants (*e*.*g*. tricyclic antidepressants, tetracyclic antidepressants, monoamine release drugs, monoamine oxidase inhibitors, monoamine reuptake inhibitors (SSRI, SNRI), CRF inhibitors, tachykinin NK₁ inhibitors, neurotensin antagonists *etc*.), antiparkinson drugs *(e.g.* anticholinergic drugs, dopamine agonists, monoamine oxidase inhibitors, *etc*.), schizophrenia drugs (*e*.*g*. dopamine antagonists, *etc*.), antiepileptic drugs (*e*.*g*. barbituric acid series, hydantoin series *etc*.), anti vertigo drugs, asthmatic drugs *(e.g.* bronchodilators, α receptor agonists, β₂ receptor agonists, xanthine series, inhaled steroids, anticholinergic drugs, 5-lipoxygenase inhibitors *etc*.), peptic ulcer drugs (*e*.*g*. offensive factor inhibitors, antipeptic drugs, antacids, histamine-H₂ receptor antagonists, anti gastrin drugs, proton pump inhibitors, muscarine receptor inhibitors, anticholinergic drugs, defensive factor enhancers, prostaglandin derivatives, *etc.*), gastrointestinal tract function regulators · gastrointestinal tract prokinetic drugs (*e*.*g*. intestinal remedies, CCK-A antagonists, neurotensin antagonists, opioid agonists, muscarine receptor inhibitors, 5-HT₄ agonists, 5-HT₃ antagonists *etc.*), antidiarrheals (*e.g.* antidiarrheal drugs, opioid µ receptor stimulators, *etc*.), evacuants (*e.g.* bulk laxatives, saline laxatives, stimulant laxatives, affinity polyacrylic resin *etc*.), antihypertensive drugs (*e*.*g*. calcium antagonists, β receptor blockers, α₁ receptor blockers, angiotensin converting enzyme inhibitors, angiotensin II receptor blockers, *etc*.), antiarrhythmic drugs (*e*.*g*. sodium inhibitors, β receptor blockers, potassium antagonists, calcium antagonists, *etc*.), cardiac stimulants (*e.g.* phosphodiesterase inhibitors, cardiac glycosides, β receptor agonists *etc*.), dysuria remedies (*e.g.* frequent urination remedies, anticholinergic drugs, muscarine agonists (antagonists), tachykinin NK₁ antagonists, NK₂ antagonists, *etc*.) and so on.

The diseases on which the preventive and/or therapeutic effect works with the above described combination drugs are not especially limited. The diseases may be those which compensate for and/or enhance the preventive and/or therapeutic effect of the compounds in the present invention.

The other pharmaceutical preparations to compensate and/or enhance for preventive and/or therapeutic effect on irritable bowel syndrome of the compounds in the present invention include, for example, antianxiety drugs (*e*.*g*. benzodiazepine anxiolytics, thienodiazepine anxiolytics, non-benzodiazepine anxiolytics, serotonergic drugs, CRF antagonists *etc.*), antidepressants (*e.g.* monoamine release drugs, monoamine oxidase inhibitors, monoamine reuptake inhibitors (SSRI, SNRI), CRF inhibitors, neurotensin antagonists, tricyclic antidepressants, tetracyclic antidepressants, *etc*.), anticholinergic drugs, gastrointestinal tract function regulators·gastrointestinal tract prokinetic drugs (*e.g.* intestinal remedies, CCK-A antagonists, neurotensin antagonists, opioid agonists, muscarine receptor agonists, 5-HT₄ agonists, *etc*.), antidiarrheals (*e.g.* antidiarrheal drugs, opioid µ receptor stimulators, *etc*.), evacuants *(e.g.* bulk laxatives, saline laxatives, stimulant laxatives, affinity polyacrylic resin *etc.),* mucosal paralytic drugs, autonomic nerve modulators, calcium antagonists, phosphodiesterase inhibitors, serotonin antagonists (*e*.*g*. 5-HT₃ antagonists, 5-HT₄ antagonists *etc*.), darifenacyn, polycarbophil calcium and so on.

The other pharmaceutical preparations to compensate and/or enhance for preventive and/or therapeutic effect on gastric ulcer and duodenal ulcer of the compounds in the present invention include, for example, peptic ulcer drugs (*e.g.* offensive factor inhibitors, antipeptic drugs, antacids, histamine-H₂ receptor antagonists, anti gastrin drugs, proton pump inhibitors, muscarine receptor inhibitors, anticholinergic drugs, defensive factor enhancers, prostaglandin derivatives, mesalazine, salazosulfapyridine, *etc*.), anticholinergic drugs, gastric mucosal paralytic drugs, antianxiety drugs (*e*.*g.* benzodiazepine anxiolytics, thienodiazepine anxiolytics, non-benzodiazepine anxiolytics, serotonergic drugs, CRF antagonists, *etc*.), dopamine antagonists and so on.

The other pharmaceutical preparations to compensate and/or enhance for preventive and/or therapeutic effect on ulcerative colitis of the compounds in the present invention include, for example, mesalazine, salazosulfapyridine, peptic ulcer drugs (*e*.*g*. offensive factor inhibitors, antipeptic drugs, antacids, histamine-H₂ receptor antagonists, anti gastrin drugs, proton pump inhibitors, muscarine receptor inhibitors, anticholinergic drugs, defensive factor enhancers, prostaglandin derivatives, *etc*.), anticholinergic drugs, steroids, 5-lipoxygenase inhibitors, antioxidant drugs, LTB₄ antagonists, local anesthetics, immunosuppressive drugs, defensive factor enhancers, metalloprotease inhibitors and so on.

The other pharmaceutical preparations to compensate and/or enhance for preventive and/or therapeutic effect on biliary tract dyskinesia of the compounds in the present invention include, for example, ceruleins, antispasmodic drugs, COMT (catechol-O-methyltransferase) inhibitors, cholinergic agonists, anticholinergic drugs, antianxiety drugs, cholagogues, antidepressants, CCK-A antagonists and so on.

The other pharmaceutical preparations to compensate and/or enhance for preventive and/or therapeutic effect on aerophagy of the compounds in the present invention include, for example, intestinal remedies, antianxiety drugs, autonomic nerve modulators, fiber formulations, digestive enzymes, gas absorbent drugs, intestinal tract prokinetic drugs and so on.

The other pharmaceutical preparations to compensate and/or enhance for preventive and/or therapeutic effect on chronic hepatitis of the compounds in the present invention include, for example, liver hydrolysate formulations, polyenephosphatidylcholine, glycyrrhizin formulations, protoporphyrin sodium, ursodeoxycholic acid, steroids, anticholinergic drugs, antacids, propagermanium, lipid peroxidase inhibitors and so on.

The other pharmaceutical preparations to compensate and/or enhance for preventive and/or therapeutic effect on chronic pancreatitis of the compounds in the present invention include, for example, protease inhibitors, gastric acid inhibitors, antispasmodic drugs (*e*.*g*. COMT inhibitors, anti serotonin drugs *etc*.), nonsteroidal antiinflammatory drugs, central analgesics, sedatives, digestive enzymes, antacids, histamine H₂ receptor inhibitors, antidepressants, gastric mucosa local anesthetics, gastrointestinal tract function regulators (CCK-A antagonists) and so on.

The other pharmaceutical preparations to compensate and/or enhance for preventive and/or therapeutic effect on esophageal spasm of the compounds in the present invention include, for example, esophageal prokinetic drugs, antianixiety drugs, autonomic nerve modulators and so on.

The other pharmaceutical preparations to compensate and/or enhance for preventive and/or therapeutic effect on gastric atony of the compounds in the present invention include, for example, gastrointestinal tract prokinetic drugs, digestive enzymes, tranquilizers and so on.

The other pharmaceutical preparations to compensate and/or enhance for preventive and/or therapeutic effect on functional dyspepsia of the compounds in the present invention include, for example, antacids, histamine H₂ receptor inhibitors, gastrointestinal tract function regulators, gastrointestinal tract prokinetic drugs, antianxiety drugs, tranquilizers, digestive enzymes, proton pump inhibitors, muscarine receptor inhibitors, anticholinergic drugs, defensive factor enhancers, dopamine antagonists and so on.

Antianxiety drugs include, for example, diazepam, oxazolam, flunitrazepam, alprazolam, etizolam, flutazolam, lorazepam, ethyl loflazepate, tofisopam, clotiazepam, γ oryzanol and so on.

Tricyclic antidepressants include, for example, amitriptyline, imipramine, clomipramine, nortriptyline, desipramine, amoxapine and so on.

Tetracyclic antidepressants include, for example, mianserin, maprotiline and so on.

Monoamine oxidase inhibitors include, for example, trazodone, fluvoxamine and so on.

Antiparkinson drugs include, for example, levodopa, amantadine, selegiline, bromocriptine, pramipexole, anticholinergic drug and so on.

Anticholinergic drugs include, for example, trihexyphenidyl, biperiden, ipratropium bromide, mepenzolate bromide and so on.

Antiepileptic drugs include, for example, phenobarbital, phenytoin, carbamazepine, valproic acid, clonazepam and so on.

Anti vertigo drugs include, for example, difenidol, betahistine and so on.

Asthmatic drugs include, for example, ephedrine, orciprenaline, salbutamol, procaterol, theophylline, aminophylline, disodium cromoglycate, anticholinergic drug, inhaled steroid and so on.

Inhaled steroids include, for example, beclomethasone, prednisolone and so on.

Antipeptic drugs include, for example, sucralfate and so on.

Antacids include, for example, sodium bicarbonate, magnesium oxide, dry aluminum hydroxide gel, aluminum silicate and so on.

Histamine H₂ receptor inhibitors include, for example, famotidine, ranitidine, cimetidine, roxatidine and so on.

Anti gastrin drugs include, for example, proglumide and so on.

Proton pump inhibitors include, for example, omeprazole, lansoprazole and so on.

Muscarine receptor inhibitors include, for example, pirenzepine and so on.

Defensive factor enhancers include, for example, gefarnate, teprenone, sucralfate, aldioxa, cetraxate hydrochloride, ornoprostil and so on.

Prostaglandin derivatives include, for example, ornaprostil, misoprostol and so on.

Gastrointestinal tract function regulators include, for example, cisapride, domperidone, sulpiride, metoclopramide, alosetron, trimebutine maleate and so on.

Gastrointestinal tract prokinetic drugs include, for example, cisapride, tegaserod, bethanechol hydrochloride and so on.

Antidiarrheals include, for example, loperamide and so on.

Bulk laxatives include, for example, methylcellulose, carmellose, lactulose and so on.

Saline laxatives include, for example, magnesium sulfate, magnesium oxide and so on.

Stimulant laxatives include, for example, picosulfate, lactulose, castor oil, senna, rhubarb and so on.

Antihypertensive drugs include, for example, nicardipine, nifedipine, nilvadipine, atenolol, allotynol, carteolol, propranolol, metoprolol, prazosin, captopril, enalapril, candesartan cilexetil, losartan potassium and so on.

Antiarrhythmic drugs include, for example, quinidine, procainamide, disopyramide, lidocaine, mexiletine, propranolol, amiodarone, verapamil and so on.

Cardiac stimulants include, for example, digitoxin, digoxin, dopamine, dobutamine, aminophylline, mimoline and so on.

Dysuria remedies include, for example, oxybutynin, tamsulosin, propiverine and so on.

Local anesthetics include, for example, lidocaine, oxethazaine, procaine hydrochloride, dibucaine hydrochloride, cocaine hydrochloride, tetracaine hydrochloride and so on.

Immunosuppressive drugs include, for example, cyclosporine, tacrolimus, azathiopurine and so on.

Autonomice nerve modulators include, for example, γ orizanol and so on.

Cholagogues include, for example, ursodeoxycholic acid and so on.

In order to use the compounds in the present invention, or the compounds in the present invention in combination with the other pharmaceutical preparations by the above-mentioned purpose, these compounds are normally administered to the entire of human body or topically, and orally or parenterally.

The dose of the compounds in the present invention depends on age, body weight, symptom, therapeutic effect, the administration method, the treatment time and so on. In practice, however, these compounds are administered orally once or several times per day each in an amount of from 100 µg to 1000 mg per adult, parentally once or several times per day each in an amount of from 50 µg to 500 mg per adult or continuously administered into vein for 1 hour to 24 hours per day.

It goes without saying that the dose of these compounds may be less than the above-mentioned dose or may need to exceed the above-mentioned range because the dose varies under various conditions as mentioned above.

When the compounds in the present invention, or the compounds in the present invention are administered in combination with the other pharmaceutical preparations, they are used in the form of solid or liquid agent for oral administration, injection, agent for external application, suppository, eye drops or inhalant for parenteral administration or the like.

Examples of the solid agent for oral administration include tablet, pill, capsule, powder, and pellet. Examples of the capsule include hard capsule, and soft capsule.

In such a solid agent for internal application, one or more active materials are used in the form of preparation produced by an ordinary method singly or in admixture with a vehicle (*e*.*g*., lactose, mannitol, glucose, microcrystalline cellulose, starch *etc*.), binder (*e*.*g*., hydroxypropyl cellulose, polyvinyl pyrrolidone, magnesium metasilicoalutninate *etc.*), disintegrant (*e*.*g*., calcium fbrinoglycolate *etc*.), glidant *(e.g.,* magnesium stearate *etc*.), stabilizer, dissolution aid (*e*.*g*., glutamic acid, aspartic acid *etc*.) or the like. The solid agent may be coated with a coating agent (*e.g.*, white sugar, gelatin, hydroxypropyl cellulose, hydroxypropyl methyl cellulose phthalate *etc*.) or two or more layers. Alternatively, the solid agent may be capsulated by an absorbable material such as gelatin.

Examples of the liquid agent for oral administration include pharmaceutically acceptable aqueous solution, suspension, emulsion, syrup, and elixir. In such a liquid agent, one or more active agents are dissolved, suspended or emulsified in a commonly used diluent (*e.g*., purified water, ethanol, mixture thereof *etc*.). Furthermore, such a liquid agent may comprise a wetting agent, a suspending agent, an emulsifier, a sweetening agent, a flavor, a fragrance, a preservative, a buffer, *etc.*

The agent for parenteral administration may be in the form of, *e*.*g*., ointment, gel, cream, wet compress, paste, liniment, nebula, inhalant, spray, aerosol, eye drops, collunarium or the like. These agents each contain one or more active materials and are prepared by any known method or commonly used formulation.

The ointment is prepared by any known or commonly used formulation. For example, one or more active materials are triturated or dissolved in a base to prepare such an ointment. The ointment base is selected from known or commonly used materials. In some detail, higher aliphatic acid or higher aliphatic acid ester (*e.g.*, adipic acid, myristic acid, palmitic acid, stearic acid, oleic acid, adipic acid ester, myristic acid ester, palmitic acid ester, stearic acid ester, oleic acid ester *etc.*), wax (*e*.*g*., beeswax, whale wax, ceresin *etc*.), surface active agent (*e*.*g*., polyoxyethylenealkylether phosphoric acid ester *etc*.), higher alcohol (*e*.*g*., cetanol, stearyl alcohol, setostearyl alcohol *etc.),* silicon oil (*e*.*g*., dimethyl polysiloxane *etc*.), hydrocarbon (*e*.*g*., hydrophilic petrolatum, white petrolatum, purified lanolin, liquid paraffin *etc.*), glycol (*e.g*., ethylene glycol, diethylene glycol, propylene glycol, polyethylene glycol, macrogol *etc*.), vegetable oil (*e*.*g*., castor oil, olive oil, sesame oil, turpentine oil), animal oil (mink oil, vitelline oil, squalane, squalene), water, absorption accelerator and rash preventive may be used singly or in admixture of two or more thereof. The base may further comprise a humectant, a preservative, a stabilizer, an antioxidant, a perfume, *etc*.

The gel is prepared by any known or commonly used formulation. For example, one or more active materials are dissolved in a base to prepare such a gel. The gel base is selected from known or commonly used materials. For example, lower alcohol (*e.g.*, ethanol, isopropyl alcohol *etc.),* gelling agent (*e*.*g*., carboxymethyl cellulose, hydroxyethyl cellulose, hydroxypropyl cellulose, ethyl cellulose *etc*.), neutralizing agent (*e.g.*, triethanolamine, diisopropanolamine *etc.*), surface active agent (*e.g.*, polyethylene glycol monostearate *etc.*), gums, water, absorption accelerator, and rash preventive are used singly or in admixture of two or more thereof The gel base may further comprise a preservative, an antioxidant, a perfume, *etc.*

The cream is prepared by any known or commonly used formulation. For example, one or more active materials are dissolved in a base to prepare such a cream. The cream base is selected from known or commonly used materials. For example, higher aliphatic acid ester, lower alcohol, hydrocarbon, polyvalent alcohol (*e*.*g*., propylene glycol, 1,3-butylene glycol *etc*.), higher alcohol (*e.g.*, 2-hexyl decanol, cetanol *etc*.), emulsifier (*e.g.*, polyoxyethylene alkyl ethers, aliphatic acid esters *etc*.), water, absorption accelerator, and rash preventive are used singly or in admixture of two or more thereof The cream base may further comprise a preservative, an antioxidant, a perfume, *etc*.

The wet compress is prepared by any known or commonly used formulation. For example, one or more active materials are dissolved in a base to prepare a kneaded mixture which is then spread over a support to prepare such a wet compress. The wet compress base is selected from known or commonly used materials. For example, thickening agent (*e.g.*, polyacrylic acid, polyvinyl pyrrolidone, gum arabic, starch, gelatin, methyl cellulose *etc*.), wetting agent *(e.g.,* urea, glycerin, propylene glycol *etc*.), filler (*e*.*g*., kaolin, zinc oxide, talc, calcium, magnesium *etc*.), water, dissolution aid, tackifier, and rash preventive may be used singly or in admixture of two or more thereof. The wet compress base may further comprise a preservative, an antioxidant, a perfume, *etc*.

The pasting agent is prepared by any known or commonly used formulation. For example, one or more active materials are dissolved in a base to prepare a kneaded mixture which is then spread over a support to prepare such a pasting agent. The pasting agent base is selected from known or commonly used materials. For example, polymer base, fat and oil, higher aliphatic acid, tackifier and rash preventive may be used singly or in admixture of two or more thereof. The pasting agent base may further comprise a preservative, an antioxidant, a perfume, *etc.*

The liniment is prepared by any known or commonly used formulation. For example, one or more active materials are dissolved, suspended or emulsified in water, alcohol (*e*.*g*., ethanol, polyethylene glycol *etc.),* higher aliphatic acid, glycerin, soap, emulsifier, suspending agent, *etc*., singly or in combination of two or more thereof, to prepare such a liniment. The liniment may further comprise a preservative, an antioxidant, a perfume, *etc*.

The nebula, inhalant, spray and aerozol each may comprise a commonly used diluent, additionally, a stabilizer such as sodium hydrogen sulfite and a buffer capable of providing isotonicity such as isotonic agent (*e.g.*, sodium chloride, sodium citrate, or citric acid *etc*.). For the process for the preparation of spray, reference can be made to US Patents 2,868,691 and 3,095,355.

The injection for parenteral administration consists of solid injection used to be dissolved or suspended in the form of solution, suspension, emulsion and a solvent to be dissolved before use. The injection is prepared by dissolving, suspending or emulsifying one or more active materials in a solvent. As such a solvent there may be used distilled water for injection, physiological saline, vegetable oil, alcohol such as propylene glycol, polyethylene glycol and ethanol, *etc.*, singly or in combination thereof. The injection may further comprise a stabilizer, a dissolution aid (*e*.*g*., glutamic acid, aspartic acid, Polysolvate 80 (trade name) *etc*.), a suspending agent, an emulsifier, a soothing agent, a buffer, a preservative, *etc*. The injection is sterilized at the final step or prepared by an aseptic process. Alternatively, an aseptic solid agent such as freeze-dried product which has previously been prepared may be rendered aseptic or dissolved in aseptic distilled water for injection or other solvents before use.

The eye drops for parenteral administration consist of eye drop, suspension eye drop, emulsion eye drop, eye drop to be dissolved before use and ointment and so on.

These eye drops are prepared by a known method. For example, it is prepared by dissolving, suspending or emulsifying one or more active materials in a solvent. As such a solvent for eye drops there may be used distilled water, physiological saline, the other aqueous solvent or nonaqueous solvent for injection (*e*.*g*. vegetable oil *etc*.), *etc*., singly or in combination thereof. The eye drops may comprise, if necessary, of materials properly selected from tonisity agent (*e.g.* sodium chloride, concentrated glycerin *etc*.), buffer agents (*e.g.* sodium phosphate, sodium acetate *etc*.), surfactants (*e.g.* polysorbate 80 (trade name), polyoxyl 40 stearate, polyoxyethylene hydrogenated castor oil *etc*.), stabilizer (*e.g.* sodium citrate, sodium edentate *etc*.), antiseptic agent (*e.g.* benzalkonium chloride, paraben *etc*.) These are sterilized at the final step or prepared by an aseptic process. Alternatively, an aseptic solid agent such as freeze-dried product which has previously been prepared may be rendered aseptic or dissolved in aseptic distilled water for injection or other solvents before use.

The inhalant for parenteral administration may be in the form of aerosol, powder for inhalation or liquid for inhalation. The liquid for inhalation may be dissolved or suspended in water or other proper medium in use.

These inhalants are prepared by a known method.

For example, the liquid for inhalation is prepared from materials properly selected from preservatives (*e*.*g*., benzalconium chloride, Paraben *etc*.), colorants, buffering agents *(e.g.,* sodium phosphate, sodium acetate *etc*.), isotonic agents (*e*.*g*., sodium chloride, concentrated glycerin *etc*.), thickening agents (*e*.*g*., carboxyvinyl polymer *etc*.), absorption accelerators, *etc*. as necessary.

The powder for inhalation is prepared from materials properly selected from glidants (*e*.*g*., stearic acid and salt thereof *etc*.), binders (*e*.*g*., starch, dextrin *etc*.), vehicles (*e*.*g*., lactose, cellulose *etc*.), colorants, preservatives (*e*.*g*., benzalconium chloride, Paraben *etc*.), absorption accelerators, *etc*., if necessary.

In order to administer the liquid for inhalation, a sprayer (*e*.*g*., atomizer, nebulizer *etc.)* is normally used. In order to administer the powder for inhalation, a powder inhaler is normally used.

Other examples of the composition for parenteral administration include suppository for rectal administration and pessary for vaginal administration prepared by an ordinary formulation comprising one or more active materials.

### Best Mode for Carrying Out the Invention

The present invention is explained below in detail base on Examples, however, the present invention is not limited thereto. The solvents in parentheses at chromatographic separations section and TLC section show the developing or eluting solvents and the ratios of the solvents used are indicated by volume. NMR is the measurement of ¹H NMR. The solvents in parentheses indicated in NMR section show solvents used in determination, unless noting deuterated chloroform used as the solvent.

All compounds described in the specification are named by using of ACD/Name (Trade mark, Advanced Chemistry Development Inc.) or ACD/Name batch (Trade mark, Advanced Chemistry Development Inc.) which is the computer program to name according to IUPAC rule, or according to IUPAC organic chemistry nomenclature.

### Example 1

### (2E)-3-(dimethylamino)-1-phenyl-2-propen-1-one:

Acetophenone (1.2 g, 10.0 mmol) and N,N-dimethylformamide dimethylacetal (1.2 g, 10.0 mmol) were got into pressure-resistant tube and exposed by microwave (300 W, 150°C) for 6 minutes with sealing. After termination of the reaction, the reaction solution was concentrated and then recrystallized with ethyl acetate-hexane to give the title compound (1.25 g) having the following physical data.
TLC: Rf 0.22 (ethyl acetate : hexane = 2 : 1);
NMR: δ 7.93-7.86 (m, 2H), 7.81 (d, J = 12 Hz, 1H), 7.49-7.33 (m, 3H), 5.71 (d, J = 12 Hz, 1H), 3.3-2.7 (br, 6H).

### Example 2

### 4-phenyl-2-aminopyrimidine:

The compound prepared in Example 1 (1.15 g, 6.55 mmol), guanidine carbonate (885 mg, 4.90 mmol) and ethanol (6 mL) were got into pressure-resistant tube and exposed by microwave (300 W, 150°C, 6 minutes × 3 times) with sealing. After termination of the reaction, the reaction solution was added by water, precipitates were obtained with filtration and dried over to give the title compound (1.036 g) having the following physical data.
TLC: Rf 0.45 (ethyl acetate : hexane = 2 : 1);
NMR: δ 8.36 (d, J = 5 Hz, 1H), 8.03-7.96 (m, 2H), 7.52-7.42 (m, 3H), 7.05 (d, J = 5 Hz, 1H), 5.1 (brs, 2H).

### Example 3

### 2-(benzyloxy)-N-(4-phenylpyrimidin-2-yl)acetamide:

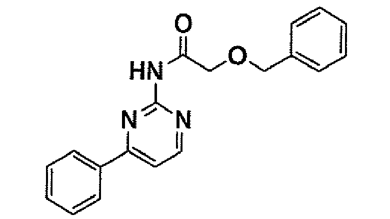

A solution of the compound (342 mg, 2.0 mmol) prepared in Example 2 in pyridine (5 mL) was added by benzyloxyacetylchloride (0.35 mL, 2.2 mmol) at room temperature and stirred for 2 hours. The reaction solution was added by water and saturated sodium hydrogen carbonate aqueous solution and extracted by ethyl acetate. The organic layer was washed with saturated brine, dried over anhydrous sodium sulfate and concentrated. The obtained residue was recrystallized with ethyl acetate-hexane to give the title compound (580 mg) having the following physical data.
TLC: Rf 0.41 (ethyl acetate : hexane = 2 : 1);
NMR: δ 4.23 (s, 2H), 4.71 (s, 2H), 7.44 (m, 9H), 8.09 (m, 2H), 8.71 (d, J=5.31 Hz, 1H), 9.09 (s, 1H).

### Example 3(1)-(68)

By the same procedure as described in Example 1, Example 2 and Example 3 using acetophenone or the corresponding ketone thereof and benzyloxyacetylchloride or the corresponding acid chloride thereof, the following compounds of the present invention were obtained.

### Example 3(1)

### 2-phenoxy-N-(4-phenylpyrimidin-2-yl)acetamide

TLC: Rf 0.42 (hexane : ethyl acetate = 3 : 1);
NMR: δ 4.81 (s, 2H), 7.06 (m, 3H), 7.36 (m, 2H), 7.49 (d, J=5.31 Hz, 1H), 7.53 (m, 3H), 8.10 (m, 2H), 8.73 (d, J=5.31 Hz, 1H), 9.03 (s, 1H).

### Example 3(2)

### N-[4-(4-methylphenyl)pyrimidin-2-yl]thiophene-2-carboxamide

TLC: Rf 0.28 (hexane : ethyl acetate = 1 : 1);
NMR: δ 2.43 (s, 3H), 7.16 (dd, J=4.94, 3.84 Hz, 1H), 7.32 (d, J=7.87 Hz, 2H), 7.44 (d, J=5.31 Hz, 1H), 7.61 (dd, J=5.03, 1.19 Hz, 1H), 7.74 (dd, J=3.84, 1.10 Hz, 1H), 8.03 (m, 2H), 8.56 (s, 1H), 8.68 (d, J=5.31 Hz, 1H).

### Example 3(3)

### N-[4-(4-methylphenyl)pyrimidin-2-yl]benzamide:

TLC: Rf0.28 (hexane : ethyl acetate = 1 : 1);
NMR: δ 2.41 (s, 3H), 7.29 (d, J=8.06 Hz, 2H), 7.41 (d, J=5.49 Hz, 1H), 7.52 (m, 3H), 7.96 (m, 4H), 8.64 (d, J=5.31 Hz, 1H), 8.89 (s, 1H).

### Example 3(4): N-[4-(4-methylphenyl)pyrimidin-2-yl]phenoxyacetamide:

TLC: Rf 0.53 (hexane : ethyl acetate = 1 : 1);
NMR: δ 2.44 (s, 3H), 4.81 (s, 2H), 7.06 (m, 3H), 7.35 (m, 4H), 7.45 (d, J=5.31 Hz, 1H), 8.00 (d, J=8.42 Hz, 2H), 8.69 (d, J=5.31 Hz, 1H), 9.01 (s, 1H).

### Example 3(5)

### 2-(benzyloxy)-N-[4-(4-methylphenyl)pyr1nlidin-2-yl]acetamide:

TLC: Rf 0.29 (hexane : ethyl acetate = 1 : 1);
NMR: δ 2.43 (s, 3H), 4.23 (s, 2H), 4.71 (s, 2H), 7.37 (m, 8H), 7.99 (d, J=8.24 Hz, 2H), 8.67 (d, J=5.31 Hz, 1H), 9.06 (s, 1H).

### Example 3(6)

### N-{4-[3-(trifluoromethyl)phenyl]pyrimidin-2-yl}thiophene-2-carboxamide:

TLC: Rf 0.49 (ethyl acetate : hexane = 2 : 1);
NMR: δ 7.18 (dd, J=5.03, 3.75 Hz, 1H), 7.51 (d, J=5.31 Hz, 1H), 7.65 (m, 2H), 7.78 (m, 2H), 8.35 (m, 2H), 8.57 (s, 1H), 8.78 (d, J=5.31 Hz, 1H).

### Example 3(7)

### N-{4-[3-(trifluoromethyl)phenyl]pyrimidin-2-yl}benzamide:

TLC: Rf 0.25 (hexane : ethyl acetate = 1 : 1);
NMR: δ 7.58 (m, 5H), 7.79 (m, J=7.87 Hz, 1H), 7.99 (m, 2H), 8.31 (m, J=8.24 Hz, 1H), 8.36 (m, 1H), 8.70 (m, 1H), 8.80 (d, J=5.13 Hz, 1H).

### Example 3(8)

### 2-phenoxy-N-{4-[3-(trifluoromethyl)phenyl]pyrimidin-2-yl}acetamide:

TLC: Rf 0.43 (hexane : ethyl acetate = 1 : 1);
NMR: δ 4.80 (s, 2H), 7.06 (m, 3H), 7.36 (m, 2H), 7.52 (d, J=5.31 Hz, 1H), 7.66 (t, J=7.78 Hz, 1H), 7.79 (d, J=7.87 Hz, 1H), 8.30 (d, J=7.87 Hz, 1H), 8.36 (s, 1H), 8.79 (d, J=5.31 Hz, 1H), 9.15(s, 1H).

### Example 3(9)

### N-[4-(4-cyanophenyl)pyrimidin-2-yl]thiophene-2-carboxamide:

TLC: Rf 0.15 (hexane : ethyl acetate = 1 : 1);
NMR: δ 7.18 (dd, J=4.94, 3.84 Hz, 1H), 7.50 (d, J=5.13 Hz, 1H), 7.64 (dd, J=5.03, 1.01 Hz, 1H), 7.75 (dd, J=3.84, 1.10 Hz, 1H), 7.81 (m, 2H), 8.27 (m, 2H), 8.55 (s, 1H), 8.78 (d, J=5.13 Hz, 1H).

### Example 3(10)

### N-[4-(4-cyanophenyl)pyrimidin-2-yl]benzamide:

TLC: Rf 0.26 (hexane : ethyl acetate = 1 : 2);
NMR: δ 7.57 (m, 4H), 7.79 (m, 2H), 7.97 (m, 2H), 8.22 (m, 2H), 8.76 (d, J=5.31 Hz, 1H), 8.88 (s, 1H).

### Example 3(11)

### N-[4-(2-fluorophenyl)pyrimidin-2-yl]thiophene-2-carboxamide:

TLC: Rf 0.66 (methylene chloride : methanol = 9 : 1);
NMR: δ 7.18 (m, 2H), 7.32 (m, 1H), 7.49 (m, 1H), 7.62 (m, J=5.13, 1.28 Hz, 2H), 7.74 (dd, J=3.75, 1.19 Hz, 1H), 8.27 (m, 1H), 8.56 (s, 1H), 8.72 (d, J=5.31 Hz, 1H).

### Example 3(12)

### N-[4-(2-fluorophenyl)pyrimidin-2-yl]benzamide:

TLC: Rf 0.64 (methylene chloride : methanol = 9 : 1);
NMR: δ 7.19 (ddd, J=11.76, 8.19, 0.92 Hz, 1H), 7.31 (m, 1H), 7.54 (m, 5H), 7.97 (m, 2H), 8.23 (m, 1H), 8.73 (m, J=5.31 Hz, 2H).

### Example 3(13)

### N-[4-(2-fluorophenyl)pyrimidin-2-yl]-2-phenoxyacetamide:

TLC: Rf 0.56 (methylene chloride : methanol = 9 : 1);
NMR: δ 4.80 (s, 2H), 7.04 (m, 3H), 7.20 (m, 1H), 7.34 (m, 3H), 7.50 (m, 1H), 7.63 (dd, J=5.31, 1.83 Hz, 1H), 8.22 (m, 1H), 8.74 (d, J=5.31 Hz, 1H), 9.05 (s, 1H).

### Example 3(14)

### 2-(benzyloxy)-N-[4-(2-fluorophenyl)pyrimidin-2-yl]acetamide:

TLC: Rf 0.73 (methylene chloride : methanol = 9 : 1);
NMR: δ 4.22 (s, 2H), 4.71 (s, 2H), 7.18 (m, 1H), 7.35 (m, 6H), 7.48 (m, 1H), 7.60 (dd, J=5.31, 1.83 Hz, 1H), 8.20 (m, 1H), 8.72 (d, J=5.31 Hz, 1H), 9.08 (s, 1H).

### Example 3(15)

### N-[4-(4-fluorophenyl)pyrimidin-2-yl]thiophene-2-carboxamide:

TLC: Rf 0.29 (hexane : ethyl acetate = 1 : 1);
NMR: δ 7.20 (m, 3H), 7.43 (d, J=5.31 Hz, 1H), 7.63 (dd, J=4.94, 1.10 Hz, 1H), 7.76 (dd, J=3.84, 1.10 Hz, 1H), 8.16 (m, 2H), 8.62 (br. s., 1H), 8.69 (d, J=5.31 Hz, 1H).

### Example 3(16)

### N-[4-(4-fluorophenyl)pyrimidin-2-yl]benzamide:

TLC: Rf 0.29 (hexane : ethyl acetate = 1 : 1);
NMR: δ 7.19 (m, 2H), 7.43 (d, J=5.31 Hz, 1H), 7.56 (m, 3H), 7.97 (m, 2H), 8.12 (m, 2H), 8.71 (m, J=5.13 Hz, 2H).

### Example 3(17)

### N-[4-(4-fluorophenyl)pyrimidin-2-yl]-2-phenoxyacetamide:

TLC: Rf 0.29 (hexane : ethyl acetate = 1 : 1);
NMR: δ 4.79 (s, 2H), 7.05 (m, 3H), 7.21 (m, 2H), 7.36 (m, 2H), 7.44 (d, J=5.31 Hz, 1H), 8.13 (m, 2H), 8.72 (d, J=5.31 Hz, 1H), 9.04 (s, 1H).

### Example 3(18)

### 2-(benzyloxy)-N-[4-(4-fluorophenyl)pyrimidin-2-yl]acetamide:

TLC: Rf 0.27 (hexane : ethyl acetate = 1 : 1);
NMR: δ 4.22 (s, 2H), 4.71 (s, 2H), 7.19 (m, 2H), 7.39 (m, 6H), 8.11 (m, 2H), 8.70 (d, J=5.49 Hz, 1H), 9.08 (s, 1H).

### Example 3(19)

### N-[4-(2,4-difluorophenyl)pyrimidin-2-yl]thiophene-2-carboxamide:

TLC: Rf 0.21 (hexane : ethyl acetate = 2 : 1);
NMR: δ 6.94 (m, 1H), 7.06 (m, 1H), 7.17 (dd, J=4.94, 3.84 Hz, 1H), 7.58 (dd, J=5.31, 1.83 Hz, 1H), 7.63 (dd, J=4.94, 1.10 Hz, 1H), 7.74 (dd, J=3.66, 1.10 Hz, 1H), 8.37 (m, 1H), 8.56 (s, 1H), 8.70 (d, J=5.31 Hz, 1H).

### Example 3(20)

### N-[4-(2,4-difluorophenyl)pyrimidin-2-yl]benzamide:

TLC: Rf 0.51 (hexane : ethyl acetate = 1 : 1);
NMR: δ 6.94 (m, 1H), 7.04 (m, 1H), 7.57 (m, 4H), 7.97 (m, 2H), 8.31 (m, 1H), 8.70 (m, 2H).

### Example 3(21)

### N-[4-(2-chlorophenyl)pyrimidin-2-yl]thiophene-2-carboxamide:

TLC: Rf 0.33 (hexane : ethyl acetate = 1 : 1);
NMR: δ 7.15 (dd, J=5.03, 3.75 Hz, 1H), 7.42 (m, 2H), 7.50 (m, 2H), 7.61 (dd, J=5.03, 1.19 Hz, 1H), 7.72 (m, 2H), 8.57 (s, 1H), 8.76 (d, J=5.13 Hz, 1H).

### Example 3(22)

### N-[4-(2-chlorophenyl)pyrimidin-2-yl]benzamide:

TLC: Rf 0.29 (hexane : ethyl acetate = 1 : 1);
NMR: δ 7.51 (m, 7H), 7.70 (m, 1H), 7.95 (m, 2H), 8.77 (m, 2H).

### Example 3(23)

### N-[4-(2-chlarophenyl)pyrimidin-2-yl]-2-phenoxyaeetamide:

TLC: Rf 0.48 (hexane: ethyl acetate= 1 : 1);
NMR: δ 4.78 (s, 2H), 7.03 (m, 3H), 7.34 (m, 2H), 7.42 (m, 2H), 7.50 (m, 2H), 7.69 (dd, J=6.04, 3.30 Hz, 1H), 8.77 (d, J=5.13 Hz, 1H), 9.06 (s, 1H).

### Example 3(24)

### N-[4-(4-chlorophenyl)pyrimidin-2-yl]thiophene-2-carboxamide:

TLC: Rf 0.46 (ethyl acetate : hexane = 2 : 1);
NMR: δ 7.17 (dd, J=5.03, 3.75 Hz, 1H), 7.44 (d, J=5.31 Hz, 1H), 7.49 (m, 2H), 7.63 (dd, J=5.03, 1.19 Hz, 1H), 7.74 (dd, J=3.75, 1.19 Hz, 1H), 8.09 (m, 2H), 8.54 (s, 1H), 8.71 (d, J=5.31 Hz, 1H).

### Example 3(25)

### N-[4-(4-chlorophenyl)pyrimidin-2-yl]benzamide:

TLC: Rf 0.27 (hexane : ethyl acetate = 1 : 1);
NMR: δ 7.54 (m, 6H), 7.98 (m, 2H), 8.07 (m, 2H), 8.74 (m, J=5.31 Hz, 2H).

### Example 3(26)

### N-[4-(4-chlorophenyl)pyrimidin-2-yl]-2-phenoxyacetamide:

TLC: Rf 0.39 (hexane : ethyl acetate = 1 : 1);
NMR: δ 4.79 (s, 2H), 7.06 (m, 3H), 7.36 (m, 2H), 7.48 (m, 3H), 8.06 (m, 2H), 8.73 (d, J=5.31 Hz, 1H), 9.04 (s, 1H).

### Example 3(27)

### N-[4-(3-bromophenyl)pyrimidin-2-yl]thiophene-2-carboxamide:

TLC: Rf 0.25 (hexane : ethyl acetate = 1 : 1);
NMR: δ 7.17 (dd, J=5.03, 3.75 Hz, 1H), 7.39 (t, J=7.96 Hz, 1H), 7.44 (d, J=5.13 Hz, 1H), 7.65 (m, 2H), 7.75 (dd, J=3.75, 1.19 Hz, 1H), 8.05 (m, 1H), 8.27 (t, J=1.83 Hz, 1H), 8.53 (s, 1H), 8.75 (d, J=5.31 Hz, 1H).

### Example 3 (28)

### N-[4-(3-bromophenyl)pyrimidin-2-yl]benzamide:

TLC: Rf 0.25 (hexane : ethyl acetate = 1 : 1);
NMR: δ 7.39 (t, J=7.87 Hz, 1H), 7.45 (d, J=5.31Hz, 1H), 7.59 (m, 4H), 7.99 (m, 3H), 8.25 (t, J=1.83 Hz, 1H), 8.68 (s, 1H), 8.77 (d, J=5.31 Hz, 1H).

### Example 3 (29)

### N-[4-(4-methoxyphenyl)pyrimidin-2-yl]thiophene-2-carboxamide:

TLC: Rf 0.33 (hexane : ethyl acetate = 2: 1);
NMR: δ 3.89 (s, 3H), 7.02 (m, 2H), 7.16 (dd, J=5.03, 3.75 Hz, 1H), 7.40 (d, J=5.49 Hz, 1H), 7.61 (dd, J=4.94, 1.10 Hz, 1H), 7.73 (dd, J=3.75, 1.19 Hz, 1H), 8.11 (m, 2H), 8.47 (s, 1H), 8.65 (d, J=5.31 Hz, 1H).

### Example 3(30)

### N-[4-(4-methoxyphenyl)pyrimidin-2-yl]benzamide:

TLC: Rf 0.19 (hexane : ethyl acetate = 1 : 1);
NMR: δ 3.88 (s, 3H), 7.00 (m, 2H), 7.39 (d, J=5.49 Hz, 1H), 7.53 (m, 3H), 7.96 (m, 2H), 8.07 (m, 2H), 8.64 (d, J=5.49 Hz, 1H), 8.76 (s, 1H).

### Example 3(31)

### N-[4-(3,4-dimethoxyphenyl)pyrimidin-2-yl]thiophene-2-carboxamide:

TLC: Rf 0.12 (hexane : ethyl acetate = 2 : 1);
NMR: δ 3.97 (s, 3H), 4.03 (s, 3H), 6.98 (d, J=8.42 Hz, 1H), 7.16 (dd, J=5.03, 3.75 Hz, 1H), 7.42 (d, J=5.31 Hz, 1H), 7.62 (dd, J=4.94, 1.10 Hz, 1H), 7.69 (dd, J=8.42,2.01 Hz, 1H), 7.75 (m, 2H), 8.55 (s, 1H), 8.66 (d, J=5.49 Hz, 1H).

### Example 3(32)

### N-[4-(3,4-dimethoxyphenyl)pyrimidin-2y]benzamide:

TLC: Rf 0.52 (methylene chloride : methanol = 9 : 1);
NMR: δ 3.96 (s, 3H), 4.00 (s, 3H), 6.96 (d, J=8.42 Hz, 1H), 7.41 (d, J=5.49 Hz, 1H), 7.54 (m, 3H), 7.66 (dd, J=8.42, 2.01 Hz, 1H), 7.74 (d, J=2.01 Hz, 1H), 7.96 (m, 2H), 8.65 (d, J=5.31 Hz, 1H), 8.74 (s, 1H).

### Example 3(33)

### N-{4-[4-(trifluoromethoxy)phenyl]pyrimidin-2-yl]thiophene-2-carboxamide:

TLC: Rf 0.44 (hexane : ethyl acetate = 1 : 2);
NMR(DMSO-d₆): δ 7.22 (dd, J=4.94, 3.84 Hz, 1H), 7.56 (m, 2H), 7.85 (d, J=5.31 Hz, 1H), 7.91 (dd, J=5.13, 1.10 Hz, 1H), 8.17 (dd, J=3.84, 1.10 Hz, 1H), 8.37 (m, 2H), 8.82 (d, J=5.31 Hz, 1H), 11.11 (s, 1H).

### Example 3(34)

### N-{4-[4-(trifluoromethoxy)phenyl]pyrimidin-2-yl}benzamide:

TLC: Rf 0.35 (hexane : ethyl acetate = 1 : 1);
NMR: δ 7.34 (m, J=8.06 Hz, 2H), 7.45 (d, J=5.31 Hz, 1H), 7.56 (m, 3H), 7.97 (m, 2H), 8.15 (m, 2H), 8.72 (d, J=5.31 Hz, 1H), 8.79 (s, 1H).

### Example 3(35)

### N-[4-(2,5-dimethoxyphenyl)pyrimidin-2-yl]benzamide:

TLC: Rf 0.48 (ethyl acetate : hexane = 2 : 1);
NMR: δ 3.86 (s, 3H), 3.87 (s, 3H), 6.96 (d, J=8.97 Hz, 1H), 7.02 (dd, J=8.97, 3.11 Hz, 1H), 7.53 (m, 3H), 7.65 (d, J=3.11 Hz, 1H), 7.78 (d, J=5.31 Hz, 1H), 7.96 (m, 2H), 8.66 (s, 1H), 8.70 (d, J=5.31 Hz, 1H).

### Example 3(36)

### N-[4-(2,5-dimethoxyphenyl)pyrimidin-2-yl]thiophene-2-carboxamide:

TLC: Rf 0.50 (ethyl acetate : hexane = 2 : 1);
NMR: δ 3.87 (s, 3H), 3.87 (s, 3H), 6.96 (d, J=8.79 Hz, 1H), 7.02 (dd, J=8.97, 2.92 Hz, 1H), 7.15 (dd, J=5.03, 3.75 Hz, 1H), 7.60 (dd, J=5.03, 1.19 Hz, 1H), 7.66 (d, J=2.75 Hz, 1H), 7.72 (dd, J=3.84, 1.10 Hz, 1H), 7.76 (d, J=5.31 Hz, 1H), 8.54 (s, 1H), 8.68 (d, J=5.31 Hz, 1H).

### Example 3(37)

### N-[4-(2,5-diisopropoxyphenyl)pyrimidin-2-yl]thiophene-2-carboxamide:

TLC: Rf 0.42 (ethyl acetate : hexane = 2 : 3);
NMR: δ 1.30 (d, J=6.04 Hz, 6H), 1.35 (d, J=6.04 Hz, 6H), 4.51 (m, 2H), 6.95 (m, 2H), 7.15 (dd, J=5.03, 3.75 Hz, 1H), 7.60 (m, 2H), 7.73 (dd, J=3.84, 1.10 Hz, 1H), 7.82 (d, J=5.31 Hz, 1H), 8.50 (s, 1H), 8.67 (d, J=5.31 Hz, 1H).

### Example 3(38)

### N-{4-[2,5-bis(difluoromethoxy)phenyl]pyrimidin-2-yl}thiophene-2-carboxamide:

TLC: Rf0.48 (ethyl acetate : hexane = 1 : 1);
NMR: δ 6.59 (m, 2H), 7.17 (dd, J=5.03, 3.75 Hz, 1H), 7.28 (m, 2H), 7.55 (d, J=5.13 Hz, 1H), 7.63 (dd, J=4.94, 1.10 Hz, 1H), 7.74 (dd, J=3.84, 1.10 Hz, 1H), 7.79 (m, 1H), 8.55 (s, 1H), 8.75 (d, J=5.31 Hz, 1H).

### Example 3(39)

### N-[4-(3-nitrophenyl)pyrimidin-2-yl]thiophene-2-carboxamide:

TLC: Rf 0.63 (methylene chloride : methanol =9:1);
NMR: δ 7.19 (dd, J=5.03, 3.75 Hz, 1H), 7.56 (d, J=5.31 Hz, 1H), 7.65 (dd, J=5.03, 1.19 Hz, 1H), 7.73 (t, J=7.96 Hz, 1H), 7.78 (dd, J=3.75, 1.19 Hz, 1H), 8.39 (m, 1H), 8.54 (m, 1H), 8.60 (s, 1H), 8.82 (d, J=5.31 Hz, 1H), 8.95 (t, J=2.01 Hz, 1H).

### Example 3(40)

### N-[4-(3-nitrophenyl)pyrimidin-2-yl]benzamide:

TLC: Rf 0.61 (methylene chloride : methanol = 9 : 1);
NMR: δ 7.58 (m, 4H), 7.72 (t, J=7.96 Hz, 1H), 8.00 (m, 2H), 8.38 (m, 1H), 8.50 (m, 1H), 8.76 (s, 1H), 8.83 (d, J=5.31 Hz, 1H), 8.95 (t, J=1.92 Hz, 1H).

### Example 3(41)

### N-[4-(3-nitrophenyl)pyrimidin-2-yl]-2-phenoxyacetamide:

TLC: Rf 0.55 (hexane : ethyl acetate = 1 : 3);
NMR: δ 4.79 (s, 2H), 7.06 (m, 3H), 7.37 (m, 2H), 7.58 (d, J=5.31 Hz, 1H), 7.73 (t, J=8.06 Hz, 1H), 8.39 (m, 1H), 8.49 (m, J=7.87, 1.10 Hz, 1H), 8.83 (d, J=5.31 Hz, 1H), 8.94 (t, J=2.0 Hz, 1H), 9.13 (s, 1H).

### Example 3(42)

### 2-(benzyloxy)-N-[4-(3-nitrophenyl)pyrimidin-2-yl]acetamide:

TLC: Rf 0.47 (hexane : ethyl acetate = 1 : 3);
NMR: δ 4.22 (s, 2H), 4.73 (s, 2H), 7.39 (m, 5H), 7.55 (d, J=5.31 Hz, 1H), 7.72 (t, J=7.96 Hz, 1H), 8.38 (m, 1H), 8.47 (m, 1H), 8.81 (d, J=5.31 Hz, 1H), 8.94 (t, J=2.01 Hz, 1H), 9.18 (s, 1H).

### Example 3(43)

### N-[4-(4-nitrophenyl)pyrimidin-2-yl]thiophene-2-carboxamide:

TLC: Rf 0.35 (hexane : ethyl acetate = 1 : 2);
NMR(DMSO-d₆): δ 7.23 (dd, J=4.94, 3.84 Hz, 1H), 7.92 (dd, J=5.13, 1.10 Hz, 1H), 7.97 (d, J=5.13 Hz, 1H), 8.18 (dd, J=3.75, 1.01 Hz, 1H), 8.40 (m, 2H), 8.50 (m, 2H), 8.90 (d, J=5.31Hz, 1H), 11.24 (s, 1H).

### Example 3(44)

### N-[4-(4-nitrophenyl)pyrimidin-2-yl]benzamide:

TLC: Rf 0.64 (methylene chloride : methanol = 20 : 1);
NMR: δ 7.58 (m, 4H), 7.99 (m, 2H), 8.33 (m, 4H), 8.74 (s, 1H), 8.82 (d, J=5.13 Hz, 1H).

### Example 3(45)

### N-{4-[3-(acetylamino)phenyl]pyrimidin-2-yl}thiophene-2-carboxamide:

TLC: Rf 0.53 (methylene chloride : methanol = 9 : 1);
NMR: δ 2.22 (s, 3H), 7.16 (dd, J=5.03, 3.75 Hz, 1H), 7.43 (m, 2H), 7.62 (dd, J=5.13, 1.10 Hz, 1H), 7.66 (s, 1H), 7.74 (dd, J=3.84, 1.10 Hz, 1H), 7.51 (m, 2H), 8.23 (m, 1H), 8.62 (s, 1H), 8.65 (d, J=5.13 Hz, 1H).

### Example 3(46)

### N-{4-[3-(acetylamino)phenyl]pyrimidin-2-yl}benzamide:

TLC: Rf 0.21 (hexane : ethyl acetate = 1 : 10);
NMR: δ 2.22 (s, 3H), 7.52 (m, 6H), 7.78 (m, J=7.69 Hz, 2H), 7.97 (m, 2H), 8.24 (m, 1H), 8.70 (m, J=5.31 Hz, 2H).

### Example 3(47)

### N-{4-[4-(acetylamino)phenyl]pyrimidin-2-yl}thiophene-2-carboxamide:

TLC: Rf 0.19 (methanol: methylene chloride = 1 : 19);
NMR(DMSO-d₆): δ 2.08 (s, 3H), 7.22 (m, 1H), 7.75 (m, 3H), 7.90 (d, J= 4.94 Hz, 1H), 8.17 (m, 3H), 8.72 (d, J=5.31 Hz, 1H), 10.21 (s, 1H), 11.02 (s, 1H).

### Example 3(48)

### N-{4-[4-(acetylamino)phenyl]pyrimidin-2-yl}benzamide:

TLC: Rf 0.22 (hexane : ethyl acetate = 1 : 10);
NMR: δ 2.22 (s, 3H), 7.43 (d, J=5.31 Hz, 1H), 7.54 (m, 4H), 7.67 (d, J=8.60 Hz, 2H), 7.97 (m, 2H), 8.09 (m, 2H), 8.68 (m, J=5.31 Hz, 2H).

### Example 3(49)

### N-[4-(1-naphthyl)pyrimidin-2-yl]thiophene-2-carboxamide;

TLC: Rf 0.26 (hexane : ethyl acetate = 1 : 1);
NMR: δ 7.14 (dd, J=4.94, 3.84 Hz, 1H), 7.38 (d, J=5.13 Hz, 1H), 7.58 (m, 4H), 7.70 (m, 2H), 7.96 (m, 2H), 8.24 (m, 1H), 8.63 (s, 1H), 8.84 (d, J=5.13 Hz, 1H).

### Example 3(50)

### N-[4-(1-naphthyl)pyrimidin-2-yl]benzamide:

TLC: Rf 0.33 (hexane : ethyl acetate = 1 : 1);
NMR: δ 7.39 (d, J=5.13 Hz, 1H), 7.54 (m, 6H), 7.69 (m, 1H), 7.96 (m, 4H), 8.25 (m, 1H), 8.76 (s, 1H), 8.86 (d, J=5.13 Hz, 1H).

### Example 3(51)

### N-[4-(2-naphthyl)pyrimidin-2-yl]thiophene-2-carboxamide:

TLC: Rf 0.25 (hexane : ethyl acetate = 1 : 1);
NMR: δ 7.18 (dd, J=5.03, 3.75 Hz, 1H), 7.59 (m, 4H), 7.77 (dd, J=3.75, 1.19 Hz, 1H), 7.94 (m, 3H), 8.21 (dd, J=8.60, 1.83 Hz, 1H), 8.60 (s, 1H), 8.64 (d, J=1.65 Hz, 1H), 8.76 (d, J=5.31 Hz, 1H).

### Example 3(52)

### N-[4-(2-naphthyl)pyrimidin-2-yl]benzamide:

TLC: Rf 0.29 (hexane : ethyl acetate = 1 : 1);
NMR: δ 7.54 (m, 6H), 7.92 (m, 5H), 8.14 (dd, J=8.70, 1.74 Hz, 1H), 8.57 (d, J=1.28 Hz, 1H), 8.70 (d, J=5.31 Hz, 1H), 8.96 (s, 1H).

### Example 3(53)

### N-[4-(6-methoxy-2-naphthyl)pyrimidin-2-yl]thiophene-2-carboxamide:

TLC: Rf 0.35 (hexane : ethyl acetate = 1 : 2);
NMR(DMSO-d₆): δ 3.91 (s, 3H), 7.24 (m, 2H), 7.41 (d, J=2.56 Hz, 1H), 7.95 (m, 4H), 8.19 (dd, J=3.84, 1.10 Hz, 1H), 8.32 (dd, J=8.60, 1.83 Hz, 1H), 8.77 (m, 2H), 11.09 (s, 1H).

### Example 3(54)

### N-[4-(6-methoxy-2-naphthyl)pyrimidin-2-yl]benzamide:

TLC: Rf 0.76 (methylene chloride : methanol = 9 : 1);
NMR: δ 3.96 (s, 3H), 7.20 (m, 2H), 7.56 (m, 4H), 7.86 (m, J=8,33 Hz, 2H), 8.00 (m, 2H), 8.16 (dd, J=8.70, 1.92 Hz, 1H), 8.55 (d, J=1.83 Hz, 1H), 8.70 (s, 1H), 8.74 (d, J=5.31 Hz, 1H).

### Example 3(55)

### N-(4-phenylpyrimidin-2-yl)thiophene-2-carboxamide:

TLC: Rf 0.21 (hexane : ethyl acetate = 1 : 1);
NMR: δ 7.15 (dd, J=5.03, 3.75 Hz, 1H), 7.46 (d, J=5.31 Hz, 1H), 7.51 (m, 3H), 7.61 (dd, J=5.03, 1.19 Hz, 1H), 7.75 (dd, J=3.75, 1.19 Hz, 1H), 8.11 (m, 2H), 8.66 (m, 1H), 8.70 (d, J=5.31 Hz, 1H).

### Example 3(56)

### N-benzoyl-N-(4-phenylpyrimidin-2-yl)benzamide:

TLC: Rf 0.75 (hexane : ethyl acetate = 2 : 3);
NMR: δ 7.43 (m, 10 H), 7.76 (m, 2H), 7.88 (m, 4H), 8.66 (d, J=5.31 Hz, 1H).

### Example 3(57)

### N-(5,6-dihydrobenzo[h]quinazolin-2-yl)thiophene-2-carboxamde

TLC: Rf 0.42 (hexane : ethyl acetate = 2 : 3);
NMR: δ 2.96 (m, 4H), 7.16 (dd, J=5.03, 3.75 Hz, 1H), 7.27 (m, 1H), 7.41 (m, 2H), 7.60 (dd, J=4,94, 1.10 Hz, 1H), 7.74 (dd, J=3.75, 1.19 Hz, 1H), 8.35 (m, 1H), 8.49 (m, 2H).

### Example 3(58)

### N-(5,6-dihydrobenzo[h]quinazolin-2-yl)-N-(thiophen-2-ylcarbonyl)thiophene-2-carboxamide:

TLC: Rf 0.71 (hexane : ethyl acetate = 2 : 3);
NMR: δ 2.96 (m, 4H), 7.02 (dd, J=4.94, 3.84 Hz, 2H), 7.29 (m, 3H), 7.60 (m, 4H), 8.04 (dd, J=7.60, 1.56 Hz, 1H), 8.48 (s, 1H).

### Example 3(59)

### N-(5,6-dihydrobenzo[h]quinazolin-2-yl)benzamide:

TLC: Rf 0.37 (hexane : ethyl acetate = 2 : 3);
NMR: δ 2.95 (m, 4H), 7.45 (m, 6H), 7.97 (m, 2H), 8.31 (m, 1H), 8.51 (s, 1H), 8.62 (s, 1H).

### Example 3(60)

### N-benzoyl-N-(5,6-dihydrobenzo[h]quinazolin-2-yl)benzamide:

TLC: Rf 0.77 (hexane : ethyl acetate = 2 : 3);
NMR: δ 2.90 (m, 4H), 7.37 (m, 9H), 7.88 (m, 5H), 8.43 (s, 1H).

### Example 3(61)

### N-(4-phenylpyrimidin-2-yl)benzamide:

TLC: Rf 0.37 (ethyl acetate : hexane = 2 : 1);
NMR: δ 8.80-8.63 (m, 2H), 8.16-8.05 (m, 2H), 8.01-7.96 (m, 2H), 7.64-7.43 (m, 7H).

### Example 3(62)

### N-benzoyl-N-[4-(4-methylphenyl)pyrimidin-2-yl]benzamide:

TLC: Rf 0.45 (hexane : ethyl acetate = 2 : 1);
NMR: δ 2.38 (s, 3H), 7.20 (d, J=8.1 Hz, 2H), 7.35-7.51 (m, 7H), 7.68 (d, 8.1 Hz, 2H), 7.85-7.89 (m, 4H), 8.62 (d, J=5.1 Hz, 1H).

### Example 3(63)

### N-[4-(4-methylphenyl)pyrimidin-2-yl]-N-(thiophen-2-ylcarbonyl)thiophene-2-carboxamide:

TLC: Rf 0.36 (hexane : ethyl acetate = 2 : 1);
NMR: δ 2.39 (s, 3H), 7.01 (dd, J=4.8, 1.2 Hz, 2H), 7.23 (d, J=8.1 Hz, 2H), 7.50 (d, J=5.4 Hz, 1H), 7.58-7.62 (m, 4H), 7.80 (d, J=8.1 Hz, 2H), 8.67 (d, J=5.4 Hz, 1H).

### Example 3(64)

### N-[4-(4-cyanophenyl)pyrimidin-2-yl]-2-phenoxyacetamide:

NMR: δ 4.77 (s, 2H), 6.99-7.13 (m, 3H), 7.31-7.43 (m, 2H), 7.52 (d, J=5.13 Hz, 1H), 7.78-7.87 (m, 2H), 8.19-8.29 (m, 2H), 8.80 (d, J=5.31 Hz, 1H), 9.1 (s, 1H).

### Example 3(65)

### N-[4-(4-methoxyphenyl)pyrimidin-2-yl]-2-phenoxyacetamide:

NMR: δ 3.89 (s, 3H), 4.81 (s, 2H), 6.97-7.10 (m, 5H), 7.30-7.40 (m, 2H), 7.42 (d, J=5.49 Hz, 1H), 8.04-8.15 (m, 2H), 8.66 (d, J=5.31 Hz, 1H), 9.00 (s, 1H).

### Example 3(66)

### 2-phenoxy-N-{4-[4-(trifluoromethoxy)phenyl]pyrimidin-2-yl}acetamide:

NMR: δ 4.79 (s, 2H), 6.98-7.13 (m, 3H), 7.31-7.43 (m, 4H), 7.47 (d, J=5.31 Hz, 1H), 8.09-8.22 (m, 2H), 8.75 (d, J=5.31 Hz, 1H), 9.06 (s, 1H).

### Example 3(67)

### N-[4-(3,4-dimethoxyphenyl)pyrimidin-2-yl]-2-phenoxyacetamide:

NMR: δ 3.97 (s, 3H), 4.01 (s, 3H), 4.80 (s, 2H), 6.97 (d, J=8.42 Hz, 1H), 7.01-7.11 (m, 3H), 7.31-7.40 (m, 2H), 7.44 (d, J=5.31 Hz, 1H), 7.67 (dd, J=8.42, 2.20 Hz, 1H), 7.75 (d, J=2.01 Hz, 1H), 8.67 (d, J=5.49 Hz, 1H), 9.01 (s, 1H).

### Example 3(68)

### N-{4-[2,5-bis(difluoromethoxy)phenyl]pyrimidin-2-yl}-5-chlorothiophene-2-carboxamide:

TLC: Rf 0.28 (ethyl acetate : hexane =1 : 2);
NMR: δ 8.74 (d, J=5 Hz, 1H), 8.45 (br s, 1H), 7.78-7.73 (m, 1H), 7.55 (d, J=5 Hz, 1H), 7.49 (d, J=5 Hz, 1H), 7.33-7.23 (m, 2H), 6.98 (d, J=5 Hz, 1H), 6.58 (t, J=75 Hz, 1H), 6.57 (t, J=75 Hz, 1H).

### Example 4

### N-{4-[2,5-bis(difluoromethoxy)phenyl]pyrimidin-2-yl}-N'-phenylurea:

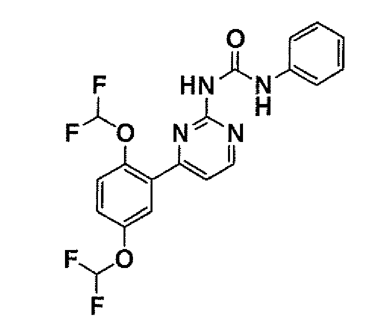

A solution of 4-[2,5-bis(difluoromethoxy)phenyl]-2-pyrimidinamine (100 mg, 0.33 mmol) in anhydrous tetrahydrofuran (1 mL) was added by isocynatobenzene (38 µL, 0.35 mmol) at room temperature and stirred overnight. The reaction solution was added by water and extracted by ethyl acetate. The organic layer was washed with saturated brine, dried over anhydrous sodium sulfate and then concentrated. The residue was purified by column chromatography on silica gel (hexane : ethyl acetate = from 3 : 1 to 1 : 1) to give the compound (87 mg) of the present invention having the following physical data.
TLC: Rf 0.37 (hexane : ethyl acetate = 2 : 1);
NMR: δ 6.54 (m, 2H), 7.10 (t, J=7.41 Hz, 1H), 7.34 (m, 4H), 7.44 (d, J=5.31 Hz, 1H), 7.58 (d, J=8.42 Hz, 2H), 7.70 (s, 1H), 8.00 (s, 1H), 8.67 (d, J=5.13 Hz, 1H), 11.25 (s, 1H).

### Example 4(1)

N-{4-[2,5-bis(difluoromethoxy)phenyl]pyrimidin-2-yl}-N'-isopropylurea:

By the same procedure as described in Example 4 using 2-isocyanatopropane instead of isocyanatobenzene, the compound of the present invention having the following physical data was obtained.
TLC: Rf 0.35 (hexane : ethyl acetate = 2 : 1);
NMR: δ 1.26 (d, J=6.41 Hz, 6H), 4.10 (m, 1H), 6.52 (m, 2H), 7.29 (d, J=1.65 Hz, 2H), 7.36 (d, J=5.12 Hz, 1H), 7.42 (s, 1H), 7.64 (t, J=1.65 Hz, 1H), 8.54 (d, J=5.31 Hz, 1H), 8.83 (d, J=8.60 Hz, 1H).

### Example 5(1)-5(7)

By the same procedure as described in Example 1, Example 2 and Example 3 using the corresponding ketone instead of acetophenone and the corresponding sulfonic acid chloride instead of benzyloxyacetylchloride, the following compound of the present invention was obtained.

### Example 5(1)

### N-{4-[2,5-bis(difluoromethoxy)phenyl]pyrimidin-2-yl}methanesulfonamide:

TLC: Rf 0.35 (hexane : ethyl acetate = 2 : 1);
NMR: δ 3.49 (s, 3H), 6.55 (m, 2H), 7.29 (d, J=1.46 Hz, 2H), 7.56 (d, J=5.31 Hz, 1H), 7.80 (m, 1H), 8.69 (d, J=5.31 Hz, 1H), 8.98 (s, 1H).

### Example 5(2)

### N-{4-[2,5-bis(difluoromethoxy)phenyl]pyrimidin-2-yl}benzenesulfonamide:

TLC: Rf 0.28 (toluene : ethyl acetate = 5 : 1);
NMR: δ 6.52 (m, 2H), 7.26 (m, 2H), 7.56 (m, 5H), 8.14 (m, 2H), 8.77 (d, J=5.49 Hz, 1H), 10.94 (s, 1H).

### Example 5(3)

### N-{4-[2,5-bis(difluoromethoxy)phenyl]pyrimidin-2-yl}-4-methylbenzenesulfonamide:

TLC: Rf 0.28 (toluene : ethyl acetate = 5 : 1);
NMR: δ 2.41 (s, 3H), 6.52 (m, 2H), 7.24 (m, 2H), 7.31 (d, J=8.06 Hz, 2H), 7.51 (d, J=5.31 Hz, 1H), 7.63 (d, J=2.75 Hz, 1H), 8.02 (d, J=8.42 Hz, 2H), 8.68 (d, J=5.31 Hz, 1H), 9.84 (s, 1H).

### Example 5(4)

### N-{4-[2,5-bis(difluoromethoxy)phenyl]pyrimidin-2-yl}-1-phenylmethanesulfonamide;

TLC: Rf0.41 (toluene : ethyl acetate = 5 : 1);
NMR: δ 4.86 (s, 2H), 6.57 (m, 2H), 7.30 (m, 7H), 7.55 (d, J=5.31 Hz, 1H), 7.87 (m, 1H), 8.54 (d, J=5.31 Hz, 1H), 9.23 (s, 1H).

### Example 5(5)

### N-{4-[2,5-bis(2,2,2-trifluoroethoxy)phenyl]pyrimidin-2-yl}benzenesulfonamide:

TLC: Rf 0.36 (toluene : ethyl acetate = 5 : 1);
NMR: δ 4.37 (m, 4H), 6.91 (d, J=9.15 Hz, 1H), 7.04 (dd, J=8.97, 3.34 Hz, 1H), 7.55 (m, 4H), 7.63 (d, J=5.49 Hz, 1H), 8.15 (m, 2H), 8.64 (d, J=5.31 Hz, 1H), 9.76 (s, 1H).

### Example 5(6)

### N-{4-[2,5-bis(2,2,2-trifluoroethoxy)phenyl]pyrimidin-2-yl}-4-methylbenzensulfonamide:

TLC: Rf 0.36 (toluene : ethyl acetate = 5 : 1);
NMR: δ 2.41 (s, 3H), 4.37 (m, 4H), 6.91 (d, J=9.15 Hz, 1H), 7.04 (dd, J=8.97, 3.30 Hz, 1H), 7.31 (m, 2H), 7.57 (d, J=3.30 Hz, 1H), 7.62 (d, J=5.49 Hz, 1H), 8.03 (d, J=8.24 Hz, 2H), 8.63 (d, J=5.49 Hz, 1H), 9.74 (s, 1H).

### Example 5(7)

### N-{4-[2,5-bis(2,2,2-trifluoroethoxy)phenyl]pyrimidin-2-yl}methanesulfonamide:

TLC: Rf 0.24 (toluene : ethyl acetate = 5 : 1);
NMR: δ 3.48 (s, 3H), 4.41 (m, 4H), 6.96 (d, J=9.15 Hz, 1H), 7.11 (dd, J=9.15, 3.11 Hz, 1H), 7.69 (d, J=5.49 Hz, 1H), 7.74 (d, J=3.11 Hz, 1H), 8.66 (d, J=5.31 Hz, 1H), 8.99 (s, 1H).

### Example 6(1)-(6)

By the same procedure as described in Example 1 and Example 2 using the corresponding ketone instead of acetophenone, the following compounds were obtained.

### Example 6(1)

### 4-[2,5-bis(difluoromethoxy)phenyl]pyrimidin-2-amine:

TLC: Rf 0.40 (ethyl acetate : hexane = 1 : 1);
NMR: δ 5.11 (s, 2H), 6.51 (m, 2H), 7.08 (d, J=5.13 Hz, 1H), 7.24 (m, 2H), 7.64 (d, J=2.75 Hz, 1H), 8.37 (d, J=5.13 Hz, 1H).

### Example 6(2)

### 4-[2,5-bis(2,2,2-trifluoroethoxy)phenyl]-2-pyrimidinamine:

TLC: Rf 0.34 (hexane : ethyl acetate = 2 : 1);
NMR: δ 4.36 (m, 4H), 5.06 (s, 2H), 6.94 (d, J=8.97 Hz, 1H), 7.04 (dd, J=8.97, 3.30 Hz, 1H), 7.22 (d, J=5.13 Hz, 1H), 7.55 (d, J=3.30 Hz, 1H), 8.35 (d, J=5.13 Hz, 1H).

### Example 6(3)

### 4-(2,5-dimethoxyphenyl)pyrimdin-2-amine:

TLC: Rf 0.23 (ethyl acetate : hexane = 1 : 1);
NMR: δ 3.83 (s, 6H), 5.05 (bs, 2H), 6.91-6.99 (m, 2H), 7.23-7.24 (m, 1H), 7.45 (dd, J=2.8, 0.7 Hz, 1H), 8.30 (d, J=5.3 Hz, 1H).

### Example 6(4)

### 4-(2,5-diisopropoxyphenyl)pyrimidin-2-amine:

TLC: Rf 0.52 (ethyl acetate : hexane = 1 : 1);
NMR: δ 1.27 (d, J=6.0 Hz, 6H), 1.33 (d, J=6.2 Hz, 6H), 4.32-4.44 (m, 1H), 4.47-4.58 (m, 1H), 5.02 (s, 2H), 6.90-6.91 (m, 2H), 7.33 (d, J=5.3 Hz, 1H), 7.43-7.45 (m, 1H), 8.28 (d, J=5.3 Hz, 1H).

### Example 6(5)

### 2-(2-aminopyrimidin-4-yl)-4-(difluoromethoxy)phenol:

TLC: Rf 0.38 (ethyl acetate : hexane = 1 : 1);
NMR(DMSO-d₆): δ 6.94 (d, J=9.0 Hz, 1H), 7.13 (t, J=74.7 Hz, 1H), 7.15-7.24 (m, 1H), 7.28 (d, J=5.5 Hz, 1H), 7.75 (d, J=2.9 Hz, 1H), 8.38 (d, J=5.5 Hz, 1H), 13.7 (s, 1H).

### Example 6(6)

### 2-(2-aminopyrimidin-4-yl)-4-(difluoromethoxy)phenyl thiophene-2-carboxylate:

TLC: Rf 0.19 (ethyl acetate : hexane = 2 : 3);
NMR: δ 4.91 (s, 2H), 6.57 (t, J=73.4 Hz, 1H), 6.94 (d, J=5.3 Hz, 1H), 7.18 (dd, J=4.9, 3.8 Hz, 1H), 7.23-7.38 (m, 2H), 7.61 (d, J=2.8 Hz, 1H), 7.68 (dd, J=4.9, 1.3 Hz, 1H), 7.93 (dd, J=3.8, 1.3 Hz, 1H), 8.28 (d, J=5.3 Hz, 1H).

### Example 7(1)-(14)

By the same procedure as described in Example 1, Example 2 and Example 3 using the corresponding ketone instead of acetophenone and the corresponding acid chloride or sulfonic acid chloride instead of benzyloxyacetylchloride, the following compounds of the present invention were obtained.

### Example 7(1)

### N-(4-phenylpyrimidin-2-yl)benzenesulfonamide:

TLC: Rf 0.45 (ethyl acetate : hexane = 1 : 1);
NMR(DMSO-d₆): δ 7.54 (m, 8H), 8.00 (m, 4H), 8.53 (d, J=5.31 Hz, 1H).

### Example 7(2)

### N-{4-[3-(trifluoromethyl)phenyl]pyrimidin-2-yl}benzenesulfonamide:

TLC: Rf 0.45 (hexane : ethyl acetate = 2 : 1);
NMR: δ 7.39 (d, J=5.31 Hz, 1H), 7.56 (m, 4H), 7.77 (d, J=7.69 Hz, 1H), 8.11 (d, J=8.06 Hz, 1H), 8.18 (m, 2H), 8.24 (s, 1H), 8.71 (d, J=5.31 Hz, 1H), 9.89 (s, 1H).

### Example 7(3)

N-[4-(2-chlorophenyl)pyrimidin-2-yl]benzenesulfonamide:
TLC: Rf 0.63 (hexane : ethyl acetate = 2 : 1);
NMR: δ 7.45 (m, 7H), 7.59 (m, 1H), 8.14 (m, 2H), 8.70 (d, J=5.31 Hz, 1H), 10.29 (s, 1H).

### Example 7(4)

### N-[4-(4-fluorophenyl)pyrimidin-2-yl]benzenesulfonamide:

TLC: Rf 0.31 (hexane : ethyl acetate = 2 : 1);
NMR(DMSO-d₆): δ 7.36 (t, J=8.79 Hz, 2H), 7.59 (m, 4H), 8.04 (m, 4H), 8.54 (d, J=5.31 Hz, 1H), 11.91 (s, 1H).

### Example 7(5)

### N-[4-(4-methylphenyl)pyrimidin-2-yl]benzenesulfonamide:

TLC: Rf 0.43 (hexane : ethyl acetate = 2 : 1);
NMR(DMSO-d₆): δ 2.36 (s, 3H), 7.31 (d, J=8.06 Hz, 2H), 7.57 (m, 4H), 7.89 (m, 2H), 8.00 (m, 2H), 8.50 (d, J=5.49 Hz, 1H), 11.90 (s, 1H).

### Example 7(6)

### N-[4-(4-cyanophenyl)pyrimidin-2-yl]benzenesulfonamide:

TLC: Rf 0.44 (hexane : ethyl acetate = 1 : 1);
NMR(DMSO-d₆): δ 7.59 (m, 3H), 7.72 (d, J=5.31 Hz, 1H), 8.02 (m, 4H), 8.16 (m, 2H), 8.65 (d, J=5.31 Hz, 1H), 12.03 (s, 1H).

### Example 7(7)

### N-[4-(2,5-diisopropoxyphenyl)pyrimidin-2-yl]benzenesulfonamide:

TLC: Rf 0.34 (hexane : ethyl acetate = 2 : 1);
NMR: δ 1.29 (d, J=6.04 Hz, 6H), 1.37 (d, J=6. 04 Hz, 6H), 4.51 (m, 2H), 6.89 (d, J=8.97 Hz, 1H), 6.96 (dd, J=8.97, 2.93 Hz, 1H), 7.52 (m, 4H), 7.77 (d, J=5.49 Hz, 1H), 8.17 (m, 2H), 8.57 (d, J=5.31 Hz, 1H), 10.08 (s, 1H).

### Example 7(8)

### N-{4-[2,5-bis(2,2,2-trifluoroethoxy)phenyl]pyrimidin-2-yl}-1-phenylmethanesulfonamide:

TLC: Rf 0.44 (hexane : ethyl acetate = 2 : 1);
NMR: δ 4.43 (m, 4H), 4.86 (s, 2H), 6.97 (d, J=8.97 Hz, 1H), 7.12 (dd, J=8.97, 3.11 Hz, 1H), 7.32 (s, 5H), 7.70 (d, J=5.31 Hz, 1H), 7.84 (d, J=3.11 Hz, 1H), 8.51 (d, J=5.31 Hz, 1H), 8.90 (s, 1H).

### Example 7(9)

### N-[4-(2,5-dimethoxyphenyl)pyrimidin-2-yl]benzenesulfonamide:

TLC: Rf 0.31 (ethyl acetate : hexane = 1 : 1);
NMR: δ 3.84 (s, 3H), 3.88 (s, 3H), 6.93 (d, J=8.97 Hz, 1H), 7.01 (m, 1H), 7.49 (m, 3H), 7.62 (d, J=3.30 Hz, 1H), 7.73 (d, J=5.49 Hz, 1H), 8.16 (m, 2H), 8.67 (d, J=5.49 Hz, 1H), 10.99 (m, 1H).

### Example 7(10)

### N-{4-[2,5-bis(difluoromethoxy)phenyl]pyrimidin-2-yl}-3-methylbenzenesulfonamide:

TLC: Rf 0.63 (hexane : ethyl acetate = 1 : 1);
NMR: δ 2.36 (s, 3H), 6.16-6.84 (m, 2H), 7.17-7.32 (m, 2H), 7.33-7.42 (m, 2H), 7.52 (d, J=5.49 Hz, 1H), 7.64 (d, J=2.56 Hz, 1H), 7.84-8.01 (m, 2H), 8.72 (d, J=5.49 Hz, 1H), 10.38 (s, 1H).

### Example 7(11)

### N-{4-[2,5-bis(difluoromethoxy)phenyl]pyrimidin-2-yl}-3,5-dimethylbenzenesulfonamide:

TLC: Rf 0.63 (hexane : ethyl acetate = 1 : 1);
NMR: δ 2.31 (s, 6H), 6.16-6.84 (m, 2H), 7.18 (s, 1H), 7.22-7.29 (m, 2H), 7.51 (d, J=5.49 Hz, 1H), 7.67 (d, J=2.38 Hz, 1H), 7.73 (s, 2H), 8.70 (d, J=5.49 Hz, 1H), 10.14 (s, 1H).

### Example 7(12)

### N-{4-[2,5-bis(difluoromethoxy)phenyl]pyrimidin-2-yl}-3,5-dichlorobenzenesulfonamide:

TLC: Rf 0.45 (hexane : ethyl acetate = 1 : 1);
NMR: δ 6.19-6.87 (m, 2H), 7.20-7.37 (m, 2H), 7.48-7.58 (m, 2H), 7.68 (d, J=2.38 Hz, 1H), 8.02 (d, J=1.83 Hz, 2H), 8.68 (d, J=5.31 Hz, 1H), 9.84 (s, 1H).

### Example 7(13)

### N-{4-[2,5-bis(difluoromethoxy)phenyl]pyrimidin-2-yl}-4-(methylsulfonyl)benzenesulfonamide:

TLC: Rf 0.13 (hexane : ethyl acetate = 1 : 1);
NMR: δ 3.07 (s, 3H), 6.23-6.92 (m, 2H), 7.26 (d, J=8.96 Hz, 1H), 7.32 (dd, J=8.96, 2.93 Hz, 1H), 7.57-7.62 (m, 2H), 8.08 (d, J=8.78 Hz, 2H), 8.35 (d, J=8.78 Hz, 2H), 8.72 (d, J=5.49 Hz, 1H), 10.56 (s, 1H).

### Example 7(14)

### N-{4-[2,5-bis(2,2,2-trifluoroethoxy)phenyl]pyrimidin-2-yl}acetamide:

TLC: Rf 0.52 (hexane : ethyl acetate = 1 : 1);
NMR: δ 2.56 (s, 3H), 4.30-4.45 (m, 4H), 6.96 (d, J=8.97 Hz, 1H), 7.09 (dd, J=8.97, 3.30 Hz, 1H), 7.64 (d, J=5.31 Hz, 1H), 7.66 (d, J=3.30 Hz, 1H), 8.05 (s, 1H), 8.61 (d, J=5.31 Hz, 1H).

### Example 8:

N-benzyl-N-{4-[2,5-bis(difluoromethoxy)phenyl]pyrimidin-2-yl}methanesulfonamide:

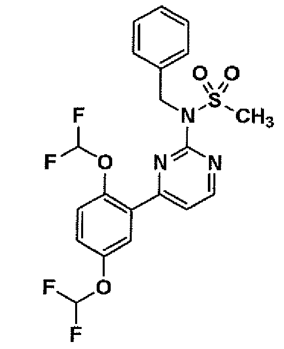

A solution of the compound (75 mg, 0.20 mmol) prepared in Example 5(1) in anhydrous dimethylformamide (1 mL) was added by potassium carbonate (41 mg, 0.30 mmol) and benzylbromide (28 µL, 0.24 mmol) and stirred at room temperature until materials disappearing. The reaction solution was diluted with a mixed solvate of hexane and ethyl acetate, washed with saturated sodium hydrogen carbonate aqueous solution, water and saturated brine successively, dried over anhydrous sodium sulfate and then concentrated. The residue was purified by column chromatography on silica gel (hexane : ethyl acetate = 2 : 1) to give the compound (64 mg) of the present invention having the following physical data.
TLC: Rf0.57 (hexane : ethyl acetate = 2 : 1);
NMR: δ 3.46 (s, 3H), 5.44 (s, 2H), 6.49 (m, 2H), 7.20-7.36 (m, 5H), 7.41 (m, 2H), 7.53 (d, J=5.13 Hz, 1H), 7.68 (s, 1H), 8.65 (d, J=5.13 Hz, 1H).

### Example 8(1)-(5)

By the same procedure as described in Example 8 using the compound prepared in Example 5(2) or Example 7(9) instead of the compound prepared in Example 5(1) and the corresponding halide compound instead of benzylbromide, the following compounds of the present invention were obtained.

### Example 8(1)

### N-[4-(2,5-dimethoxyphenyl)pyrimidin-2-yl]-N-methylbenzenesulfonamide:

TLC: Rf 0.37 (ethyl acetate : hexane = 1 : 3);
NMR: δ 3.76 (s, 3H), 3.82 (s, 3H), 3.86 (s, 3H), 6.92 (d, J=8.97 Hz, 1H), 6.99 (m, 1H), 7.42 (m, 2H), 7.49 (m, 1H), 7.62 (m, 2H), 8.07 (m, 2H), 8.43 (d, J=5.31 Hz, 1 H).

### Example 8(2)

### N-{4-[2,5-bis(difluoromethoxy)phenyl]pyrimidin-2-yl}-N-methylbenzenesulfonamide:

TLC: Rf 0.47 (hexane : ethyl acetate = 2 : 1);
NMR: δ 1.52 (t, J=6.96 Hz, 3H), 4.41 (q, J=6.96 Hz, 2H), 6.12-6.86 (m, 2H), 7.13-7.28 (m, 2H), 7.34-7.67 (m, 5H), 7.97-8.11 (m, 2H), 8.51 (d, J=5.13 Hz, 1H).

### Example 8(3)

### N-{4-[2,5-bis(difluoromethoxy)phenyl]pyrimidin-2-yl}-N-propylbenzenesulfonamide:

TLC: Rf 0.51 (hexane : ethyl acetate = 2 : 1);
NMR: δ 1.04 (t, J=7.41 Hz, 3H), 1.86-2.03 (m, 2H), 4.22-4.33 (m, 2H), 6.13-6.82 (m, 2H), 7.16-7.25 (m, 2H), 7.37-7.47 (m, 3H), 7.48-7.56 (m, 1H), 7.58 (d, J=2.56 Hz, 1H), 7.96-8.12 (m, 2H), 8.50 (d, J=5.13 Hz, 1H).

### Example 8(4)

### N-{4-[2,5-bis(difluoromethoxy)phenyl]pyrimidin-2-yl}-N-isobuthylbenzenesulfonamide:

TLC: Rf 0.51 (hexane : ethyl acetate = 2 : 1);
NMR: δ 1.04 (d, J=6.59 Hz, 6H), 2.29-2.48 (m, 1H), 4.18 (d, J=7.32 Hz, 2H), 6.05-6.89 (m, 2H), 7.16-7.25 (m, 2H), 7.35-7.46 (m, 3H), 7.46-7.55 (m, 1H), 7.63 (d, J=2.75 Hz, 1H), 7.97-8.05 (m, 2H), 8.49 (d, J=5.31 Hz, 1H).

### Example 8(5)

### N-{4-[2,5-bis(difluoromethoxy)phenyl]pyrimidin-2-yl}-N-(2-methoxymethyl)benzenesulfonamide:

TLC: Rf 0.33 (hexane : ethyl acetate = 2 : 1);
NMR: δ 3.42 (s, 3H), 3.83 (t, J=6.13 Hz, 2H), 4.56 (t, J=6.13 Hz, 2H), 6.12-6.83 (m, 2H), 7.15-7.25 (m, 2H), 7.35-7.48 (m, 3H), 7.49-7.63 (m, 2H), 8.03-8.15 (m, 2H), 8.51 (d,J=5.31Hz, 1H).

### Example 9:

### 4-[2,5-bis(2,2,2-trifluoroethoxy)phenyl]-N-ethyl-2-pyrimidinamine:

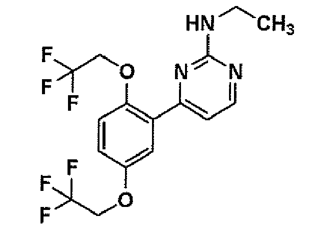

A solution of the compound (205 mg, 0.50 mmol) prepared in Example 7(14) in anhydrous tetrahydrofuran (2 mL) was added by lithium aluminum hydride (38 mg, 1.00 mmol) at room temperature and stirred for an hour. The reaction solution was added by t-butylmethylether, added by 2N sodium hydroxide aqueous solution (0.25 mL) under ice and stirred at room temperature for 30 minutes. The reaction solution was filtrated by sellite (trade name) and the filtrate was extracted by t-butylmethylether. The organic layer was washed with saturated ammonium chloride solution and saturated brine successively, dried over anhydrous sodium sulfate and then concentrated. The residue was purified by column chromatography on silica gel (hexane : ethyl acetate = 5 : 1) to give the compound (107 mg) of the present invention having the following physical data.
TLC: Rf 0.48 (hexane : ethyl acetate = 2 : 1);
NMR: δ 1.28 (t, J=7.14 Hz, 3H), 3.52 (m, 2H), 4.35 (m, 4H), 5.09 (s, 1H), 6.94 (d, J=8.79 Hz, 1H), 7.02 (dd, J=8.79, 3.12 Hz, 1H), 7.12 (d, J=5.31 Hz, 1H), 7.59 (d, J =3.12 Hz, 1H), 8.33 (d, J=5.31 Hz, 1H).

### Example 10:

### N-{4-[2,5-bis(2,2,2-trifluoroethoxy)phenyl]pyrimidin-2yl}-N-methylbenzenesulfonamide:

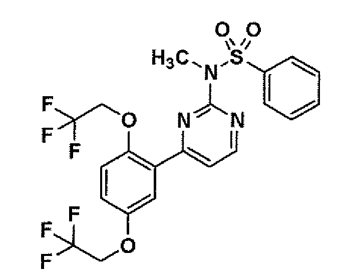

A mixed solution of the compound (100 mg, 0.20 mmol) prepared in Example 5(5) in anhydrous N,N-dimethylformamide (1 mL) and anhydrous tetrahydrofuran (0.2 mL) was added by sodium hydride (60% in oil, 12 mg, 0.30 mmol) under ice and stirred for five minutes under ice and for five minutes at room temperature. The reaction solution was added by methyl iodide (15 µL, 0.24 mmol) and stirred at room temperature until materials disappearing. The reaction solution was added by saturated ammonium chloride aqueous solution and extracted by ethyl acetate. The organic layer was washed with saturated brine, dried over anhydrous sodium sulfate and concentrated. The residue was purified by column chromatography on silica gel (hexane : ethyl acetate = 3 : 1) to give the compound (85 mg) of the present invention having the following physical data.
TLC: Rf 0.28 (hexane : ethyl acetate = 2 : 1);
NMR: δ 3.77 (s, 3H), 4.35 (m, 4H), 6.90 (d, J=8.97 Hz, 1H), 7.03 (dd, J=8.97, 3.11 Hz, 1H), 7.46 (m, 2H), 7.53 (m, 3H), 8.03 (m, 2H), 8.50 (d, J=5.31 Hz, 1H).

### Example 10(1)-(3)

By the same procedure as described in Example 10 using the compound prepared in Example 5(2), Example 5(6) or Example 7(14) instead of the compound prepared in Example 5(5) and methyiodide or ethylbromide instead thereof, the following compounds of the present invention were obtained.

### Example 10(1)

### N-{4-[2,5-bis(2,2,2-trifluoroethoxy)phenyl]pyrimidin-2-yl}-N,4-dimethylbenzenesulfonamide:

TLC: Rf 0.28 (hexane : ethyl acetate = 2 : 1);
NMR: δ 2.38 (s, 3H), 3.75 (s, 3H), 4.36 (m, 4H), 6.91 (d, J=9.15 Hz, 1H), 7.04 (dd, J=9.15, 3.11 Hz, 1H), 7.25 (m, 2H), 7.53 (d, J=5.31 Hz, 1H), 7.60 (d, J=3.11 Hz, 1H), 7.92 (m, 2H), 8.50 (d, J=5.31 Hz, 1H).

### Example 10(2)

### N-{4-[2,5-bis(difluoromethoxy)phenyl]pyrimidin-2-yl}-N-methylbenzenesulfonamide:

TLC: Rf 0.32 (hexane : ethyl acetate = 2 : 1);
NMR: δ 3.77 (s, 3H), 6.49 (m, 2H), 7.25 (m, 2H), 7.49 (m, 5H), 8.03 (m, 2H), 8.53 (d, J=5.13 Hz, 1H).

### Example 10(3)

### N-{4-[2,5-bis(2,2,2-trifluoroethoxy)phenyl]pyrimidin-2-yl}-N-ethylacetamide:

TLC: Rf 0.61 (hexane : ethyl acetate = 2 : 1);
NMR: δ 1.28 (t, J=6.96 Hz, 1H), 2.45 (s, 3H), 4.30-4.48 (m, 4H), 6.96 (d, J=8.97 Hz, 1H), 7.10 (dd, J=8.97, 3.30 Hz, 1H), 7.68-7.75 (m, 2H), 8.67 (d, J=5.31 Hz, 1H).

### Example 11

### 4-[2,5-bis(2,2,2-trifluoroethoxy)phenyl]-2-chloropyrimidine:

A mixture of the compound (367 mg, 1.0 mmol) prepared in Example 6(2), acetic acid (5 mL) and concentrated hydrochloric acid (2 mL) was added by sodium nitrite (138 mg, 2.0 mmol) at -30°C and stirred for 3 hours. The reaction solution was extracted by toluene. The organic layer was washed with saturated brine, dried over anhydrous sodium sulfate and concentrated. The residue was purified by column chromatography on silica gel (ethyl acetate : hexane = 1 : 3) to give the title compound (250.8 mg) having the following physical data.
TLC: Rf 0.62 (ethyl acetate : hexane = 1 : 2);
NMR: δ 4.41 (m, 4H), 6.96 (d, J=9.1 Hz, 1H), 7.12 (dd, J=9.1, 3.2 Hz, 1H), 7.72 (d, J=3.2 Hz, 1H), 7.92 (d, J=5.3 Hz, 1H), 8.66 (d, J=5.3 Hz, 1H).

### Example 12:

### N-benzyl-4-[2,5-bis(2,2,2-trifluoroethoxy)phenyl]-N-methyl-2-pyrimidinamine:

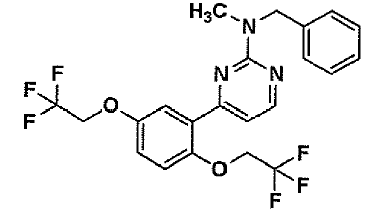

A mixed solution of the compound (80 mg, 0.21 mmol) prepared in Example 11 in tetrahydrofuran (2 mL) and dimethylformamide (2 mL) was added by methylbenzylamine (50 mg, 0.41 mmol) and stirred at 75°C overnight. The reaction solution was added by water and extracted by ethyl acetate. The organic layer was washed with IN hydrochloric acid, saturated sodium hydrogen carbonate aqueous solution and saturated brine, successively, dried over anhydrous sodium sulfate and concentrated. The residue was purified by column chromatography on silica gel (ethyl acetate : hexane = 1 : 5) to give the compound (102.5 mg) of the present invention having the following physical data.
TLC: Rf0.45 (ethyl acetate : hexane = 1 : 5);
NMR: δ 3.25 (s, 3H), 4.17 (m, 2H), 4.31 (q, J=8.06 Hz, 2H), 4.96 (s, 2H), 6.92 (d, J=8.97 Hz, 1H), 6.99 (m, 1H), 7.15 (d, J=5.31 Hz, 1H), 7.29 (m, 5H), 7.48 (s, 1H), 8.40 (d, J=5,13 Hz, 1H).

### Example 12(1)

### N-benzyl-4-[2,5-bis(2,2,2-trifluoroethoxy)phenyl]-2-pyrimidinamine:

By the same procedure as described in Example 12 using benzylamine instead of N-methylbenzylamine, the compound of the present invention having the following physical data was obtained.
TLC: Rf 0.28 (ethyl acetate : hexane = 1 : 4);
NMR: δ 4.29 (m, 4H), 4.70 (d, J=5.86 Hz, 2H), 5.55 (t, J=5.68 Hz, 1H), 6.92 (d, J=8.97 Hz, 1H), 7.01 (m, 1H), 7.20 (d, J=5.13 Hz, 1H), 7.33 (m, 5H), 7.46 (d, J=3.11 Hz, 1H), 8.3 6 (d, J=5.13 Hz, 1H).

### Example 13:

### 1-{4-[2,5-bis(2,2,2-trifluoroethoxy)phenyl]pyrimidin-2-yl}-2-piperidinone:

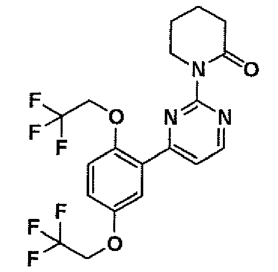

A solution of the compound (180 mg, 0.50 mmol) prepared in Example 6(2) in anhydrous pyridine (1.5 mL) was added by 5-bromopentanoylchloride (80 µL, 0.60 mmol) and stirred at room temperature overnight. The reaction solution was diluted by ethyl acetate, washed with water, citric acid aqueous solution, saturated sodium hydrogen carbonate aqueous solution and saturated brine, successively, dried over anhydrous sodium sulfate, concentrated and crude purified compound of N-{4-[2,5-bis(2,2,2-trifluoroethoxy)phenyl]pyrimidin-2-yl}-5-bromopentanamide (132 mg) was obtained. After that, the above obtained crude purified compound was dissolved in anhydrous dimethylformamide (5 mL), was added by anhydrous tetrahydrofuran (1 mL), was added by sodium halide (60% in oil, 11 mg, 0.27 mmol) and stirred by materials disappering. The reaction solution was added by saturated ammonium chloride aqueous solution and extracted by ethyl acetate. The organic layer was washed with saturated brine, dried over anhydrous sodium sulfate and then concentrated to give the compound (103 mg) of the present invention having the following physical data.
TLC: Rf 0.21 (hexane: ethyl acetate = 2 : 1);
NMR: δ 2.00 (m, 4H), 2.66 (t, J=6.13 Hz, 2H), 3.97 (t, J=6.22 Hz, 2H), 4.38 (m, 4H), 6.95 (d, J=9.16 Hz, 1H), 7.07 (dd, J=9.16, 3.30 Hz, 1H), 7.69 (d, J=3.30 Hz, 1H), 7.74 (d, J=5.31 Hz, 1H), 8.77 (d, J=5.31 Hz, 1H).

### Example 14

### 4-[2,5-bis(2,2,2-trifluoroethoxy)phenyl]-N,N-diethyl-2-pyrimidinamine:

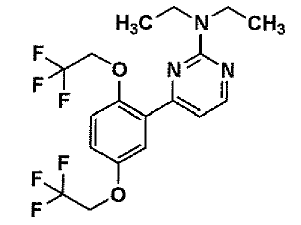

A solution of the compound (80 mg, 0.18 mmol) prepared in Example 10(3) in anhydrous tetrahydrofuran (1 mL) was dropped by BH₃·THF complex (684 µL, 0.73 mmol) under the atmosphere of argon at 60°C and stirred for 7 minutes. The reaction solution was added by ice-1N hydrochloric acid, stirred, added by 1N sodium hydroxide aqueous solution to be neutralized and then extracted by ethyl acetate. The organic layer was washed by saturated brine, dried over anhydrous sodium sulfate and then concentrated. The residue was purified by chromatography on silica gel (hexane : ethyl acetate = from 20 : 1 to 5 : 1) to give the compound (7.5 mg) of the present invention having the following physical data.
TLC: Rf 0.64 (hexane : ethyl acetate = 2 : 1);
NMR: δ 1.24 (t, J=7.05 Hz, 6H), 3.69 (q, J=7.08 Hz, 4H), 4.35 (m, 4H), 6.94 (d, J=8.97 Hz, 1H), 7.01 (dd, J=8.97, 3.11 Hz, 1H), 7.07 (d, J=5.31 Hz, 1H), 7.67 (d, J=3.11 Hz, 1H), 8.35 (d, J=5.31 Hz, 1H).

### Example 14(1)

### 4-[2,5-bis(2,2,2-trifluoroethoxy)phenyl]-2-piperidin-1-yl)pyrimidine (compound A) and 5-({4-[2,5-bis(2,2,2-trifluoroethoxy)phenyl]pyrimidin-2-yl}amino)-1-pentanol (compound B):

By the same procedure as described in Example 14 using the compound prepared in Example 13 instead of the compound prepared in Example 10(3), the compounds of the present invention having the following physical data were obtained.

### Compound A

TLC: Rf 0.39 (hexane : ethyl acetate = 1 : 1);
NMR: δ 1.49-1.79 (m, 6H), 3.78-3.95 (m, 4H), 4.20-4.49 (m, 4H), 6.94 (d, J=8.79 Hz, 1H), 6.97-7.08 (m, 2H), 7.59 (d, J=3.11 Hz, 1H), 8.35 (d, J=5.13 Hz, 1H).

### Compound B

TLC: Rf 0.17 (hexane : ethyl acetate = 1 : 1);
NMR: δ 1.39-1.81 (m, 6H), 3.41-3.57 (m, 2H), 3.67 (t, J=6.41 Hz, 2H), 4.22-4.49 (m, 4H), 5.18 (t, J=5.40 Hz, 1H), 6.94 (d, J=8.97 Hz, 1H), 7.02 (dd, J=8.97, 3.12 Hz, 1H), 7.12 (d, J=5.13 Hz, 1H), 7.58 (d, J=3.12 Hz, 1H), 8.32 (d, J=5.13 Hz, 1H).

### Example 15(1)-(3)

By the same procedure as described in Example 3 using the corresponding amine compound instead of the compound prepared in Example 2 and benzenesulfonyl chloride instead of benzyloxyacetyl chloride, the following compounds of the present invention were obtained.

### Example 15(1)

### N-(6-phenylpyridin-2-yl)benzenesulfonamide:

TLC: Rf 0.43 (ethyl acetate : hexane = 1 : 2);
NMR: δ 7.21 (d, J=8.24 Hz, 1H), 7.32 (d, J=7.69 Hz, 1H), 7.48 (m, 7H), 7.67 (m, 1H), 7.81 (m, 2H), 7.96 (m, 2H).

### Example 15(2)

### N-(4-phenylpyridin-2-yl)benzenesulfonamide:

TLC: Rf 0.35 (ethyl acetate : hexane = 1 : 1);
NMR(DMSO-d₆): δ 7.18 (dd, J=5.77, 1.37 Hz, 1H), 7.37 (d, J=0.92 Hz, 1H), 7.57 (m, 9H), 7.90 (m, 2H), 8.06 (d, J=6.04 Hz, 1H).

### Example 15(3)

### N-[6-(2,5-dimethoxyphenyl)pyridin-2-yl]benzenesulfonamide:

TLC: Rf 0.48 (ethyl acetate : hexane = 1 : 1);
NMR: δ 3.82 (s, 3H), 3.92 (s, 3H), 6.99 (m, 4H), 7.11 (dd, J=2.20, 1.10 Hz, 1H), 7.47 (m, 3H), 7.61 (dd, J=8.70, 7.41 Hz, 1H), 7.98 (m, 2H), 11.43 (m, 1H).

### Example 16:

### N-[6-(2,5-dimethoxyphonyl)pyridin-2-yl]-N-methylbenzenesulfonamide:

By the same procedure as described in Example 10 using the compound prepared in Example 15(3) instead of the compound prepared in Example 5(5), the compound of the present invention was obtained.
TLC: Rf 0.40 (ethyl acetate : hexane = 1 : 3);
NMR: δ 3.34 (s, 3H), 3.72 (s, 3H), 3.80 (s, 3H), 6.86 (m, 1H), 6.91 (m, 1H), 7.10 (d, J=2.93 Hz, 1H), 7.42 (m, 2H), 7.58 (m, 4H), 7.71 (t, J=7. 78 Hz, 1H), 7.81 (m, 1H).

### Biological Example 1

### Receptor binding experiment:

The affinity of the compounds in the present invention to MBR was determined using rat brain membrane preparation. In addition, the measurement in the present invention was improved the accuracy of measurement and the sensitivity of measurement for evaluating the compounds in the present invention as follows. After male Wister rats were decapitated to extirpate the whole brain and cerebellums were removed. They were homogenized in ice-cold 50 mmol/L Tris-HCL buffer solution (pH 7.4), centrifuged and the obtained pellets were washed. The pellets resuspended and adjusted to about 1 mg/mL were used as rat brain membrane preparations for binding assay. The binding assay were experimented using [³H]PK11195 as a MBR selective ligand. In addition, PK11195 was described in "*European Journal of Pharmacology*, 119, 153-167 (1985)" as a MBR selective ligand, (1-(2-chlorophenyl)-N-methyl-N-(1-methylpropyl)-3-isoquinolinecarboxamide).

To determine the amount of total binding in saturation binding experiment, membrane preparations, various concentrations of [³H]PK11195, final concentration: 0.5 vol% dimethylsulfoxide (DMSO) and 50 mmol/L Tris-HCL buffer solution (pH 7.4) were mixed (total volume 200 µL) and were incubated for 1 hour at room temperature. To determine the amount of non-specific binding, the mixture was added by final concentration 20 µmol/L of PK11195 instead of DMSO to be incubated for 1 hour. The mixture was rapidly filtrated on GF/B filter pretreated with 0.3% polyethyleneimine using cell harvester and washed over 50 mmol/L Tris-HCl buffer solution (pH 7.4) twice. The filter was dried and then the radioactivity on the filter was measured by liquid scintillation counter. The data obtained by the binding experiments were Scatchard analyzed using analysis software, KELL (Ver.6, BIOSOFT) and the dissociation constant (K_{D} value) was determined.

To determine the amount of total binding in competition binding experiment, membrane preparations, final concentration 0.5 or 1 nmol/L [³H]PK11195, final concentration: 0.5 vol% dimethylsulfoxide (DMSO) and 50 mmol/L Tris-HCL buffer solution (p H7.4) were mixed (total volume 200 µL) and were incubated for 1 hour at room temperature. To determine the amount of non-specific binding, the mixture was added by final concentration 20 µmol/L of PK11195 instead of DMSO to be incubated. In addition, to determine the affinity of the compounds in the present invention, the mixture was added by final concentration 10 pmol/L to 1 µmol/L of the solution of the compounds in the present invention in DMSO instead of DMSO to be incubated. After 1 hour, the mixture was saction filtrated by the above-mentioned method and the radioactivity on the filter was measured by liquid scintillation counter. The concentration of the compounds in the present invention (IC₅₀) which was necessary for inhibiting the amount of specific binding of [³H]PK1 1195 by 50% was determined from the obtained data. The inhibition constant (Kᵢ value) was calculated according to Cheng and Prusoff formula *(Biochemical Pharmacolgy*, 22, 3099-3108 (1973)) using K_{D} value and IC₅₀.

In consequence, it was clear that the compounds in the present invention had high affinity to MBR.

For example, Kᵢ value of the compound of Example 3(46) was 0.36 µmol/L, Kᵢ value of the compound of Example 5(2) was 0.01 µmol/L.

### Biological Example 2

### Evaluation of anti-stress effects:

Psychological stress was loaded to male Wistar rats *(Brain Research,* 641, 21-28 (1994)). Water was stored up to the depth of about 10 cm in a container where the platform was set at the center. A stressor began to load to rats 30 minutes after the vehicle or the compound in the present invention (the compound of Example 17) were orally administered by dosage of 10 mg/kg. The number of defecation was counted 1 hour later (10 per each group). Rats without administration and load of stressor rarely defecated for 1 hour. In contrast, remarkable defecation was admitted in media treated group (average 8.2 feces) loaded by stressor. However, it proved that the compound in the present invention controlled the number of defecation (average 6.4 feces) more significantly than that of media treated group. The results clarified that the compounds in the present invention had anti-stress effects.

### Preparation Example 1:

The following components were admixed in conventional method, punched out to give 10000 tablets each containing 10 mg of active ingredient.
N-{4-[3-(acetylamino)phenyl]pyrimidin-2-yl}benzamide (100 g)
carboxymethylcellulosecalcium (disintegrant) (20.0 g)
magnesium stearate (lubricant) (10.0 g)
microcrystalline cellulose (870 g)

### Preparation Example 2:

After mixing the following components by a conventional method, the resulting solution was filtrated by dust-proof filter and 5 mL portions thereof were filled in amples, respectively, and heat-sterilized by autoclave to obtain 10000 amples of injection containing each 20 mg of the active ingredient.
N-{4-[3-(acetylamino)phenyl]pyrimidin-2-yl}benzamide (200 g)
mannitol (2 kg)
distilled water (50 L)

### Industrial Applicability

The compound of the present invention is able to apply to the following drug.

Since the compounds of the present invention represented by formula (I) have the affinity to MBR, they are useful for the prevention and/or treatment for disease induced or exacerbated and/or reignited by stressor or useful for the prevention and/or treatment for disease caused by stress.

The disease induced or exacerbated and/or reignited by stressor or the disease caused by stress include, for example, central nervous system diseases caused by stress (*e*.*g*. anxiety related disease (neurosis, psychosomatic disorder, generalized anxiety disorder (GAD), social-anxiety disorder (SAD), panic disorder, hyperactivity disorder, attention-deficit, personality disorder, bipolar disorder, autism *etc*.), sleep disorder, depression, reactive depression, epilepsy, Parkinson's disease, Perkinsonian syndrome, schizophrenia, autonomic dystonia, Huntington's disease, Alzheimer's disease, affective disorder, cognitive disorder, migraine, tension headache, cluster headache, posttraumatic stress disorder, dissociative disorder, insomnia, nervous vomiting, nervous cough, psychogenic convulsive seizure, psychogenic syncopal attack, maladjustment to job, burn-out syndrome, chronic fatigue syndrome, writer's cramp, spastic torticollis, *etc*.), respiratory system diseases caused by stress (*e*.*g*. asthma, bronchial asthma, hyperventilation syndrome, laryngeal spasm, chronic obstructive pulmonary diseases, *etc*.), digestive system diseases caused by stress *(e.g.* irritable bowel syndrome, peptic ulcer, functional dyspepsia, gastric ulcer, duodenal ulcer, ulcerative colitis, biliary tract dyskinesia, esophageal spasm, gastric atony, aerophagy, chronic hepatitis, chronic panceatitis, *etc*.), cardiovascular system diseases caused by stress (*e*.*g*. essential hypertension, arrhythmia, (neurological) angina pectoris, essential hypotension, orthostatic dysregulation, myocardial infarction, arteriosclerosis, vertigo *etc*.), uropathy·reproductive system diseases caused by stress (*e*.*g*. dysuria, nervous pollakisuria (hyperreflexic bladder), nocturia, enuresis, psychogenic ischuria, impotentia, prostatism, urethral syndrome *etc*.), gynecologic disorder caused by stress (*e*.*g*. menopausal disorder, menstrual pain, premenstrual syndrome, infertility, frigidity, serious vomiting of pregnancy, abortion, immature birth, *etc*.), endocrine and metabolic disease caused by stress (*e*.*g*. anorexia nervosa, eating disorder, anorexia, hyperphagia, Bartter's syndrome, hyperthyroidism, diabetes, psychogenic polydipsia, adiposity, reflex hypoglycemia *etc.*), ophthalmologic diseases caused by stress (*e*.*g*. asthenopia, central retinitis, floaters, blepharospasm, primary glaucoma, vertigo *etc.),* otolaryngological diseases caused by stress (*e*.*g*. tinnitus, vertigo, psychogenic deafness, chronic sinusitis, allergic rhinitis, smell disorder, stuttering, aphonia, *etc*.), dental surgery and dentistry caused by stress (*e*.*g*. temporomandibular arthrosis, glossopharyngeal neuralgia, sudden glossodynia, stomatitis, toothache, ozostomia, abnormal salivation, bruxism *etc*.), surgical and orthopedic diseases caused by stress (*e*.*g*. postoperative abdominal neurosis, dumping syndrome, polysurgery, plastic postoperative neurosis, rheumatoid arthritis, low back pain, cervico-omo-brachial syndrome, stiff neck, fibrositis, polyarthralgia, systemic myalgia, gout, *etc*.), skin diseases caused by stress (*e*.*g*. chronic urticaria, atopic dermatitis, hyperhidrosis, eczema, skin pruritus, alopecia areata, *etc.)* and other diseases caused by stress (*e*.*g*. cancer, systemic lupus erythematosus *etc*.).

## Claims

1. A compound represented by formula (I) 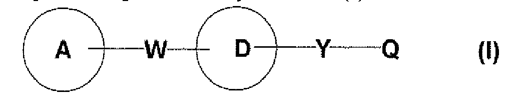 wherein ringA is a cyclic ring which may have a substituent(s),
Q is alkyl which may have a substituent(s) or a cyclic ring which may have a substituent(s),
ringD is a cyclic ring which may have a substituent(s),
W is a single bond or a spacer of which main chain has an atom number of 1-4, and
Y is a spacer of which main chain has an atom number of 1-4,
a salt thereof, an N-oxide thereof or a solvate thereof, or a prodrug thereof.

2. The compound according to claim 1, wherein ringA is (1) a C3-10 mono-or bi-carbocyclic ring, or (2) a 3-10 membered mono- or bi-heterocyclic ring containing 1 to 5 hetero atom(s) selected from oxygen atom(s), nitrogen atom(s) and sulfur atom(s), which may have a substituent(s).

3. The compound according to claim 2, wherein ringA is benzene which may have a substituent(s).

4. The compound according to claim 1, wherein W is a single bond.

5. The compound according to claim 1, wherein Y is a spacer of which main chain has an atom number of 1-4 containing a hydrogen-bond acceptor site.

6. The compound according to claim 1, wherein Y is 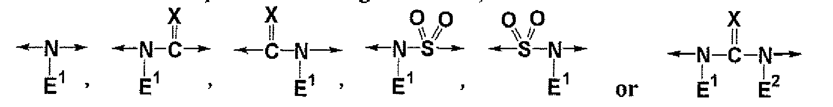 wherein E¹ and E² are each independently a hydrogen atom(s) or a substituent(s), X is an oxygen atom or a sulfur atom, left-pointing arrow binds to ringD, right-pointing arrow binds to Q.

7. The compound according to claim 1, wherein ringD is a 3-10 membered mono-, bi-heterocyclic ring containing of 1 to 5 hetero atom(s) selected from oxygen atom(s), nitrogen atom(s) and sulfur atom(s), which may have a substituent(s).

8. The compound according to claim 7, ringD is pyridine or pyrimidine which may have a substituent(s).

9. The compound according to claim 8, wherein the compound is the compound represented by formula (Ia) 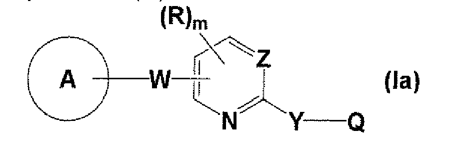 wherein Z is a carbon atom or nitrogen atom, R is an hydrogen atom(s) or a substituent(s), m is an integer of 1-3, and other symbols have the same meanings as in claim 1.

10. The compound according to claim 9, wherein the compound is the compound represented by formula (Ib) 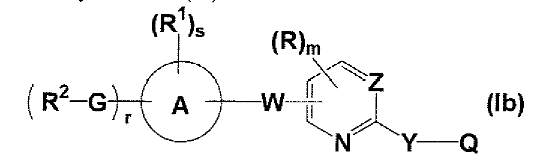 wherein R¹ is a hydrogen atom(s) or a substituent(s), R² is C1-6 alkyl which is substituted with 1-5 halogen atom(s), G is -O- or -S-, r is an integer of 1-2, s is an integer of 1-4, in which sum of r and s is below 5, and other symbols have the same meanings as in claims 1 and 9.

11. The compound according to claim 10, wherein R² is C1-2 alkyl which is substituted with 1-5 fluorine atom(s).

12. The compound according to claim 10, wherein the compound is the compound represented by formula (Ib-1) 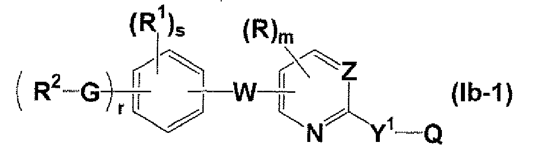 wherein Y¹ is 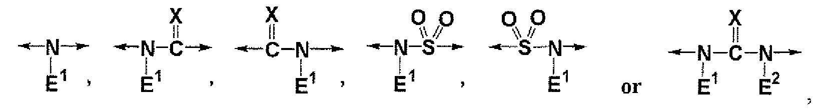 other symbols have the same meanings as in claim 1, 6 and 10, and wherein
(1) N-{4-[2,5-bis(2,2,2-trifluoroethoxy)phenyl]pyrimidin-2-yl}-N'-phenylurea,
(2) N-{4-[2,5-bis(2,2,2-trifluoroethoxy)phenyl]pyrimidin-2-yl}-N'-(4-chlorophenyl)urea,
(3) N-{4-[2,5-bis(2,2,2-trifluoroethoxy)phenyl]pyrimidin-2-yl}-N'-(3-chlorophenyl)urea and
(4) N-{4-[2,5-bis(2,2,2-trifluoroethoxy)phenyl]pyrimidin-2-yl}-2-thiophenecarboxamide are excepted.

13. The compound according to claim 10, the compound is
(1) N-{4-[2,5-bis(difluoromethoxy)phenyl]pyrimidin-2-yl}thiophene-2-carboxamide,
(2) N-{4-[2,5-bis(difuloromethoxy)phenyl]pyrimidin-2-yl}-N'-isopropylurea,
(3) N-{4-[2,5-bis(difluoromethoxy)phenyl]pyrimidin-2-yl}methanesulfonamide,
(4) N-{4-[2,5-bis(difluoromethoxy)phenyl]pyrimidin-2-yl}benzenesulfonamide,
(5) N-{4-[2,5-bis(difluoromethoxy)phenyl]pyrimidin-2-yl}-1-phenylmethanesulfonamide,
(6) N-{4-[2,5-bis(difluoromethoxy)phenyl]pyrimidin-2-yl}-4-methylbenzenesulfonamide,
(7) N-{4-[2,5-bis(difluoromethoxy)phenyl]pyrimidin-2-yl}-3-methylbenzenesulfonamide,
(8) N-{4-[2,5-bis(difluoromethoxy)phenyl]pyrimidin-2-yl}-3,5-dimethylbenzenesulfonamide,
(9) N-{4-[2,5-bis(difluoromethoxy)phenyl]pyrimidin-2-yl}-3,5-dichlorobenzenesulfonamide,
(10) N-{4-[2,5-bis(2,2,2-trifluoroethoxy)phenyl]pyrimidin-2-yl}benzenesulfonamide,
(11) N-{4-[2,5-bis(2,2,2-trifluoroethoxy)phenyl]pyrimidin-2-yl}-4-methylbenzenesulfonamide,
(12) N-{4-[2,5-bis(2,2,2-trifluoroethoxy)phenyl]pyrimidin-2-yl}-N-methylbenzenesulfonamide,
(13) N-{4[2,5-bis(2,2,2-trifluoroethoxy)phenyl]pyrimidin-2-yl}-N,4-dimethylbenzenesulfonamide,
(14) N-{4-[2,5-bis(difluoromethoxy)phenyl]pyrimidin-2-yl}-N-methylbenzenesulfonamide,
(15) N-benzyl-N-{4-[2,5-bis(difluoromethoxy)phenyl]pyrimidin-2-yl}methanesulfonamide,
(16) N-{4-[2,5-bis(difluoromethoxy)phenyl]pyrimidin-2-yl}-N-ethylbenzenesulfonamide,
(17) N-{4-[2,5-bis(difluoroemethoxy)phenyl]pyrimidin-2-yl}-N-propylbenzenesulfonamide,
(18) N-{4-[2,5-bis(difluoromethoxy)phenyl]pyrimidin-2-yl}-N-isobutylbenzenesulfonamide,
(19) N-{4-[2,5-bis(difluoromethoxy)phenyl]pyrimidin-2-yl}-N-(2-methoxyethyl)benzenesulfonamide,
(20) 4-[2,5-bis(2,2,2-trifluoroethoxy)phenyl]-N-ethyl-2-pyrimidinamine,
(21) N-benzyl-4-[2,5-bis(2,2,2-trifluoroethoxy)phenyl]-N-methyl-2-pyrmidinamine, or
(22) N-benzyl-4-[2,5-bis(2,2,2-trifluoroethoxy)phenyl]-2-pyrimidinamine.

14. A pharmaceutical composition comprising of the compound represented by formula (I) according to claim 1, a salt thereof, an N-oxide thereof, a solvate thereof or a prodrug thereof.

15. The pharmaceutical composition according to claim 14, wherein the pharmaceutical composition is preventive and/or therapeutic agent for mitochondrial benzodiazepine receptor mediated disease.

16. The pharmaceutical composition according to claim 15, wherein the mitochondrial benzodiazepine receptor mediated disease is a disease caused by stress.

17. The pharmaceutical composition according to claim 16, wherein the disease caused by stress is a central nervous system disease caused by stress, a respiratory system disease caused by stress and/or a digestive system disease caused by stress.

18. The pharmaceutical composition according to claim 17, wherein the central nervous system disease caused by stress is anxiety-related disease, sleep disorder, depression and/or epilepsy, a respiratory system disease caused by stress is asthma, a digestive system disease caused by stress is irritable bowel syndrome.

19. A pharmaceutical composition combining of the compound represented by formula (I) according to claim 1, a salt thereof, an N-oxide thereof a solvate thereof or a prodrug thereof and one kind or more kind selected from antianxiety drugs, antidepressant drugs, antiparkinson drugs, therapeutic drugs for schizophrenia, antiepileptic drugs, therapeutic drugs for asthma, therapeutic drugs for peptic ulcer, adjustive drugs for gastrointestinal function, antidiarrheals, evacuants, antihypertensive drugs, antiarrhythmic drugs, inotropic drugs and therapeutic drugs for urination disorder.

20. A method for prevention and/or treatment for a mitochondrial benzodiazepine receptor mediated disease in mammals, which comprises administering an effective amount of the compound represented by formula (I) according to claim 1, a salt thereof, an N-oxide, a solvate or a prodrug thereof to the mammals.

21. Use of the compound represented by formula (I) according to claim 1, a salt thereof, an N-oxide thereof, a solvate thereof or a prodrug thereof for preparing a preventive and/or therapeutic agent for a mitochondrial benzodiazepine receptor mediated disease.
